# EUROPEAN PATENT APPLICATION

(11) **EP 4 740 938 A1**
(43) Date of publication of application: **13.05.2026**
(21) Application number: 24835319.5
(22) Date of filing: 01.07.2024
(51) Int. Cl.: A61K 9/19, A61P 9/10, C12N 15/57, A61K 38/48

(54) **PREPARATION METHOD FOR KLK1 FUSION PROTEIN**

(30) Priority: 04.07.2023 CN 202310813280
(71) Applicant: ALEBUND PHARMACEUTICALS (HONG KONG) LIMITED, Hong Kong (HK)
(72) Inventor: SHU, Chutian, Shanghai 200020 (CN)
(74) Representative: Simmons & Simmons LLP (Munich)
(86) International application number: PCT/CN2024/102903
(87) International publication number: WO 2025/007838

(57) **Abstract**

A method for preparing a fusion protein comprising tissue kallikrein 1 (KLK1), a fusion protein comprising KLK1, a pharmaceutical composition comprising the fusion protein, and a use of the pharmaceutical composition in treating or preventing diseases.

## Description

### FIELD OF THE INVENTION

The present application relates to the technical field of biomedicines, and specifically, to a method for preparing a fusion protein comprising tissue kallikrein 1 (KLK1) (for example, KLK1-Fc fusion protein), a fusion protein comprising KLK1, a pharmaceutical composition comprising the fusion protein, and use thereof in treating or preventing diseases.

### RELATED ART

The kallikrein-kinin system (also known as "KKS") is a complex endogenous multi-enzyme system consisting of kininogen, kallikrein (also referred to as "KLK" or "kininogenase"), and kinin, which is involved in the blood pressure regulation, the glucose metabolism, and the physiological functions of the kidney and the nervous system. KLK can catalyze the cleavage of kininogen, to produce biologically active kinin. KLK can be divided into plasma KLK and tissue KLK. There are great differences between the substrates, molecular weights and functions of the plasma KLK and the tissue KLK. For example, the plasma KLK is encoded by *KLKB1* gene located on chromosome 4q34-35, and no other known paralogues are found; and the tissue KLK is encoded by *KLK1-KLK15* genes located on chromosome 19q13, and 15 known paralogues (KLK1-KLK15) are found. KLK1 is the only member of the tissue KLK family that can produce bioactive kinin. KLK1 can relax isolated arteries, have vasodilation, anti-inflammation, and cell repair effects and reduce the apoptosis.

At present, the commercially available KLK1 is mainly extracted from human urine or porcine pancreas, and is an endogenous protein. Because of the complex production process, short half-life (for example, Kailikang^{®} has a half-life of less than 4 h in human), high administration frequency (for example, Kailikang^{®} is intravenously injected once a day for consecutive 21 days according to the clinical instructions), high cost and difficult quality control, the use is restricted to some extent. Therefore, there is obviously an urgent unmet need in clinic to develop a KLK1 protein product with prolonged half-life, lower frequency of administration, and good production cost and quality controllability.

### SUMMARY OF THE INVENTION

In an aspect, the present application provides a method for preparing a fusion protein comprising tissue kallikrein 1 (KLK1). The method comprises Step (1): introducing a recombinant vector comprising a nucleic acid molecule encoding a fusion protein into a suitable protein expression system. The fusion protein comprises a masking peptide, a first polypeptide and a second polypeptide. The masking peptide, the first polypeptide and the second polypeptide are linked directly or covalently via a linker, and,
a) the masking peptide has an amino acid sequence that can be specifically recognized and cleaved by a protease;
b) the first polypeptide comprises KLK1 or a variant thereof; and
c) the second polypeptide is used to extend the half-life of the first polypeptide.

In certain embodiments, the method further comprises Step (2): mixing the fusion protein obtained in Step (1) with a protease to perform a specific enzymatic cleavage reaction, thereby removing the masking peptide. In certain embodiments, the fusion protein obtained in Step (1) is purified before performing the specific enzymatic cleavage reaction in Step (2).

In certain embodiments, the amino acid sequence of the masking peptide is as shown in SEQ ID NO: 5. In certain embodiments, an amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122. In certain embodiments, the amino acid sequence of the second polypeptide is as shown in SEQ ID NO: 11, SEQ ID NO: 12, SEQ ID NO: 13, SEQ ID NO: 14 or SEQ ID NO: 15.

In another aspect, the present application provides a fusion protein, comprising a masking peptide, a first polypeptide and a second polypeptide. The masking peptide, the first polypeptide and the second polypeptide are linked directly or covalently via a linker, and
a) the masking peptide has an amino acid sequence that can be specifically recognized and cleaved by a protease;
b) the first polypeptide comprises KLK1 or a variant thereof; and
c) the second polypeptide is used to extend the half-life of the first polypeptide.

In certain embodiments, the amino acid sequence of the masking peptide is as shown in SEQ ID NO: 5. In certain embodiments, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122. In certain embodiments, the amino acid sequence of the second polypeptide is as shown in SEQ ID NO: 11, SEQ ID NO: 12, SEQ ID NO: 13, SEQ ID NO: 14 or SEQ ID NO: 15. In certain embodiments, the amino acid sequence of the KLK1 fusion protein is as shown in any one of SEQ ID NOs: 36-55, SEQ ID NOs: 100-109, SEQ ID NOs: 111-113, and SEQ ID NOs: 125-127.

In another aspect, the present application provides a nucleic acid molecule comprising a nucleotide sequence encoding the KLK1 fusion protein of the present application.

In another aspect, the present application provides a vector comprising the nucleic acid molecule of the present application.

In another aspect, the present application provides a protein expression system comprising the nucleic acid molecule of the present application or the vector of the present application.

In another aspect, the present application provides a pharmaceutical composition comprising the KLK1 fusion protein of the present application, the nucleic acid molecule of the present application, the vector of the present application, or the protein expression system of the present application, and a pharmaceutically acceptable carrier.

In another aspect, the present application provides a method for constructing a protein expression system comprising introducing a nucleic acid molecule encoding the KLK1 fusion protein of the present application into a vector to construct an expression vector; and introducing the expression vector into a protein expression system.

In another aspect, the present application provides a method for treating or preventing a disease comprising administering an isolated KLK1 fusion protein prepared by the method according to the present application to a subject in need thereof. The disease is selected from the group consisting of an ischemic disease, a hemorrhagic disease, a kidney disease, a metabolic disease, and a neurodegenerative disease. In certain embodiments, the ischemic disease is acute ischemic stroke. In certain embodiments, the kidney disease is chronic nephropathy or acute renal injury.

### BRIEF DESCRIPTION OF THE DRAWINGS

FIG. 1 shows an exemplary plasmid map, in which the "gene" indicated by the arrow represents a target gene.
FIG. 2 shows the enzymatic activity of a positive control (FIG. 2A), a KLK1 fusion protein which has an EK recognition sequence in the preparation process and is obtained after cleavage with EK (FIG. 2B), a KLK1 fusion protein without an EK recognition sequence in the preparation process (FIG. 2C), or a KLK1 fusion protein with an EK recognition sequence and without cleavage with EK (FIG. 2D).
FIG. 3 shows the enzymatic activity of KLK1 fusion proteins of different sequences which have an EK recognition sequence in the preparation process and are obtained after cleavage with EK.
FIG. 4 shows a total ion current (TIC) spectrum (FIG. 4A), a mass spectrometry (MS) spectrum (FIG. 4B), a deconvoluted spectrum (FIG. 4C), and a partial enlargement of the deconvoluted spectrum (FIG. 4D) in the reduced mass analysis of deglycosylated fusion protein 2.4.
FIG. 5 shows a total ion current (TIC) spectrum (FIG. 5A), a mass spectrometry (MS) spectrum (FIG. 5B), a deconvoluted spectrum (FIG. 5C), and a partial enlargement of the deconvoluted spectrum (FIG. 5D) in the intact mass analysis of deglycosylated fusion protein 2.4.
FIG. 6 shows the PK curves of fusion protein 2.1 and Kailikang^{®} in mice.
FIG. 7 shows the behavioral scores after the administration of a positive control (Edaravone), low-dose (25 µg/kg) fusion protein 2.1 and high-dose (150 µg/kg) fusion protein 2.1 in cerebral ischemia-reperfusion rats (i.e. transient middle cerebral artery occlusion (tMCAO) model) (*#P* < 0.05, ## *P <* 0.01, ### *P* < 0.001 vs sham group;* *P <* 0.05, ** *P <* 0.01, *** *P <* 0.001 vs model group).
FIG. 8 shows the results of TTC staining of the cerebral infarction area after the administration of a positive control (Edaravone), low-dose (25 µg/kg) fusion protein 2.1 and high-dose (150 µg/kg) fusion protein 2.1 in tMCAO rat model (#*P* < 0.05, ## *P <* 0.01, ### *P <* 0.001 vs sham group; * *P <* 0.05, ** *P <* 0.01, *** *P* < 0.001 vs model group, one-way ANOVA with Dunnett's test).
FIG. 9 shows the brain tissue after the administration of a positive control (Edaravone), low-dose (25 µg/kg) fusion protein 2.1 and high-dose (150 µg/kg) fusion protein 2.1 in cerebral ischemia-reperfusion rats (i.e. tMCAO model).

### DETAILED DESCRIPTION

Although various aspects and embodiments will be disclosed hereinafter, apparently, equivalent changes or modifications can be made by those skilled in the art, without departing from the scope and spirit of the present application. The various aspects and embodiments disclosed in the present application are intended to be illustrative only, and are not intended to limit the scope of the present application as defined by the claims. Unless otherwise specified, all technical and scientific terms used herein have the same meaning as commonly understood by one of ordinary skill in the art to which the present invention belongs. All references, patents, and patent applications cited in the present application are hereby incorporated by reference in their entireties.

### Definitions

When a polypeptide or protein is mentioned, the term "wild-type" used in the present application refers to a naturally occurring polypeptide or protein that does not comprise artificial mutation, insertion, deletion or modification at one or more amino acid positions. When a nucleic acid, nucleotide or polynucleotide is mentioned, the term "wild-type" used in the present application refers to a naturally occurring nucleic acid, nucleotide or polynucleotide that does not comprise artificial mutation, insertion, deletion or modification at one or more nucleotide positions. However, the polynucleotides encoding wild-type polypeptides are not limited to naturally occurring polynucleotides, and also comprise any polynucleotides encoding wild-type polypeptides (for example, artificially synthetic polynucleotides).

The term "subject" used in the present application comprises human beings and non-human animals. Non-human animals include all vertebrates, such as mammals and non-mammals. The term "subject" can also be livestocks, such as cattle, pigs, sheep, poultry and horses; rodents, such as rats, and mice; primates, such as apes, monkeys, chimpanzees, gorillas, orangutans, and baboons; or domestic animals such as dogs and cats. The "subject" can be male or female, and can be the old, adults, teenagers, children or babies.

The terms "protein", "polypeptide" and "peptide" used in the present application can be used interchangeably and refer to polymers of amino acids. The protein, polypeptide or peptide mentioned in the present application can comprise natural amino acids, non-natural amino acids, or amino acid analogues or mimics. The protein, polypeptide or peptide mentioned in the present application can be obtained by any method known in the art, for example, but not limited to, natural isolation, recombinant expression, and chemical synthesis, etc.

The term "amino acid" used in the present application refers to an organic compound comprising amino (-NH₂) and carboxyl (-COOH) functional groups and a side chain unique to each amino acid. Amino acid names are represented by standard one-letter or three-letter codes in the present application, which are summarized as follows:

| Name | Three-letter code | One-letter code |
|---|---|---|
| Alanine | Ala | A |
| Arginine | Arg | R |
| Asparagine | Asn | N |
| Aspartate | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

When "conservative substitution" is used with an amino acid sequence in the present application, it means that one amino acid residue is replaced by another amino acid residue with a side chain having similar physical and chemical properties. For example, conservative substitution can be made between amino acid residues with hydrophobic side chains (such as Met, Ala, Val, Leu and Ile), amino acid residues with neutral hydrophilic side chains (such as Cys, Ser, Thr, Asn and Gln), amino acid residues with acidic side chains (such as Asp, and Glu), amino acid residues with basic side chains (such as His, Lys and Arg), or amino acid residues with aromatic side chains (such as Trp, Tyr and Phe). It is known in the art that conservative substitution usually does not cause significant changes in the conformation structure of a protein, and thus the biological activity of the protein can be retained.

The term "homologous" as used in the present application means that a nucleic acid sequence (or its complementary strand) or an amino acid sequence has at least 60% (for example, at least 65%, 70%, 75%, 80%, 85%, 88%, 90%, 91%, 92%, 93%, 94%, 95%, 96%, 97%, 98%, or 99%) sequence identity to another sequence in optimal alignment.

When "percentage (%) sequence identity" is used with an amino acid sequence (or nucleic acid sequence), it refers to the percentage of amino acid (or nucleic acid) residues in a candidate sequence that are the same as those in a reference sequence relative to the total amino acid (or nucleic acid) residues in the candidate sequence after aligning the sequences and introducing gaps when necessary to achieve the maximum number of the same amino acid (or nucleic acid). In other words, the percentage (%) sequence identity of an amino acid sequence (or nucleic acid sequence) can be calculated by dividing the same number of amino acid residues (or bases) as the reference sequence compared with it by the total number of amino acid residues (or bases) in the candidate sequence or the reference sequence (whichever is shorter). The conservative substitution of an amino acid residue may or may not be regarded as the same residue. The tools disclosed in the art, such as BLASTN BLASTp (website of the National Center for Biotechnology Information (NCBI), also see Altschul S.F. et al., J. Mol. Biol., 215: 403-410 (1990); and Stephen F. et al., Nucleic Acids Res., 25: 3389-3402 (1997)), ClustalW2 (website of European Bioinformatics Institute, see Higgins D.G. et al., Methods in Enzymology, 266: 383-402 (1996); and Larkin M.A. et al., Bioinformatics (Oxford, England), 23 (21): 2947-8 (2007)) and ALIGN or Megalign (DNASTAR) software, can be used to align the sequences to determine the percentage sequence identity of an amino acid (or nucleic acid) sequence. Those skilled in the art can use default parameters by the tool or appropriately customize the parameters according to the needs of alignment, for example, by selecting an appropriate algorithm.

An "isolated" material has been artificially changed from its natural state. If an "isolated" composition or material appears in nature, it has been changed or detached from its original state, or both. For example, the naturally occurring polynucleotides or polypeptides in a living animal are not "isolated", but they can be considered as "isolated" if these polynucleotides or polypeptides are sufficiently separated from the materials that coexist with them in the natural state and exist in a substantially pure state. "An isolated nucleic acid sequence" refers to the sequence of an isolated nucleic acid molecule. In some embodiments, "isolated KLK1" refers to KLK1 protein with a purity of at least 60%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% or a nucleic acid molecule encoding KLK1 protein. The purity is determined by electrophoresis (e.g. SDS-PAGE, isoelectric focusing, and capillary electrophoresis), chromatography (e.g. ion exchange chromatography or reversed-phase HPLC).

The term "vector" in the present application refers to a delivery vehicle into which a genetic element can be operatively inserted and expressed, for example, to produce a protein, RNA or DNA encoded by the genetic element, or replicate the genetic element. The vector can be used to transform, transduce or transfect host cells, so that the gene element carried can be expressed in the host cells. For example, the vector comprises: plasmids, phagemids, cosmids, artificial chromosomes such as yeast artificial chromosomes (YAC), bacterial artificial chromosomes (BAC) or P1-derived artificial chromosomes (PAC), bacteriophages such as bacteriophage λ phage or bacteriophage M13, and animal viruses. The vector can comprise a variety of expression control elements, including a promoter sequence, a transcription initiation sequence, an enhancer sequence, a selection element and a reporter gene. Moreover, the vector may also comprise an origin of replication. The vector can also comprise a component that facilitate it to enter cells, including, but not limited to, a virion, a liposome or a capsid. The vector can be an expression vector or a cloning vector. The vector (such as an expression vector) provided in the present application comprises a nucleic acid sequence encoding the KLK1 fusion protein described in the present application, at least one promoter (for example, SV40, CMV, EF-1α) operably linked to the nucleic acid sequence, and at least one selection marker.

As used in the present application, the "treatment" or "therapy" for a certain disease, disorder or condition includes prevention or alleviation of a certain disease, disorder or condition, reducing the speed of occurrence or development of a certain disease, disorder or condition, reducing the risk of developing a certain disease, disorder or condition, preventing or delaying the development of symptoms related to a certain disease, disorder or condition, reducing or terminating symptoms related to a certain disease, disorder or condition, totally or partially reversing a certain disease, disorder or condition, curing a certain disease, disorder or condition, or a combination thereof.

The term "pharmaceutically acceptable" means that the specified carrier, vehicle, diluent, excipient and/or salt are generally chemically and/or physically compatible with other components constituting the preparation and physiologically compatible with its receptor.

### Method for preparing fusion protein

In an aspect, the present application provides a method for preparing a fusion protein comprising tissue kallikrein 1 (KLK1). The method comprises Step (1): expressing a recombinant vector comprising a nucleic acid molecule encoding the fusion protein in a suitable protein expression system. The fusion protein comprises a masking peptide, a first polypeptide and a second polypeptide. The masking peptide, the first polypeptide and the second polypeptide are linked directly or covalently via a linker, and
d) the masking peptide has an amino acid sequence that can be specifically recognized and cleaved by a protease;
e) the first polypeptide comprises KLK1 or a variant thereof; and
f) the second polypeptide is used to extend the half-life of the first polypeptide.

"Tissue kallikrein 1", "kallikrein-1", "KLK1 protein" or "KLK1 enzyme" is a serine protease, a member of tissue kallikrein family, which is encoded by *KLK1* gene, and can catalyze kininogen to release active kinin. In the present application, "tissue kallikrein 1", "Kallikrein-1 ", "KLK1 protein" and "KLK1 enzyme" can be used interchangeably. In the present application, the KLK1 protein may be the KLK1 protein derived from any vertebrate source, including mammals, for example, primates (e.g., humans, and monkeys) and rodents (e.g., mice and rats). An exemplary sequence of human KLK1 protein is as shown in UniProtKB accession number P06870 (having an amino acid sequence as shown in SEQ ID NO: 1). An exemplary sequence of mouse KLK1 protein is as shown in UniProtKB accession number P15947 (having an amino acid sequence as shown in SEQ ID NO: 3).

In the present application, the KLK1 protein comprises KLK1 protein naturally expressed in cells or any variant, conformation, isotype or homologue of KLK1 protein expressed in recombinant cells transfected with *KLK1* gene. For example, the "KLK1 protein" in the present application comprises: 1) naturally unprocessed KLK1 protein, full-length KLK1 protein or naturally occurring KLK1 variant (for example, splicing variant or allelic variant); 2) any form of KLK1 protein produced by processing in cells; or 3) recombinantly produced KLK1 protein in the full-length, fragment (e.g., truncated form, extracellular/transmembrane domain) or modified form (e.g., mutant form, glycosylated/PEGylated form, or immunofluorescence fusion form).

In the present application, the "fusion protein comprising KLK1" (also referred to as "KLK1 fusion protein" in the present application) refers to a single-chain protein formed by operably linking the KLK1 protein to at least one another heterologous peptide. The KLK1 fusion protein of the present application can be produced by a recombinant method, a chemical method or any other method.

In certain embodiments, the method for preparing the fusion protein comprising KLK1 provided in the present application comprises Step (1). In certain embodiments, the method for preparing the fusion protein comprising KLK1 provided in the present application further comprises Step (2). These two steps are described in detail below.

### Step (1)

In Step (1), a recombinant vector comprising a nucleic acid molecule encoding the KLK1 fusion protein is expressed in a suitable protein expression system. The KLK1 fusion protein comprises a masking peptide, a first polypeptide and a second polypeptide. The masking peptide, the first polypeptide and the second polypeptide are linked directly or covalently via a linker, and the masking peptide has an amino acid sequence that can be specifically recognized and cleaved by a protease; the first polypeptide comprises KLK1 or a variant thereof; and the second polypeptide is used to extend the half-life of the first polypeptide.

### a) Masking peptide

In certain embodiments, the masking peptide of the present application has an amino acid sequence that can be specifically recognized and cleaved by a protease. In the present application, the "masking peptide" is covalently connected to the first polypeptide or the second polypeptide described in the present application directly or through a linker, to block, hinder, inhibit or otherwise prevent the binding of the first polypeptide or the second polypeptide described in the present application with its receptor or substrate.

Because the KLK1 enzyme is a mammalian serine protease, it may interfere with the normal function of a protein expression system (for example, host cell) expressing the KLK1 fusion protein, leading to the loss or significant reduction of the activity of the KLK1 fusion protein after expression. The present inventors unexpectedly find that during the preparation of the KLK1 fusion protein, the KLK1 fusion protein comprising the masking peptide is expressed in the protein expression system, and then specifically enzymatically cleaved, to remove the masking peptide. The thus obtained KLK1 fusion protein has higher KLK1 enzyme activity. Without being limited by any theory, it is believed that the masking peptide is helpful to enhance the expression and/or activity of the KLK1 fusion protein, possibly because the existence of the masking peptide avoids or reduces the interference of the KLK1 fusion protein to the protein expression system.

In certain embodiments, the masking peptide has an amino acid sequence that can be specifically recognized and cleaved by a protease, and can block, hinder or inhibit the binding of the KLK1 protein to its substrate. Those skilled in the art can reasonably expect that the masking peptide can be used in the preparation method of the KLK1 fusion protein of the present application as long as it has the above properties.

In certain embodiments, the masking peptide is selected from the group consisting of an enterokinase (EK) recognition sequence, a thrombin recognition sequence, a blood coagulation factor Xa recognition sequence, a tobacco etch virus (TEV) protease recognition sequence, an SUMO protease recognition sequence, and a sortase recognition sequence. In certain embodiments, the masking peptide is an EK recognition sequence. In certain embodiments, the enterokinase (EK) recognition sequence is SEQ ID NO: 4 (DDDDK) or SEQ ID NO: 5 (VDDDDK). In certain embodiments, the masking peptide comprises an amino acid sequence as shown in SEQ ID NO: 5. In certain embodiments, the amino acid sequence of the masking peptide is as shown in SEQ ID NO: 5.

In certain embodiments, the masking peptide is a thrombin recognition sequence. In certain embodiments, the amino acid sequence of the thrombin recognition sequence is as shown in SEQ ID NO: 6.

In certain embodiments, the masking peptide is a blood coagulation factor Xa recognition sequence. In certain embodiments, the amino acid sequence of the blood coagulation factor Xa recognition sequence is as shown in SEQ ID NO: 7.

In certain embodiments, the masking peptide is tobacco etch virus (TEV) protease recognition sequence. In certain embodiments, the amino acid sequence of the tobacco etch virus (TEV) protease recognition sequence is as shown in SEQ ID NO: 8.

In certain embodiments, the masking peptide is an SUMO protease recognition sequence. In certain embodiments, the amino acid sequence of the SUMO protease recognition sequence is as shown in SEQ ID NO: 9.

In certain embodiments, the masking peptide is a sortase recognition sequence. In certain embodiments, the amino acid sequence of the sortase recognition sequence is as shown in SEQ ID NO: 10.

In certain embodiments, the half-life of the KLK1 fusion protein obtained after removing the masking peptide by specific enzymatic cleavage is at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, or at least 14 days.

### b) First polypeptide

In certain embodiments, the first polypeptide is human KLK1 protein or a variant thereof. The "variant" of KLK1 refers to a KLK1 polypeptide which has amino acid mutation, deletion, insertion or modification compared with the amino acid sequence of wild-type KLK1 protein, and yet retains the function or activity of KLK1. In the present application, "human KLK1" is also called "hKLK1".

In certain embodiments, the amino acid sequence of the human wild-type KLK1 protein is as shown in SEQ ID NO: 1, where the polypeptide formed by amino acids at positions 1-18 in SEQ ID NO: 1 is a signal peptide, the polypeptide formed by amino acids at positions 19-24 is a propeptide (also called "prepro" in the present application), and the polypeptide formed by amino acids at positions 25-262 is a mature protein of KLK1. In certain embodiments, after removing the signal peptide at the N-terminus and the propeptide from the amino acid sequence as shown in SEQ ID NO: 1, the amino acid sequence formed is as shown in SEQ ID NO: 2.

In certain embodiments, the first polypeptide comprises an amino acid sequence as shown in SEQ ID NO: 2. In certain embodiments, the amino acid sequence of the first polypeptide is as shown in SEQ ID NO: 2. In certain embodiments, the first polypeptide has an amino acid sequence having at least 90% (e.g., at least 91%, at least 92%, at least 93%, at least 94%, at least 95%, at least 96%, at least 97%, at least 98%, or at least 99%) sequence identity to the amino acid sequence as shown in SEQ ID NO: 2, and yet retains the function or activity of KLK1.

The KLK1 protein of the present application or a variant thereof can also comprise a non-natural amino acid without affecting the activity. The non-natural amino acid comprises, for example, β-fluoroalanine, 1-methylhistidine, γ-methylene glutamic acid, α-methyl leucine, 4,5- dehydrolysine, hydroxyproline, 3-fluorophenylalanine, 3-aminotyrosine, 4-methyl tryptophan and the like.

The KLK1 protein of the present application or a variant thereof may be modified by methods known in the art, for example, but not limited to, PEGylation, glycosylation, N-terminal modification, fatty acylation, C-terminal modification, phosphorylation, and methylation, etc. A person skilled in the art can understand that after modification by methods known in the art, the KLKl protein of the present application or a variant thereof still retains substantially similar functions to the KLK1 protein.

In certain embodiments, on the basis of the amino acid sequence as shown in SEQ ID NO: 2, the KLK1 protein variant of the present application has conservative substitution(s) of amino acid(s) at one or more sites, and yet retains the function or activity of KLK1. In certain embodiments, the KLK1 protein variant provided in the present application has at least 90% (e.g., at least 91%, at least 92%, at least 93%, at least 94%, at least 95%, at least 96%, at least 97%, at least 98%, at least 99%) sequence identity to the amino acid sequence as shown in SEQ ID NO: 2, and still retains the function or activity of KLK1.

In certain embodiments, the KLK1 protein variant of the present application has a mutation at at least one amino acid position selected from the group consisting of positions corresponding to position 121, position 162 and position 164 in SEQ ID NO: 2. In certain embodiments, the KLK1 protein variant of the present application has a mutation at at least one amino acid position selected from the group consisting of positions corresponding to position 121, position 162 and position 164 in SEQ ID NO: 2, and still retains the function or activity of KLK1.

In certain embodiments, the KLK1 protein variant of the present application has a mutation at a position corresponding to position 121 in SEQ ID NO: 2. In certain embodiments, the KLK1 protein variant of the present application has a mutation at a position corresponding to position 121 in SEQ ID NO: 2, and still retains the function or activity of KLK1. In certain embodiments, the KLK1 protein variant of the present application has a mutation of glutamic acid (E) at a position corresponding to position 121 in SEQ ID NO: 2. In certain embodiments, the KLK1 protein variant of the present application has a mutation of glutamic acid (E) at a position corresponding to position 121 in SEQ ID NO: 2, and still retains the function or activity of KLK1. In certain embodiments, the KLK1 protein variant of the present application has a mutation of glutamic acid (E) to glutamine (Q) at a position corresponding to position 121 in SEQ ID NO: 2, which is also called "E121Q" in the present application. In certain embodiments, the KLK1 protein variant of the present application has mutation E121Q, and still retains the function or activity of KLK1. In certain embodiments, the KLK1 protein variant of the present application comprises an amino acid sequence as shown in SEQ ID NO: 117. In certain embodiments, the amino acid sequence of the KLK1 protein variant of the present application is as shown in SEQ ID NO: 117.

In certain embodiments, the KLK1 protein variant of the present application has a mutation at a position corresponding to position 162 in SEQ ID NO: 2. In certain embodiments, the KLK1 protein variant of the present application has a mutation at a position corresponding to position 162 in SEQ ID NO: 2, and still retains the function or activity of KLK1. In certain embodiments, the KLK1 protein variant of the present application has a mutation of lysine (K) at a position corresponding to position 162 in SEQ ID NO: 2. In certain embodiments, the KLK1 protein variant of the present application has a mutation of lysine (K) at a position corresponding to position 162 in SEQ ID NO: 2, and still retains the function or activity of KLK1. In certain embodiments, the KLK1 protein variant of the present application has a mutation of lysine (K) to glutamic acid (E) at a position corresponding to position 162 in SEQ ID NO: 2, which is also called "K162E" in the present application. In certain embodiments, the KLK1 protein variant of the present application has mutation K162E, and still retains the function or activity of KLK1. In certain embodiments, the KLK1 protein variant of the present application comprises an amino acid sequence as shown in SEQ ID NO: 118. In certain embodiments, the amino acid sequence of the KLK1 protein variant of the present application is as shown in SEQ ID NO: 118.

In certain embodiments, the KLK1 protein variant of the present application has a mutation at a position corresponding to position 164 in SEQ ID NO: 2. In certain embodiments, the KLK1 protein variant of the present application has a mutation at a position corresponding to position 164 in SEQ ID NO: 2, and still retains the function or activity of KLK1. In certain embodiments, the KLK1 protein variant of the present application has a mutation of alanine (A) at a position corresponding to position 164 in SEQ ID NO: 2. In certain embodiments, the KLK1 protein variant of the present application has a mutation of alanine (A) at a position corresponding to position 164 in SEQ ID NO: 2, and still retains the function or activity of KLK1. In certain embodiments, the KLK1 protein variant of the present application has a mutation of alanine (A) to valine (V) at a position corresponding to position 164 in SEQ ID NO: 2, which is also called "A164V" in the present application. In certain embodiments, the KLK1 protein variant of the present application has mutation A164V, and still retains the function or activity of KLK1. In certain embodiments, the KLK1 protein variant of the present application comprises an amino acid sequence as shown in SEQ ID NO: 119. In certain embodiments, the amino acid sequence of the KLK1 protein variant of the present application is as shown in SEQ ID NO: 119.

In certain embodiments, the KLK1 protein variant of the present application has mutations at positions corresponding to position 121 and position 162 in SEQ ID NO: 2. In certain embodiments, the KLK1 protein variant of the present application has mutations at positions corresponding to position 121 and position 162 in SEQ ID NO: 2, and still retains the function or activity of KLK1. In certain embodiments, the KLK1 protein variant of the present application has mutations of glutamic acid (E) and lysine (K) at positions corresponding to position 121 and position 162 in SEQ ID NO: 2. In certain embodiments, the KLK1 protein variant of the present application has mutations of glutamic acid (E) and lysine (K) at positions corresponding to position 121 and position 162 in SEQ ID NO: 2, and still retains the function or activity of KLK1. In certain embodiments, the KLK1 protein variant of the present application has a mutation of glutamic acid (E) to glutamine (Q) at a position corresponding to position 121 in SEQ ID NO: 2 and a mutation of lysine (K) to glutamic acid (E) at a position corresponding to position 162 in SEQ ID NO: 2, which are also called "E121Q+ K162E" in the present application. In certain embodiments, the KLK1 protein variant of the present application has mutations E121Q+K162E, and still retains the function or activity of KLK1. In certain embodiments, the KLK1 protein variant of the present application comprises an amino acid sequence as shown in SEQ ID NO: 120. In certain embodiments, the amino acid sequence of the KLK1 protein variant of the present application is as shown in SEQ ID NO: 120.

In certain embodiments, the KLK1 protein variant of the present application has mutations at positions corresponding to position 162 and position 164 in SEQ ID NO: 2. In certain embodiments, the KLK1 protein variant of the present application has mutations at positions corresponding to position 162 and position 164 in SEQ ID NO: 2, and still retains the function or activity of KLK1. In certain embodiments, the KLK1 protein variant of the present application has mutations of lysine (K) and alanine (A) at positions corresponding to position 162 and position 164 in SEQ ID NO: 2. In certain embodiments, the KLK1 protein variant of the present application has mutations of lysine (K) and alanine (A) at positions corresponding to position 162 and position 164 in SEQ ID NO: 2, and still retains the function or activity of KLK1. In certain embodiments, the KLK1 protein variant of the present application has a mutation of lysine (K) to glutamic acid (E) at a position corresponding to position 162 in SEQ ID NO: 2, and a mutation of alanine (A) to valine (V) at a position corresponding to position 164 in SEQ ID NO: 2, which are also called "K162E+ A164V" in the present application. In certain embodiments, the KLK1 protein variant of the present application has mutations K162E+A164V, and still retains the function or activity of KLK1. In certain embodiments, the KLK1 protein variant of the present application comprises an amino acid sequence as shown in SEQ ID NO: 121. In certain embodiments, the amino acid sequence of the KLK1 protein variant of the present application is as shown in SEQ ID NO: 121.

In certain embodiments, the KLK1 protein variant of the present application has mutations at positions corresponding to position 121 and position 164 in SEQ ID NO: 2. In certain embodiments, the KLK1 protein variant of the present application has mutations at positions corresponding to position 121 and position 164 in SEQ ID NO: 2, and still retains the function or activity of KLK1. In certain embodiments, the KLK1 protein variant of the present application has mutations of glutamic acid (E) and alanine (A) at positions corresponding to position 121 and position 164 in SEQ ID NO: 2. In certain embodiments, the KLK1 protein variant of the present application has mutations of glutamic acid (E) and alanine (A) at positions corresponding to position 121 and position 164 in SEQ ID NO: 2, and still retains the function or activity of KLK1. In certain embodiments, the KLK1 protein variant of the present application has a mutation of glutamic acid (E) to glutamine (Q) at a position corresponding to position 121 in SEQ ID NO: 2 and a mutation of alanine (A) to valine (V) at a position corresponding to position 164, which are also called "E121Q+ A164V" in the present application. In certain embodiments, the KLK1 protein variant of the present application has mutations E121Q+A164V, and still retains the function or activity of KLK1. In certain embodiments, the KLK1 protein variant of the present application comprises an amino acid sequence as shown in SEQ ID NO: 115. In certain embodiments, the amino acid sequence of the KLK1 protein variant of the present application is as shown in SEQ ID NO: 115.

In certain embodiments, the KLK1 protein variant of the present application has mutations at positions corresponding to position 121, position 162, and position 164 in SEQ ID NO: 2. In certain embodiments, the KLK1 protein variant of the present application has mutations at positions corresponding to position 121, position 162, and position 164 in SEQ ID NO: 2, and still retains the function or activity of KLK1. In certain embodiments, the KLK1 protein variant of the present application has mutations of glutamic acid (E), lysine (K), and alanine (A) at positions corresponding to position 121, position 162, and position 164 in SEQ ID NO: 2. In certain embodiments, the KLK1 protein variant of the present application has mutations of glutamic acid (E), lysine (K), and alanine (A) at positions corresponding to position 121, position 162, and position 164 in SEQ ID NO: 2, and still retains the function or activity of KLK1. In certain embodiments, the KLK1 protein variant of the present application has a mutation of glutamic acid (E) to glutamine (Q) at a position corresponding to position 121 in SEQ ID NO: 2, a mutation of lysine (K) to glutamic acid (E) at a position corresponding to position 162, and a mutation of alanine (A) to valine (V) at a position corresponding to position 164 in SEQ ID NO: 2, which are also called "E121Q+K162E+A164V" in the present application. In certain embodiments, the KLK1 protein variant of the present application has mutations E121Q+K162E+A164V, and still retains the function or activity of KLK1. In certain embodiments, the KLK1 protein variant of the present application comprises an amino acid sequence as shown in SEQ ID NO: 122. In certain embodiments, the amino acid sequence of the KLK1 protein variant of the present application is as shown in SEQ ID NO: 122.

### c) Second polypeptide

In certain embodiments, the second polypeptide is used to extend the half-life of the first polypeptide. In certain embodiments, the second polypeptide comprises an amino acid sequence serving to extend the half-life of the first polypeptide. In certain embodiments, the second polypeptide comprises an amino acid sequence serving to extend the half-life of the first polypeptide in a subject.

In certain embodiments, the second polypeptide is an Fc domain or albumin. In certain embodiments, the Fc domain comprises a hinge region. In certain embodiments, the Fc domain comprises a lower hinge region. In certain embodiments, the Fc domain comprises a core hinge region and a lower hinge region. In certain embodiments, the Fc domain comprises an upper hinge region, a core hinge region and a lower hinge region. In certain embodiments, the Fc domain does not comprise a hinge region. In certain embodiments, the Fc domain is derived from human IgG Fc domain. In certain embodiments, the Fc domain is derived from human IgG1 Fc domain, human IgG2 Fc domain, human IgG3 Fc domain or human IgG4 Fc domain. In certain embodiments, the Fc domain is derived from human IgG 1 Fc domain or human IgG4 Fc domain.

In certain embodiments, the Fc domain is derived from human IgG1 Fc domain. In certain embodiments, the Fc domain comprises an amino acid sequence as shown in SEQ ID NO: 11. In certain embodiments, the Fc domain consists of an amino acid sequence as shown in SEQ ID NO: 11. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 11. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 11, and yet retains the function of the Fc domain as shown in SEQ ID NO: 11 (for example, ability to extend the half-life of the first polypeptide).

In certain embodiments, the Fc domain is derived from human IgG4 Fc domain. In certain embodiments, the Fc domain comprises an amino acid sequence as shown in SEQ ID NO: 12. In certain embodiments, the Fc domain consists of an amino acid sequence as shown in SEQ ID NO: 12. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 12. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 12, and yet retains the function of the Fc domain as shown in SEQ ID NO: 12 (for example, ability to extend the half-life of the first polypeptide).

In certain embodiments, the Fc domain comprises one or more (for example, two, three, four, five or more) mutations of amino acid sites. In certain embodiments, the Fc domain has an amino acid mutation at position 252 (according to EU numbering) in an amino acid sequence (as shown in SEQ ID NO: 11 or SEQ ID NO: 12) corresponding to human natural IgG Fc domain. In certain embodiments, the Fc domain has a mutation of methionine at position 252 to tyrosine (also referred to as "M252Y" in the present application) in an amino acid sequence (as shown in SEQ ID NO: 11 or SEQ ID NO: 12) corresponding to human natural IgG Fc domain.

In certain embodiments, the Fc domain has an amino acid mutation at position 254 (according to EU numbering) in an amino acid sequence (as shown in SEQ ID NO: 11 or SEQ ID NO: 12) corresponding to human natural IgG Fc domain. In certain embodiments, the Fc domain has a mutation of serine at position 254 to threonine (also referred to as "S254T" in the present application) in an amino acid sequence (as shown in SEQ ID NO: 11 or SEQ ID NO: 12) corresponding to human natural IgG Fc domain.

In certain embodiments, the Fc domain has an amino acid mutation at position 256 (according to EU numbering) in an amino acid sequence (as shown in SEQ ID NO: 11 or SEQ ID NO: 12) corresponding to human natural IgG Fc domain. In certain embodiments, the Fc domain has a mutation of threonine at position 256 to glutamic acid (also referred to as "T256E" in the present application) in an amino acid sequence (as shown in SEQ ID NO: 11 or SEQ ID NO: 12) corresponding to human natural IgG Fc domain.

In certain embodiments, the Fc domain comprises one, two or three mutations selected from the group consisting of M252Y, S254T, and T256E. In certain embodiments, the Fc domain comprises mutations M252Y, S254T and T256E.

In certain embodiments, the Fc domain comprises an amino acid sequence as shown in SEQ ID NO: 13. In certain embodiments, the Fc domain consists of the amino acid sequence as shown in SEQ ID NO: 13. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 13. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 13, and yet retains the function of the Fc domain as shown in SEQ ID NO: 13 (for example, ability to extend the half-life of the first polypeptide).

In certain embodiments, the Fc domain comprises an amino acid sequence as shown in SEQ ID NO: 14. In certain embodiments, the Fc domain consists of the amino acid sequence as shown in SEQ ID NO: 14. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 14. In certain embodiments, the Fc domain comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 14, and yet retains the function of the Fc domain as shown in SEQ ID NO: 14 (for example, ability to extend the half-life of the first polypeptide).

In certain embodiments, the second polypeptide is albumin. In certain embodiments, the amino acid sequence of the albumin is as shown in SEQ ID NO: 15. In certain embodiments, the albumin comprises one or more domains of human serum albumin. In certain embodiments, the albumin comprises D3 domain of human serum albumin.

In certain embodiments, the KLK1 fusion protein of the present application comprises a masking peptide as shown in SEQ ID NO: 5, a first polypeptide as shown in SEQ ID NO: 2 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 2 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 2 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 2 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 2 and a second polypeptide as shown in SEQ ID NO: 15.

In certain embodiments, the KLK1 fusion protein of the present application comprises a masking peptide as shown in SEQ ID NO: 5, a first polypeptide as shown in SEQ ID NO: 115 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 115 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 115 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 115 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 115 and a second polypeptide as shown in SEQ ID NO: 15.

In certain embodiments, the KLK1 fusion protein of the present application comprises a masking peptide as shown in SEQ ID NO: 5, a first polypeptide as shown in SEQ ID NO: 117 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 117 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 117 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 117 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 117 and a second polypeptide as shown in SEQ ID NO: 15.

In certain embodiments, the KLK1 fusion protein of the present application comprises a masking peptide as shown in SEQ ID NO: 5, a first polypeptide as shown in SEQ ID NO: 118 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 118 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 118 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 118 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 118 and a second polypeptide as shown in SEQ ID NO: 15.

In certain embodiments, the KLK1 fusion protein of the present application comprises a masking peptide as shown in SEQ ID NO: 5, a first polypeptide as shown in SEQ ID NO: 119 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 119 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 119 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 119 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 119 and a second polypeptide as shown in SEQ ID NO: 15.

In certain embodiments, the KLK1 fusion protein of the present application comprises a masking peptide as shown in SEQ ID NO: 5, a first polypeptide as shown in SEQ ID NO: 120 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 120 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 120 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 120 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 120 and a second polypeptide as shown in SEQ ID NO: 15.

In certain embodiments, the KLK1 fusion protein of the present application comprises a masking peptide as shown in SEQ ID NO: 5, a first polypeptide as shown in SEQ ID NO: 121 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 121 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 121 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 121 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 121 and a second polypeptide as shown in SEQ ID NO: 15.

In certain embodiments, the KLK1 fusion protein of the present application comprises a masking peptide as shown in SEQ ID NO: 5, a first polypeptide as shown in SEQ ID NO: 122 and a second polypeptide as shown in SEQ ID NO: 11. In certain embodiments, the KLK1 fusion protein of the present application comprises a masking peptide as shown in SEQ ID NO: 5, a first polypeptide as shown in SEQ ID NO: 122 and a second polypeptide as shown in SEQ ID NO: 12. In certain embodiments, the KLK1 fusion protein of the present application comprises a masking peptide as shown in SEQ ID NO: 5, a first polypeptide as shown in SEQ ID NO: 122 and a second polypeptide as shown in SEQ ID NO: 13. In certain embodiments, the KLK1 fusion protein of the present application comprises a masking peptide as shown in SEQ ID NO: 5, a first polypeptide as shown in SEQ ID NO: 122 and a second polypeptide as shown in SEQ ID NO: 14. In certain embodiments, the KLK1 fusion protein of the present application comprises a masking peptide as shown in SEQ ID NO: 5, a first polypeptide as shown in SEQ ID NO: 122 and a second polypeptide as shown in SEQ ID NO: 15.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, and the amino acid sequence of the second polypeptide is as shown in SEQ ID NO: 11.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, and the amino acid sequence of the second polypeptide is as shown in SEQ ID NO: 12.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises , in order from the N-terminus to the C-terminus, a masking peptide, a first polypeptide and a second polypeptide. In certain embodiments, the KLK1 fusion protein comprises , in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, and the amino acid sequence of the second polypeptide is as shown in SEQ ID NO: 13.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, and the amino acid sequence of the second polypeptide is as shown in SEQ ID NO: 14.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, and the amino acid sequence of the second polypeptide is as shown in SEQ ID NO: 15.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, and the amino acid sequence of the second polypeptide is as shown in SEQ ID NO: 11.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, and the amino acid sequence of the second polypeptide is as shown in SEQ ID NO: 12.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, and the amino acid sequence of the second polypeptide is as shown in SEQ ID NO: 13.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, and the amino acid sequence of the second polypeptide is as shown in SEQ ID NO: 14.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 2, and the amino acid sequence of the second polypeptide is as shown in SEQ ID NO: 15.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 115, and the amino acid sequence of the second polypeptide is as shown in SEQ ID NO: 11.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 115, and the amino acid sequence of the second polypeptide is as shown in SEQ ID NO: 12.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 115, and the amino acid sequence of the second polypeptide is as shown in SEQ ID NO: 13.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 115, and the amino acid sequence of the second polypeptide is as shown in SEQ ID NO: 14.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 115, and the amino acid sequence of the second polypeptide is as shown in SEQ ID NO: 15.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 122, and the amino acid sequence of the second polypeptide is as shown in SEQ ID NO: 11.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 122, and the amino acid sequence of the second polypeptide is as shown in SEQ ID NO: 12.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 122, and the amino acid sequence of the second polypeptide is as shown in SEQ ID NO: 13.

In certain embodiments, the second polypeptide is located at the N-terminus of the first polypeptide. In certain embodiments, the second polypeptide is located at the C-terminus of the first polypeptide. In certain embodiments, the KLKl fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 122, and the amino acid sequence of the second polypeptide is as shown in SEQ ID NO: 14.

In certain embodiments, the second polypeptide is located at the N-terminus of the first polypeptide. In certain embodiments, the second polypeptide is located at the C-terminus of the first polypeptide. In certain embodiments, the KLK1 fusion protein comprises, in order from the N-terminus to the C-terminus, a masking peptide, a first polypeptide and a second polypeptide. In certain embodiments, the KLK1 fusion protein comprises, in order from the N-terminus to the C-terminus, a masking peptide, a first polypeptide and a second polypeptide. The amino acid sequence of the masking peptide is as shown in SEQ ID NO: 5, the amino acid sequence of the first polypeptide is as shown in SEQ ID NO: 122, and the amino acid sequence of the second polypeptide is as shown in SEQ ID NO: 15.

In certain embodiments, the KLK1 fusion protein of the present application comprises, from the N-terminus to the C-terminus, a structure of:

MP-L1-P1-L2-P2,

wherein, MP represents a masking peptide, L1 represents a bond or a first linker, P1 represents a first polypeptide, L2 represents a bond or a second linker, and P2 represents a second polypeptide.

In certain embodiments, MP represents a masking peptide, L1 represents a bond, P1 represents a first polypeptide, L2 represents a second linker, and P2 represents a second polypeptide.

In certain embodiments, MP represents an enterokinase recognition sequence, L1 represents a bond, P1 represents a human KLK1 protein, L2 represents a second linker, and P2 represents an Fc domain.

In certain embodiments, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 11.

In certain embodiments, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 12.

In certain embodiments, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 13.

In certain embodiments, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 14.

In certain embodiments, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 15.

In certain embodiments, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 11.

In certain embodiments, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 12.

In certain embodiments, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 13.

In certain embodiments, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 14.

In certain embodiments, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 15.

In certain embodiments, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 11.

In certain embodiments, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 12.

In certain embodiments, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 13.

In certain embodiments, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 14.

In certain embodiments, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 15.

In certain embodiments, the KLK1 fusion protein comprises an amino acid sequence as shown in SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 113 or SEQ ID NO: 127. In certain embodiments, the KLK1 fusion protein consists of an amino acid sequence as shown in SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 113 or SEQ ID NO: 127. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 113 or SEQ ID NO: 127. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 113 or SEQ ID NO: 127, and yet retains the function or activity of KLK1 after enzymatic cleavage with EK (for example, the ability to catalyze kininogen to release active kinin).

### d) Tag

In certain embodiments, the KLK1 fusion protein of the present application further comprises a tag. In certain embodiments, the tag is selected from the group consisting of a fluorescent tag, a luminescent tag, a purification tag, and a chromogenic tag. In certain embodiments, the tag is a purification tag. In certain embodiments, the tag is selected from the group consisting of HIS tag, GST tag, MBP tag, Myc tag, NusA tag, SUMO tag, FLAG tag, Avi tag, Halo tag, SNAP tag, Strep-II tag, and TrX tag. In certain embodiments, the tag is a HIS tag. In certain embodiments, the tag is a HIS tag comprising 5, 6, 7, 8, 9, 10 or more histidine. In certain embodiments, the amino acid sequence of the tag is as shown in SEQ ID NO: 16.

In certain embodiments, the KLK1 fusion protein of the present application comprises, from the N-terminus to the C-terminus, a structure of:

LB-MP-L1-P1-L2-P2,

wherein, LB is absent or represents a tag, MP represents a masking peptide, L1 represents a bond or a first linker, P1 represents a first polypeptide, L2 represents a bond or a second linker, and P2 represents a second polypeptide.

In certain embodiments, LB represents a tag, MP represents a masking peptide, L1 represents a bond, P1 represents a first polypeptide, L2 represents a second linker, and P2 represents a second polypeptide.

In certain embodiments, LB represents HIS tag, MP represents an enterokinase recognition sequence, L1 represents a bond, P1 represents a human KLK1 protein, L2 represents a second linker, and P2 represents an Fc domain.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 11.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 12.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 13.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 14.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 15.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 11.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 12.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 13.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 14.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 15.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 11.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 12.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 13.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 14.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 15.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 11.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 12.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 13.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 14.

In certain embodiments, LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 15.

### e) Linker

In certain embodiments, the first linker and the second linker are each independently selected from the group consisting of a cleavable linker, a non-cleavable linker, a flexible linker, a rigid linker, a helical linker, and a non-helical linker.

In the present application, the term "linker" refers to any chemical moiety capable of covalently linking one moiety to another. For example, the "linker" can be an artificial amino acid sequence with 1, 2, 3, 4 or 5 amino acid residues or having a length of 5, 15, 20, 30, 50 or more amino acid residues connected by a peptide bond, which is used to connect one or more polypeptides. The linker may or may not have a secondary structure. The linker sequences is known in the art, for example, see Holliger et al., Proc. Natl. Acad. Sci. USA 90:6444-6448 (1993); and Poljak et al., Structure 2:1121-1123 (1994).

In the present application, the term "cleavable linker" refers to a linker that is sensitive to factors such as protease, pH or chemistry in vivo, and readily cleavable in the presence of the above factors.

In the present application, the term "non-cleavable linker" refers to a linker that is not sensitive to factors such as protease, pH or chemistry in vivo, and not readily cleavable.

In the present application, the term "flexible linker" refers to a linker that can increase the spatial extensibility when used to connect different protein moieties, so that the spatial folding and conformation of the protein moieties are not affected by each other as much as possible.

In the present application, the term "rigid linker" refers to a linker that can maintain a fixed distance between protein moieties when used to connect different protein moieties.

In the present application, the term "helical linker" refers to a linker in which a rigid unit can form a helix (e.g., alpha-helix) by itself or with the same adjacent sequence, so that the formed fusion protein has a relatively stable three-dimensional conformation.

In the present application, the term "non-helical linker" refers to a linker that cannot form a helical structure.

In certain embodiments, the flexible linker comprises a linker comprising glycine and serine. In certain embodiments, the linker comprising glycine and serine comprises one, two, three, four or more repeats of the sequence as shown in SEQ ID NO: 17, SEQ ID NO: 18, SEQ ID NO: 19, SEQ ID NO: 20, SEQ ID NO: 21 or SEQ ID NO: 22. In certain embodiments, the first linker or the second linker comprises an amino acid sequence as shown in SEQ ID NO: 20, SEQ ID NO: 21 or SEQ ID NO: 22. In certain embodiments, the first linker or the second linker consists of an amino acid sequence as shown in SEQ ID NO: 20, SEQ ID NO: 21 or SEQ ID NO: 22.

In certain embodiments, the rigid linker comprises one, two, three, four or more repeats of the sequence as shown in SEQ ID NO: 23. In certain embodiments, the first linker or the second linker comprises an amino acid sequence as shown in SEQ ID NO: 23. In certain embodiments, the first linker or the second linker consists of an amino acid sequence as shown in SEQ ID NO: 23.

In certain embodiments, the KLK1 fusion protein comprises an amino acid sequence as shown in SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 112 or SEQ ID NO: 126. In certain embodiments, the KLK1 fusion protein consists of an amino acid sequence as shown in SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 112 or SEQ ID NO: 126. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 112 or SEQ ID NO: 126. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 112 or SEQ ID NO: 126, and yet retains the function or activity of KLK1 after enzymatic cleavage with EK (for example, the ability to catalyze kininogen to release active kinin).

### f) Signal peptide

In certain embodiments, the KLK1 fusion protein further comprises a signal peptide.

As used in the present application, the term "signal peptide" refers to a sequence of amino acid residues that can participate in the secretion or targeted transport of a mature or precursor form of a protein. The signal peptide is usually located at the N-terminus of the precursor or mature protein sequence. The signal peptide may be endogenous or exogenous. Generally, there is no signal peptide in a mature protein. Typically, after protein transport, the signal peptide is cleaved off from the protein by a signal peptidase.

In certain embodiments, the signal peptide is exogenous. In certain embodiments, the signal peptide is located at the N-terminus of the KLK1 fusion protein. In certain embodiments, the signal peptide comprises an amino acid sequence as shown in SEQ ID NO: 24 or SEQ ID NO: 25. In certain embodiments, the amino acid sequence of the signal peptide is as shown in SEQ ID NO: 24 or SEQ ID NO: 25, or has at least 70%, at least 75%, at least 80%, at least 85%, at least 90% or at least 95% sequence identity to the amino acid sequence as shown in SEQ ID NO: 24 or SEQ ID NO: 25. In certain embodiments, the signal peptide consists of an amino acid sequence as shown in SEQ ID NO: 24. In certain embodiments, the signal peptide consists of an amino acid sequence as shown in SEQ ID NO: 25.

In certain embodiments, the KLK1 fusion protein of the present application comprises, from the N-terminus to the C-terminus, a structure of the follows:

SP-L3-LB-MP-L1-P1-L2-P2,

wherein, SP is absent or represents a signal peptide, L3 represents a bond or a third linker, LB is absent or represents a tag, MP represents a masking peptide, L1 represents a bond or a first linker, P1 represents a first polypeptide, L2 represents a bond or a second linker, and P2 represents a second polypeptide.

In certain embodiments, SP represents a signal peptide as shown in SEQ ID NO: 24 or SEQ ID NO: 25, L3 represents a linker formed by two glycine (GG), LB represents a tag, MP represents a masking peptide, L1 represents a bond, P1 represents a first polypeptide, L2 represents a second linker, and P2 represents a second polypeptide.

In certain embodiments, SP represents a signal peptide as shown in SEQ ID NO: 24 or SEQ ID NO: 25, L3 represents a linker formed by two glycine (GG), LB represents HIS tag, MP represents an enterokinase recognition sequence, L1 represents a bond, P1 represents a human KLK1 protein, L2 represents a second linker, and P2 represents an Fc domain.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NOs: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 11.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NOs: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 12.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NOs: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 13.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NOs: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 14.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NOs: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 15.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 11.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 12.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 13.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 14.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 15.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 11.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 12.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 13.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 14.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 15.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 11.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 12.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 13.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 14.

In certain embodiments, SP represents a signal peptide as shown in SEQ ID NO: 24 or SEQ ID NO: 25, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 15.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 11.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 12.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 13.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 14.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents a second polypeptide as shown in SEQ ID NO: 15.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 11.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 12.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 13.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 14.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 2, L2 represents a second linker, and P2 represents a second polypeptide as shown in SEQ ID NO: 15.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 11.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 12.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 13.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 14.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 115, L2 represents a second linker, and P2 represents a second polypeptide as shown in SEQ ID NO: 15.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 11.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 12.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 13.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 14.

In certain embodiments, SP is absent, L3 represents a linker formed by two glycine (GG), LB represents HIS tag as shown in SEQ ID NO: 16, MP represents a masking peptide as shown in SEQ ID NO: 5, L1 represents a bond, P1 represents a first polypeptide as shown in SEQ ID NO: 122, L2 represents a second linker, and P2 represents a second polypeptide as shown in SEQ ID NO: 15.

In certain embodiments, the KLK1 fusion protein comprises an amino acid sequence as shown in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 110 or SEQ ID NO: 124. In certain embodiments, the KLK1 fusion protein consists of an amino acid sequence as shown in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 110 or SEQ ID NO: 124. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 110 or SEQ ID NO: 124. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 110 or SEQ ID NO: 124, and yet retains the function or activity of KLK1 after enzymatic cleavage with EK (for example, the ability to catalyze kininogen to release active kinin).

In certain embodiments, the KLK1 fusion protein comprises an amino acid sequence as shown in SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 111 or SEQ ID NO: 125. In certain embodiments, the KLK1 fusion protein consists of an amino acid sequence as shown in SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 111 or SEQ ID NO: 125. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 111 or SEQ ID NO: 125. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 111 or SEQ ID NO: 125, and yet retains the function or activity of KLK1 after enzymatic cleavage with EK (for example, the ability to catalyze kininogen to release active kinin).

### g) Nucleic acid

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 77, and a nucleotide sequence as shown in SEQ ID NO: 78.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 77, and a nucleotide sequence as shown in SEQ ID NO: 79.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 77, and a nucleotide sequence as shown in SEQ ID NO: 80.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 77, and a nucleotide sequence as shown in SEQ ID NO: 81.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 123, and a nucleotide sequence as shown in SEQ ID NO: 78.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 123, and a nucleotide sequence as shown in SEQ ID NO: 79.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 123, and a nucleotide sequence as shown in SEQ ID NO: 80.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 123, and a nucleotide sequence as shown in SEQ ID NO: 81.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 130, and a nucleotide sequence as shown in SEQ ID NO: 78.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 130, and a nucleotide sequence as shown in SEQ ID NO: 79.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 130, and a nucleotide sequence as shown in SEQ ID NO: 80.

In certain embodiments, in the method for preparing the KLK1 fusion protein provided in the present application, the nucleic acid molecule encoding the KLK1 fusion protein comprises a nucleotide sequence as shown in SEQ ID NO: 76, a nucleotide sequence as shown in SEQ ID NO: 130, and a nucleotide sequence as shown in SEQ ID NO: 81.

In certain embodiments, in the method for preparing the KLK1 fusion protein provided in the present application, the nucleic acid molecule encoding the KLK1 fusion protein further comprises a nucleotide sequence as shown in SEQ ID NO: 82.

In certain embodiments, in the method for preparing the KLK1 fusion protein provided in the present application, the nucleic acid molecule encoding the KLK1 fusion protein further comprises a nucleotide sequence as shown in SEQ ID NO: 83, SEQ ID NO: 84, SEQ ID NO: 85 or SEQ ID NO: 86.

In certain embodiments, in the method for preparing the KLK1 fusion protein provided in the present application, the nucleic acid molecule encoding the KLK1 fusion protein further comprises a nucleotide sequence as shown in SEQ ID NO: 87 or SEQ ID NO: 88.

In certain embodiments, in the method for preparing the KLK1 fusion protein provided in the present application, the nucleic acid molecule encoding the KLK1 fusion protein comprises a nucleotide sequence having at least 70%, at least 75%, at least 80%, at least 85%, at least 90%, or at least 95% sequence identity to the nucleotide sequence as shown in SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 116 or SEQ ID NO: 129. In certain embodiments, in the method for preparing the KLK1 fusion protein provided in the present application, the nucleic acid molecule encoding the KLK1 fusion protein comprises a nucleotide sequence as shown in SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 116 or SEQ ID NO: 129. in the method for preparing the KLK1 fusion protein provided in the present application, the nucleic acid molecule encoding the KLK1 fusion protein consists of a nucleotide sequence as shown in SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 116 or SEQ ID NO: 129.

As used herein, the term "nucleic acid" or "nucleotide" refers to single-stranded or double-stranded deoxyribonucleic acid (DNA) or ribonucleic acid (RNA) and polymers thereof. Unless otherwise indicated, a specific nucleotide sequence also implicitly covers a conservatively modified variant thereof (for example, with degenerate codon substitution), an allele, an ortholog, SNP, and a complementary sequence, as well as an explicitly indicated sequence. Specifically, the degenerate codon substitution can be achieved by generating a sequence where the third position of one or more selected (or all) codons is/are substituted with mixed bases and/or deoxyinosine residues (see Batzer et al., Nucleic Acid Res. 19: 5081 (1991); Ohtsuka et al., J. Biol. Chem. 260:2605-2608 (1985); and Rossolini et al., Mol. Cell. Probes 8:91-98 (1994)).

The nucleic acid molecule encoding the KLK1 fusion protein can be easily isolated and sequenced using conventional procedures (for example, by using an oligonucleotide probe that can specifically bind to the gene encoding the fusion protein). The coding DNA can also be synthesized.

### h) Vector and protein expression system

In certain embodiments, in the method for preparing the KLK1 fusion protein provided in the present application, the recombinant vector comprising the nucleic acid molecule encoding the KLK1 fusion protein is selected from the group consisting of a plasmid, an artificial chromosome, and a viral vector.

The nucleic acid molecule encoding the KLK1 fusion protein can be inserted into a vector by using recombinant techniques well known in the art, for further cloning (amplification of DNA) or for expression. Many vectors are available. The vector component usually comprises, but is not limited to, one or more of a signal sequence, an origin of replication, one or more marker genes, an enhancer element, a promoter (such as SV40, CMV, and EF-1α) and a transcription termination sequence.

Examples of the vector comprise, but are not limited to, retroviruses (including lentiviruses), adenoviruses, adeno-associated viruses, herpes viruses (such as herpes simplex viruses), poxviruses, baculovirus, papillomaviruses, papovaviruses (such as SV40), λ phage and M13 phage, pcDNA plasmid (e.g. pcDNA3.4 plasmid), pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM., pCDM8, pCDNA1.1/amp, pcDNA3.1, pRc/RSV, PCR 2.1, pEF-1, pFB, pSG5, pXT1, pCDEF3, pSVSPORT, or pEF-Bos.

In certain embodiments, the protein expression system used in the method for preparing the KLK1 fusion protein provided in present application is a cell expression system. In certain embodiments, the cell expression system is selected from the group consisting of a bacterium, a fungus, a plant or animal cell.

In certain embodiments, the bacterium is selected from the group consisting of *Bacilli, Clostridia, Corynebacterium, Cupriavidus, Delftia, Escherichia, Enterobacter, Erwinia, Klebsiella, Lactobacillus, Lactococcus, Proteus, Pseudomonas, Rhodococcus, Salmonella, Serratia, Shigella,* and *Streptomyces.* In certain embodiments, the bacterium is selected from the group consisting of *Escherichia coli, Salmonella typhimurium, Serratia marcescens, Bacillus subtilis, B. licheniformis,* and *P. aeruginosa.* In certain embodiments, the cell is *E. coli.*

In certain embodiments, the fungus is selected from the group consisting of *Candida, Saccharomyces cerevisiae, Kluyveromyces, Pichia pastoris,* and *Schizosaccharomyces pombe.*

In certain embodiments, the animal cell is a mammalian cell. In certain embodiments, the mammalian cell is a human cell or Chinese hamster ovary (CHO) cell. In certain embodiments, the mammalian cell is a CHO-K1 cell or CHO-S cell. In certain embodiments, the mammalian cell is a human embryonic kidney 293 cell (HEK293 cell).

### Step (2)

In Step (2), the KLK1 fusion protein obtained in Step (1) is mixed with a protease to perform a specific enzymatic cleavage reaction, thereby removing the masking peptide. In certain embodiments, the KLK1 fusion protein obtained in Step (1) is purified before performing the specific enzymatic cleavage reaction in Step (2).

Without being limited by any theory, it is believed that the KLK1 fusion protein comprising the masking peptide is an intermediate produced in the process of preparing the active KLK1 fusion protein, and does not have the activity of KLK1 enzyme itself. For example, it does not have the activity of cleaving kininogen to release kinin. However, when the KLK1 fusion protein comprising the masking peptide is mixed with a protease capable of specifically recognizing and cleaving the masking peptide for specific cleavage to remove the masking peptide, the formed KLK1 fusion protein (e.g., KLK1-Fc fusion protein) has KLKl enzyme activity. For example, it can catalyze the cleavage of kininogen to release kinin. In certain embodiments, the KLK1 fusion protein formed after removing the masking peptide still retains the function or activity of wild-type KLK1 (e.g., still retains the enzymatic activity of cleaving kininogen to release kinin). Those skilled in the art can determine the enzymatic activity of the KLK1 fusion protein by methods known in the art, for example, the method described in Example 4 of the present application. In certain embodiments, the KLK1 fusion protein prepared by the method described in the present application has an activity of cleaving kininogen to release kinin in the range of 0.3-3 PNAU/mg (e.g.0.4 PNAU/mg, 0.5 PNAU/mg, 0.6 PNAU/mg, 0.7 PNAU/mg, 0.8 PNAU/mg, 0.9 PNAU/mg, 1 PNAU/mg, 1.5 PNAU/mg, 2 PNAU/mg, or 2.5 PNAU/mg).

In certain embodiments, the KLK1 fusion protein obtained in Step (1) is mixed with the protease to perform a specific enzymatic cleavage reaction, and the KLK1 fusion protein, obtained by removing the masking peptide, comprises an amino acid sequence as shown in SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 114 or SEQ ID NO: 128. In certain embodiments, the KLK1 fusion protein consists of an amino acid sequence as shown in SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 114 or SEQ ID NO: 128. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 114 or SEQ ID NO: 128. In certain embodiments, the KLK1 fusion protein has an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence as shown in SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 114 or SEQ ID NO: 128, and yet retains the function or activity of KLK1 (for example, the ability to catalyze kininogen to release active kinin).

### Fusion protein

In an aspect, the present application provides a fusion protein comprising a masking peptide, a first polypeptide and a second polypeptide, wherein, the masking peptide, the first polypeptide and the second polypeptide are linked directly or covalently via a linker, and,
a) the masking peptide has an amino acid sequence that can be specifically recognized and cleaved by a protease;
b) the first polypeptide comprises KLK1 or a variant thereof; and
c) the second polypeptide is used to extend the half-life of the first polypeptide.

The masking peptide, the first polypeptide and the second polypeptide described in the "method for preparing fusion protein" section of the present application are also applicable to the masking peptide, the first polypeptide and the second polypeptide in the "fusion protein" section, and thus are not repeated here.

In certain embodiments, the masking peptide is selected from the group consisting of an enterokinase (EK) recognition sequence, a thrombin recognition sequence, a blood coagulation factor Xa recognition sequence, a tobacco etch virus (TEV) protease recognition sequence, an SUMO protease recognition sequence, and a sortase recognition sequence. In certain embodiments, the masking peptide is an EK recognition sequence. In certain embodiments, the amino acid sequence of the masking peptide is as shown in SEQ ID NO: 5 (VDDDDK).

In certain embodiments, the first polypeptide is human KLK1 protein or a variant thereof. In certain embodiments, the first polypeptide is human KLK1 protein. In certain embodiments, the first polypeptide has an amino acid sequence having at least 90% (e.g., at least 91%, at least 92%, at least 93%, at least 94%, at least 95%, at least 96%, at least 97%, at least 98% or at least 99%) sequence identity to the amino acid sequence as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, and yet retains the function or activity of KLK1. In certain embodiments, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122. In certain embodiments, the first polypeptide has an amino acid sequence having at least 90% (e.g., at least 91%, at least 92%, at least 93%, at least 94%, at least 95%, at least 96%, at least 97%, at least 98% or at least 99%) sequence identity to the amino acid sequence as shown in SEQ ID NO: 2, and yet retains the function or activity of KLK1. In certain embodiments, the amino acid sequence of the first polypeptide is as shown in SEQ ID NO: 2. In certain embodiments, the first polypeptide has an amino acid sequence having at least 90% (e.g., at least 91%, at least 92%, at least 93%, at least 94%, at least 95%, at least 96%, at least 97%, at least 98% or at least 99%) sequence identity to the amino acid sequence as shown in SEQ ID NO: 115, and yet retains the function or activity of KLK1. In certain embodiments, the amino acid sequence of the first polypeptide is as shown in SEQ ID NO: 115. In certain embodiments, the first polypeptide has an amino acid sequence having at least 90% (e.g., at least 91%, at least 92%, at least 93%, at least 94%, at least 95%, at least 96%, at least 97%, at least 98% or at least 99%) sequence identity to the amino acid sequence as shown in SEQ ID NO: 122, and yet retains the function or activity of KLK1. In certain embodiments, the amino acid sequence of the first polypeptide is as shown in SEQ ID NO: 122.

In certain embodiments, the second polypeptide is an Fc domain or albumin. In certain embodiments, the amino acid sequence of the second polypeptide is as shown in SEQ ID NO: 11, SEQ ID NO: 12, SEQ ID NO: 13, SEQ ID NO: 14 or SEQ ID NO: 15.

In certain embodiments, the KLK1 fusion protein further comprises a tag. In certain embodiments, the KLK1 fusion protein further comprises HIS tag. In certain embodiments, the KLK1 fusion protein further comprises HIS tag consisting of 5, 6, 7, 8, 9, 10 or more histidine.

In certain embodiments, the KLK1 fusion protein comprises, from the N-terminus to the C-terminus, a structure of:

LB-MP-L1-P1-L2-P2,

wherein, LB is absent or represents a tag, MP represents the masking peptide, L1 represents a bond or a first linker, P1 represents the first polypeptide, L2 represents a bond or a second linker, and P2 represents the second polypeptide.

In certain embodiments, the KLK1 fusion protein comprises, from the N-terminus to the C-terminus, a structure of:

LB-MP-L1-P1-L2-P2,

wherein, LB represents a tag, MP represents a masking peptide, L1 represents a bond, P1 represents a first polypeptide, L2 represents a second linker, and P2 represents a second polypeptide.

In certain embodiments, the KLK1 fusion protein comprises, from the N-terminus to the C-terminus, a structure of:

LB-MP-L1-P1 -L2-P2,

wherein, LB represents HIS tag, MP represents an enterokinase recognition sequence, L1 represents a bond, P1 represents a human KLK1 protein, L2 represents a second linker, and P2 represents an Fc domain.

In certain embodiments, the KLK1 fusion protein comprises, from the N-terminus to the C-terminus, a structure of:

LB-MP-L1-P1-L2-P2,

wherein, LB represents HIS tag, MP represents an amino acid sequence as shown in SEQ ID NO: 5, L1 represents a bond, P1 represents an amino acid sequence as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, L2 represents a second linker, and P2 represents an amino acid sequence as shown in SEQ ID NO: 11, SEQ ID NO: 12, SEQ ID NO: 13, SEQ ID NO: 14 or SEQ ID NO: 15.

In certain embodiments, the KLK1 fusion protein comprises, from the N-terminus to the C-terminus, a structure of:

LB-MP-L1-P1-L2-P2,

wherein, LB represents HIS tag, MP represents an amino acid sequence as shown in SEQ ID NO: 5, L1 represents a bond, P1 represents an amino acid sequence as shown in SEQ ID NO: 2, L2 represents a second linker, and P2 represents an amino acid sequence as shown in SEQ ID NO: 11, SEQ ID NO: 12, SEQ ID NO: 13, SEQ ID NO: 14 or SEQ ID NO: 15.

In certain embodiments, the KLK1 fusion protein comprises, from the N-terminus to the C-terminus, a structure of:

LB-MP-L1-P1-L2-P2,

wherein, LB represents HIS tag, MP represents an amino acid sequence as shown in SEQ ID NO: 5, L1 represents a bond, P1 represents an amino acid sequence as shown in SEQ ID NO: 115, L2 represents a second linker, and P2 represents an amino acid sequence as shown in SEQ ID NO: 11, SEQ ID NO: 12, SEQ ID NO: 13, SEQ ID NO: 14 or SEQ ID NO: 15.

In certain embodiments, the KLK1 fusion protein comprises, from the N-terminus to the C-terminus, a structure of:

LB-MP-L1-P1-L2-P2,

wherein, LB represents HIS tag, MP represents an amino acid sequence as shown in SEQ ID NO: 5, L1 represents a bond, P1 represents an amino acid sequence as shown in SEQ ID NO: 122, L2 represents a second linker, and P2 represents an amino acid sequence as shown in SEQ ID NO: 11, SEQ ID NO: 12, SEQ ID NO: 13, SEQ ID NO: 14 or SEQ ID NO: 15.

In certain embodiments, the KLK1 fusion protein comprises, from the N-terminus to the C-terminus, a structure of:

LB-MP-L1-P1-L2-P2,

wherein, LB represents HIS tag, MP represents an amino acid sequence as shown in SEQ ID NO: 5, L1 represents a bond, P1 represents an amino acid sequence as shown in SEQ ID NO: 2, L2 represents an amino acid sequence as shown in SEQ ID NO: 17, SEQ ID NO: 18, SEQ ID NO: 19, SEQ ID NO: 20, SEQ ID NO: 21, SEQ ID NO: 22 or SEQ ID NO: 23, P2 represents an amino acid sequence as shown in SEQ ID NO: 11, SEQ ID NO: 12, SEQ ID NO: 13, SEQ ID NO: 14 or SEQ ID NO: 15.

In certain embodiments, the KLK1 fusion protein comprises, from the N-terminus to the C-terminus, a structure of:

LB-MP-L1-P1-L2-P2,

wherein, LB represents HIS tag, MP represents an amino acid sequence as shown in SEQ ID NO: 5, L1 represents a bond, P1 represents an amino acid sequence as shown in SEQ ID NO: 115, L2 represents an amino acid sequence as shown in SEQ ID NO: 17, SEQ ID NO: 18, SEQ ID NO: 19, SEQ ID NO: 20, SEQ ID NO: 21, SEQ ID NO: 22 or SEQ ID NO: 23, P2 represents an amino acid sequence as shown in SEQ ID NO: 11, SEQ ID NO: 12, SEQ ID NO: 13, SEQ ID NO: 14 or SEQ ID NO: 15.

In certain embodiments, the KLK1 fusion protein comprises, from the N-terminus to the C-terminus, a structure of:

LB-MP-L1-P1-L2-P2,

wherein, LB represents HIS tag, MP represents an amino acid sequence as shown in SEQ ID NO: 5, L1 represents a bond, P1 represents an amino acid sequence as shown in SEQ ID NO: 122, L2 represents an amino acid sequence as shown in SEQ ID NO: 17, SEQ ID NO: 18, SEQ ID NO: 19, SEQ ID NO: 20, SEQ ID NO: 21, SEQ ID NO: 22 or SEQ ID NO: 23, P2 represents an amino acid sequence as shown in SEQ ID NO: 11, SEQ ID NO: 12, SEQ ID NO: 13, SEQ ID NO: 14 or SEQ ID NO: 15.

In certain embodiments, the KLK1 fusion protein comprises an amino acid sequence as shown in any one of SEQ ID NOs: 26-65, 100-114, and 124-128. In certain embodiments, the KLK1 fusion protein consists of an amino acid sequence as shown in any one of SEQ ID NO: 26-65, 110-114, and 124-128.

### Nucleic acid

In another aspect, the present application provides an isolated nucleic acid molecule comprising a nucleotide sequence encoding the KLK1 fusion protein of the present application.

In certain embodiments, the nucleic acid molecule provided in the present application comprises:
a) a nucleotide sequence as shown in SEQ ID NO: 76, a nucleotide sequence as shown in SEQ ID NO: 77, and a nucleotide sequence as shown in SEQ ID NO: 78;
b) a nucleotide sequence as shown in SEQ ID NO: 76, a nucleotide sequence as shown in SEQ ID NO: 77, and a nucleotide sequence as shown in SEQ ID NO: 79;
c) a nucleotide sequence as shown in SEQ ID NO: 76, a nucleotide sequence as shown in SEQ ID NO: 77, and a nucleotide sequence as shown in SEQ ID NO: 80;
d) a nucleotide sequence as shown in SEQ ID NO: 76, a nucleotide sequence as shown in SEQ ID NO: 77, and a nucleotide sequence as shown in SEQ ID NO: 81;
e) a nucleotide sequence as shown in SEQ ID NO: 76, a nucleotide sequence as shown in SEQ ID NO: 123, and a nucleotide sequence as shown in SEQ ID NO: 78;
f) a nucleotide sequence as shown in SEQ ID NO: 76, a nucleotide sequence as shown in SEQ ID NO: 123, and a nucleotide sequence as shown in SEQ ID NO: 79;
g) a nucleotide sequence as shown in SEQ ID NO: 76, a nucleotide sequence as shown in SEQ ID NO: 123, and a nucleotide sequence as shown in SEQ ID NO: 80;
h) a nucleotide sequence as shown in SEQ ID NO: 76, a nucleotide sequence as shown in SEQ ID NO: 123, and a nucleotide sequence as shown in SEQ ID NO: 81;
i) a nucleotide sequence as shown in SEQ ID NO: 76, a nucleotide sequence as shown in SEQ ID NO: 130, and a nucleotide sequence as shown in SEQ ID NO: 78;
j) a nucleotide sequence as shown in SEQ ID NO: 76, a nucleotide sequence as shown in SEQ ID NO: 130, and a nucleotide sequence as shown in SEQ ID NO: 79;
k) a nucleotide sequence as shown in SEQ ID NO: 76, a nucleotide sequence as shown in SEQ ID NO: 130, and a nucleotide sequence as shown in SEQ ID NO: 80; or
l) a nucleotide sequence as shown in SEQ ID NO: 76, a nucleotide sequence as shown in SEQ ID NO: 130, and a nucleotide sequence as shown in SEQ ID NO: 81.

In certain embodiments, the nucleic acid molecule provided in the present application further comprises a nucleotide sequence as shown in SEQ ID NO: 82. In certain embodiments, the nucleic acid molecule provided in the present application further comprises a nucleotide sequence as shown in SEQ ID NO: 83, SEQ ID NO: 84, SEQ ID NO: 85 or SEQ ID NO: 86. In certain embodiments, the nucleic acid molecule provided in the present application further comprises a nucleotide sequence as shown in SEQ ID NO: 87 or SEQ ID NO: 88.

In certain embodiments, the nucleic acid molecule provided in the present application comprises a nucleotide sequence as shown in SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 116 or SEQ ID NO: 129, or has at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity thereto. In certain embodiments, the nucleic acid molecule provided in the present application consists of a nucleotide sequence as shown in SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 116 or SEQ ID NO: 129.

### Vector and cell

In another aspect, the present application provides a vector comprising the nucleic acid molecule encoding the KLK1 fusion protein of the present application.

In another aspect, the present application further provides a protein expression system comprising the nucleic acid molecule encoding the KLK1 fusion protein of the present application or the vector of the present application.

In another aspect, the present application further provides a method for constructing a protein expression system comprising: introducing the nucleic acid molecule encoding the KLK1 fusion protein of the present application into a vector to construct an expression vector; and introducing the expression vector into a protein expression system.

An isolated polynucleotide encoding the KLK1 fusion protein can be inserted into a vector by using recombinant techniques well known in the art, for further cloning (amplification of DNA) or for expression. Many vectors are available. The vector component usually comprises, but is not limited to, one or more of a signal sequence, an origin of replication, one or more marker genes, an enhancer element, a promoter (such as SV40, CMV, and EF-1α) and a transcription termination sequence.

In certain embodiments, the vector provided in the present application comprises the nucleic acid encoding the KLK1 fusion protein, at least one promoter operably linked to the nucleic acid sequence (for example, SV40, CMV, and EF-1α), and at least one selection tag. Examples of the vector comprise, but are not limited to, retroviruses (including lentiviruses), adenoviruses, adeno-associated viruses, herpes viruses (such as herpes simplex viruses), poxviruses, baculovirus, papillomaviruses, papovaviruses (such as SV40), λ phage and M13 phage, plasmid pcDNA3.4, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM., pCDM8, pCDNA1.1/amp, pcDNA3.1, pRc/RSV, PCR 2.1, pEF-1, pFB, pSG5, pXT1, pCDEF3, pSVSPORT, or pEF-Bos.

The vector comprising the nucleic acid sequence encoding the KLK1 fusion protein can be introduced into a host cell for cloning or gene expression. The host cells suitable for cloning or expressing DNA in the vector described in the present application comprise the above-mentioned prokaryotic cells, yeast or higher eukaryotic cells. The prokaryotic cells suitable for use in the present application comprise eubacteria, such as gram-negative bacteria or gram-positive bacteria, for example, *Enterobacteriaceae*, for example, *Escherichia* (for example, *E. coli*), *Enterobacter*, *Erwinia*, *Klebsiella*, *Proteus*, *Salmonella* (for example, *Salmonella typhimurium*), *Serratia* (for example, *Serratia marcescans*), *Shigella, Bacilli* (for example, *B. subtilis* and *B. licheniformis*), *Pseudomonas* (for example, *P. aeruginosa*, and *Streptomyces*. In certain embodiments, the cell is *E. coli.*

In addition to prokaryotic cells, eukaryotic cells, for example, eukaryotic microorganisms such as filamentous fungi or yeast are suitable cloning or expression hosts for the vector expressing the fusion protein. *Saccharomyces cerevisiae* or baker's yeast is the most commonly used lower eukaryotic host microorganism. However, many other genera, species and strains are commonly used and applicable in the present application, for example, *Schizosaccharomyces pombe; Kluyveromyces* host, for example, *K. lactis*, *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans* and *K. marxianus*; *Yarrowia* (EP 402,226); *Pichia pastoris* (EP 183,070); *Candida*; *Trichoderma reesia* (EP 244,234); *Neurospora crassa*; *Schwanniomyces,* for example, *Schwanniomyces occidentalis*; *filamentous fungi*, for example, *Neurospora*, *Penicillium*, *Tolypocladium* and *Aspergillus* (for example, *A. nidulans* and *A. niger*). In certain embodiments, the eukaryotic cell is a mammalian cell. In certain embodiments, the mammalian cell is a human cell or Chinese hamster ovary (CHO) cell. In certain embodiments, the mammalian cell is a CHO-K1 cell or CHO-S cell. In certain embodiments, the mammalian cell is a human embryonic kidney 293 cell (HEK293 cell).

### Pharmaceutical composition

In another aspect, the present application provides a pharmaceutical composition comprising the KLK1 fusion protein of the present application, the nucleic acid of the present application, the vector of the present application or the cell of the present application, and a pharmaceutically acceptable carrier.

The pharmaceutically acceptable carrier for the pharmaceutical composition disclosed in the present application may comprise, for example, a pharmaceutically acceptable liquid, gel or solid carrier, an aqueous solvent, a non-aqueous solvent, an antimicrobial substance, an isotonic substance, a buffer, an antioxidant, an anesthetic, a suspending agent/dispersant, a chelating agent, a diluent, an adjuvant, an auxiliary material or non-toxic auxiliary substance, and other components known in the art or various combinations thereof.

Suitable components may comprise, for example, an antioxidant. a filler, a binder, a disintegrant, a buffer, a preservative, a lubricant, a flavoring agent, a thickener, a colorant, an emulsifier or a stabilizer such as sugars and cyclodextrins. Suitable antioxidants may comprise, for example, methionine, ascorbic acid, EDTA, sodium thiosulfate, platinum, hydrogen peroxidase, citric acid, cysteine, mercaptoglycerol, mercaptoacetic acid, mercaptosorbitol, butyl methyl anisole, butylated hydroxytoluene and/or propyl gallate. As disclosed in the present application, the presence of one or more antioxidants such as methionine in the composition comprising the KLK1 fusion protein disclosed in the present application can reduce the oxidation of the KLK1 fusion protein. The present application further provides various methods for preventing the KLK1 fusion protein from oxidation, prolonging its shelf life and/or improving its activity, for example, by mixing the KLK1 fusion protein provided in the present application with one or more antioxidants (e.g., methionine).

Further, the pharmaceutically acceptable carrier may comprise, for example, an aqueous medium such as sodium chloride injection, Ringer's solution for injection, isotonic glucose injection, sterile water for injection, or glucose and lactated Ringer's injection, a non-aqueous medium such as plant-derived non-volatile oil, cottonseed oil, corn oil, sesame oil, or peanut oil, an antibacterial substance at bacterial or fungal inhibition concentration, an isotonic agent such as sodium chloride or glucose, a buffer such as a phosphate or citrate buffer, an antioxidant such as sodium bisulfate, a local anesthetic such as procaine hydrochloride, a suspending and dispersing agent such as sodium carboxymethyl cellulose, hydroxypropyl methylcellulose or polyvinylpyrrolidone, an emulsifier such as polysorbate 80 (Tween -80), a chelating agent such as EDTA (ethylene diamine tetraacetic acid) or EGTA (ethylene glycol bis (2-aminoethyl ether)tetraacetic acid), ethanol, polyethylene glycol, propylene glycol, sodium hydroxide, hydrochloric acid, citric acid or lactic acid. Antimicrobial agents as carriers can be added to the pharmaceutical composition in a multi-dose container, including phenols or cresols, mercury preparations, benzyl alcohol, chlorobutanol, methyl and propyl parabens, thiomersal, chlorobenzyltrimethylammonium and chlorobenzyldiethylammonium. Suitable adjuvants may comprise, for example, water, a salt, glucose, glycerol or ethanol. Suitable non-toxic auxiliary substances may comprise, for example, a moisturizer, an emulsifier, a pH buffer, a stabilizer, a solubilizer, or substances such as sodium acetate, sorbitan laurate, triethanolamine oleate or cyclodextrin.

The pharmaceutical composition may be a liquid solution, a suspension, an emulsion, pills, capsules, tablets, a sustained release preparation or a powder. The oral preparation may comprise a standard carrier such as pharmaceutical-grade mannitol, lactose, starch, magnesium stearate, polyvinylpyrrolidone, saccharin sodium, cellulose, and magnesium carbonate, etc.

In certain embodiments, the pharmaceutical composition is prepared into an injectable composition. The injectable pharmaceutical composition can be prepared in any conventional form, for example, a liquid solvent, a suspension, an emulsion or a solid form suitable for producing a liquid solvent, a suspension or an emulsion. The injectable preparation may comprise sterile and/or pyrogen-free solutions for immediate use, sterile dry soluble substances combined with solvents before use, such as freeze-dried powders, including hypodermic tablets, sterile suspensions ready for injection, sterile dry insoluble products combined with a medium immediately before use, and sterile and/or pyrogen-free emulsions. The solvent can be aqueous or non-aqueous.

In certain embodiments, the unit dose of injectable preparation is packaged in an ampoule, a vial or a syringe with a needle. It is well known in the art that all preparations for injection should be sterile and pyrogen-free.

In certain embodiments, the sterile freeze-dried powder can be prepared by dissolving the KLK1 fusion protein disclosed in the present application in an appropriate solvent. The solvent may comprise an additional pharmacological component that can improve the stability of the powder or a reconstituted solution prepared from the powder, or improve the powder or the reconstituted solution. Suitable adjuvants comprise, but are not limited to, water, glucose, sorbitol, fructose, corn syrup, xylitol, glycerol, glucose, sucrose or other suitable substances. The solvent may comprise a buffer, such as a citric acid buffer, a sodium phosphate or potassium phosphate buffer or other buffers known to those skilled in the art. In an embodiment, the pH of the buffer is neutral. Under the standard conditions well known in the art, the dissolution is done, followed by sterilization by filtration and then freeze-drying, to prepare a desirable preparation. In an embodiment, the obtained solvent is packaged in vials for freeze-drying. Each vial can accommodate a single dose or multiple doses of the KLK1 fusion protein or its composition. The amount filled in each vial can be slightly higher than that required for each dose or multiple doses (for example, 10% excess), to ensure the accurate sampling and accurate administration. The freeze-dried powder can be stored under appropriate conditions, for example, at a temperature in the range of about 4°C to room temperature.

The freeze-dried powder is re-dissolved in water for injection to obtain a preparation for administration by injection. In an embodiment, the freeze-dried powder can be added to and re-dissolved in sterile pyrogen-free water or other suitable liquid carriers. The exact amount depends on the selected therapy, and can be determined empirically.

### Method for treating or preventing diseases

In another aspect, the present application provides a method for treating or preventing a disease, comprising administering the KLK1 fusion protein of the present application (for example, isolated KLK1 fusion protein prepared by the method according to the present application) to a subject in need thereof. The disease is selected from the group consisting of an ischemic disease, a hemorrhagic disease, a kidney disease, a metabolic disease, and a neurodegenerative disease.

In another aspect, the present application provides use of the KLK1 fusion protein of the present application (for example, isolated KLK1 fusion protein prepared by the method according to the present application) in the preparation of a drug for treating or preventing an ischemic disease, a hemorrhagic disease, a kidney disease, a metabolic disease, and a neurodegenerative disease. In certain embodiments, the present application provides use of the KLK1 fusion protein of the present application (for example, isolated KLK1 fusion protein prepared by the method according to the present application) in the preparation of a drug for treating or preventing an ischemic disease, a hemorrhagic disease, a kidney disease, a metabolic disease, and a neurodegenerative disease, wherein the treatment or prevention comprises administering the drug to a subject in need thereof.

In another aspect, the present application provides use of the KLK1 fusion protein of the present application (for example, isolated KLK1 fusion protein prepared by the method according to the present application) in treating or preventing diseases. The disease is selected from the group consisting of an ischemic disease, a hemorrhagic disease, a kidney disease, a metabolic disease, and a neurodegenerative disease.

In certain embodiments, the ischemic disease is selected from the group consisting of cerebral ischemia, ischemic stroke, myocardial ischemia, ischemic colitis, critical limb ischemia, skin ischemia, ischemic stroke, transient ischemic attack (TIA), and traumatic brain injury (TBI). In certain embodiments, the ischemic disease is acute ischemic stroke. In certain embodiments, the ischemic disease is mild to moderate acute thrombotic cerebral infarction.

In certain embodiments, the hemorrhagic disease is hemorrhagic stroke. In certain embodiments, the hemorrhagic stroke is intracerebral hemorrhagic stroke or subarachnoid hemorrhagic stroke.

In certain embodiments, the kidney disease is chronic nephropathy, diabetic nephropathy or acute renal injury.

In certain embodiments, the metabolic disease is selected from the group consisting of diabetes (e.g., diabetes type 1, and diabetes type 2), obesity, insulin resistance, hyperglycemia and hyperinsulinemia.

In certain embodiments, the neurodegenerative disease is selected from the group consisting of Alzheimer's disease, motor neuron disease, Huntington's disease and Parkinson's disease.

SEQ ID NOs and the descriptions, and corresponding amino acid sequences or nucleotide sequences mentioned in the present application are shown in Table 1.

**Table 1. SEQ ID NOs and the descriptions as well as amino acid or nucleotide sequences**

| **SEQ ID NO:** | **Description** | **Amino acid or nucleotide sequence** |
|---|---|---|
| 1 | Human wild-type KLK1 protein (UniProtKB Accession No. P06870) | MWFLVLCLALSLGGTGAAPPIQSRIVGGWECEQHSQ PWQAALYHFSTFQCGGILVHRQWVLTAAHCISDNYQ LWLGRHNLFDDENTAQFVHVSESFPHPGFNMSLLEN HTRQADEDYSHDLMLLRLTEPADTITDAVKVVELPTE EPEVGSTCLASGWGSIEPENFSFPDDLQCVDLKILPND ECKKAHVQKVTDFMLCVGHLEGGKDTCVGDSGGPL MCDGVLQGVTSWGYVPCGTPNKPSVAVRVLSYVKW IEDTIAENS |
| 2 | Human mature KLK1 protein (AA25-262) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENS |
| 3 | Mouse wild-type KLK1 protein (UniProtKB Accession No. P15947) | MRFLILFLALSLGGIDAAPPVQSRIVGGFNCEKNSQP WQVAVYRFTKYQCGGILLNANWVLTAAHCHNDKYQ VWLGKNNFLEDEPSAQHRLVSKAIPHPDFNMSLLNE HTPQPEDDYSNDLMLLRLKKPADITDVVKPIDLPTEE |
| | | PKLGSTCLASGWGSITPVKYEYPDELQCVNLKLLPNE DCAKAHIEKVTDDMLCAGDMDGGKDTCAGDSGGP LICDGVLQGITSWGPSPCGKPNVPGIYTRVLNFNTWI RETMAEND |
| 4 | EK recognition sequence 1 | DDDDK |
| 5 | EK recognition sequence 2 | VDDDDK |
| 6 | Thrombin recognition sequence | LVPRGS |
| 7 | Blood coagulation factor Xa recognition sequence | IEGRG |
| 8 | Tobacco etch virus (TEV) protease recognition sequence | ENLYFQG |
| 9 | SUMO protease recognition sequence | |
| 10 | Sortase recognition sequence | LPXTG (X is any amino acid) |
| 11 | IgG1 Fc | TCPPCPAPELLGGPSVFLFPPKPKDTLMISRTPEVTCV VVDVSHEDPEVKFNWYVDGVEVHNAKTKPREEQYN STYRVVSVLTVLHQDWLNGKEYKCKVSNKALPAPIE KTISKAKGQPREPQVYTLPPSRDELTKNQVSLTCLVK GFYPSDIAVEWESNGQPENNYKTTPPVLDSDGSFFLY SKLTVDKSRWQQGNVFSCSVMHEALHNHYTQKSLS LSPGK |
| 12 | IgG4 Fc | GPPCPSCPAPEFLGGPSVFLFPPKPKDTLMISRTPEVTC VVVDVSQEDPEVQFNWYVDGVEVHNAKTKPREEQF NSTYRVVSVLTVLHQDWLNGKEYKCKVSNKGLPSSI EKTISKAKGQPREPQVYTLPPSQEEMTKNQVSLTCLV KGFYPSDIAVEWESNGQPENNYKTTPPVLDSDGSFFL YSRLTVDKSRWQEGNVFSCSVMHEALHNHYTQKSLS LSLGK |
| 13 | IgG1 Fc (having YTE mutation) | TCPPCPAPELLGGPSVFLFPPKPKDTLYITREPEVTCV VVDVSHEDPEVKFNWYVDGVEVHNAKTKPREEQYN STYRVVSVLTVLHQDWLNGKEYKCKVSNKALPAPIE KTISKAKGQPREPQVYTLPPSRDELTKNQVSLTCLVK GFYPSDIAVEWESNGQPENNYKTTPPVLDSDGSFFLY SKLTVDKSRWQQGNVFSCSVMHEALHNHYTQKSLS LSPGK |
| 14 | IgG4 Fc (having YTE mutation) | GPPCPSCPAPEFLGGPSVFLFPPKPKDTLYITREPEVTC VVVDVSQEDPEVQFNWYVDGVEVHNAKTKPREEQF NSTYRVVSVLTVLHQDWLNGKEYKCKVSNKGLPSSI EKTISKAKGQPREPQVYTLPPSQEEMTKNQVSLTCLV KGFYPSDIAVEWESNGQPENNYKTTPPVLDSDGSFFL YSRLTVDKSRWQEGNVFSCSVMHEALHNHYTQKSLS LSLGK |
| 15 | Albumin | |
| 16 | HIS tag | HHHHHH |
| 17 | Linker | GGGS |
| 18 | Linker | GGGGS |
| 19 | Linker | GGGGGS |
| 20 | Linker | GGGGSGGGGS |
| 21 | Linker | GGGSSSS |
| 22 | Linker | GSSGSSG |
| 23 | Linker | EAAAK |
| 24 | Signal peptide | MWFLVLCLALSLGGTGAA |
| 25 | IL-2 signal peptide | MYRMQLLSCIALSLALVTNS |
| 26 | SP-GG-His-VDDDDK-hKLK1-IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL QCVDLKILPNDECKKAHVQKVTDFMLCVGHLEGGK DTCVGDSGGPLMCDGVLQGVTSWGYVPCGTPNKPS VAVRVLSYVKWIEDTIAENSTCPPCPAPELLGGPSVFL FPPKPKDTLMISRTPEVTCVVVDVSHEDPEVKFNWY VDGVEVHNAKTKPREEQYNSTYRVVSVLTVLHQDW LNGKEYKCKVSNKALPAPIEKTISKAKGQPREPQVYT LPPSRDELTKNQVSLTCLVKGFYPSDIAVEWESNGQP ENNYKTTPPVLDSDGSFFLYSKLTVDKSRWQQGNVF SCSVMHEALHNHYTQKSLSLSPGK |
| 27 | SP-GG-His-VDDDDK-hKLK1-GGGSSSS-IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL QCVDLKILPNDECKKAHVQKVTDFMLCVGHLEGGK DTCVGDSGGPLMCDGVLQGVTSWGYVPCGTPNKPS VAVRVLSYVKWIEDTIAENSGGGSSSSTCPPCPAPELL GGPSVFLFPPKPKDTLMISRTPEVTCVVVDVSHEDPE VKFNWYVDGVEVHNAKTKPREEQYNSTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 28 | SP-GG-His-VDDDDK-hKLK1-EAAAK-IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL QCVDLKILPNDECKKAHVQKVTDFMLCVGHLEGGK DTCVGDSGGPLMCDGVLQGVTSWGYVPCGTPNKPS VAVRVLSYVKWIEDTIAENSEAAAKTCPPCPAPELLG GPSVFLFPPKPKDTLMISRTPEVTCVVVDVSHEDPEV KFNWYVDGVEVHNAKTKPREEQYNSTYRVVSVLTV LHQDWLNGKEYKCKVSNKALPAPIEKTISKAKGQPR EPQVYTLPPSRDELTKNQVSLTCLVKGFYPSDIAVEW ESNGQPENNYKTTPPVLDSDGSFFLYSKLTVDKSRWQ QGNVFSCSVMHEALHNHYTQKSLSLSPGK |
| 29 | IL2-GG-His-VDDDDK-hKLK1-GGGSSSS-IgG1 Fc | MYRMQLLSCIALSLALVTNSGGHHHHHHVDDDDKI VGGWECEQHSQPWQAALYHFSTFQCGGILVHRQWV LTAAHCISDNYQLWLGRHNLFDDENTAQFVHVSESFP HPGFNMSLLENHTRQADEDYSHDLMLLRLTEPADTIT DAVKVVELPTEEPEVGSTCLASGWGSIEPENFSFPDD LQCVDLKILPNDECKKAHVQKVTDFMLCVGHLEGG KDTCVGDSGGPLMCDGVLQGVTSWGYVPCGTPNKP SVAVRVLSYVKWIEDTIAENSGGGSSSTCPPCPAPEL LGGPSVFLFPPKPKDTLMISRTPEVTCVVVDVSHEDP EVKFNWYVDGVEVHNAKTKPREEQYNSTYRVVSVL TVLHQDWLNGKEYKCKVSNKALPAPIEKTISKAKGQ PREPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 30 | IL2-GG-His-VDDDDK-hKLK1-EAAAK-IgG1 Fc | MYRMQLLSCIALSLALVTNSGGHHHHHHVDDDDKI VGGWECEQHSQPWQAALYHFSTFQCGGILVHRQWV LTAAHCISDNYQLWLGRHNLFDDENTAQFVHVSESFP HPGFNMSLLENHTRQADEDYSHDLMLLRLTEPADTIT DAVKVVELPTEEPEVGSTCLASGWGSIEPENFSFPDD LQCVDLKILPNDECKKAHVQKVTDFMLCVGHLEGG KDTCVGDSGGPLMCDGVLQGVTSWGYVPCGTPNKP SVAVRVLSYVKWIEDTIAENSEAAAKTCPPCPAPELLG GPSVFLFPPKPKDTLMISRTPEVTCVVVDVSHEDPEV KFNWYVDGVEVHNAKTKPREEQYNSTYRVVSVLTV LHQDWLNGKEYKCKVSNKALPAPIEKTISKAKGQPR EPQVYTLPPSRDELTKNQVSLTCLVKGFYPSDIAVEW ESNGQPENNYKTTPPVLDSDGSFFLYSKLTVDKSRWQ QGNVFSCSVMHEALHNHYTQKSLSLSPGK |
| 31 | SP-GG-His-VDDDDK-hKLK1-GSSGSSG-IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL QCVDLKILPNDECKKAHVQKVTDFMLCVGHLEGGK DTCVGDSGGPLMCDGVLQGVTSWGYVPCGTPNKPS VAVRVLSYVKWIEDTIAENSGSSGSSGTCPPCPAPELL GGPSVFLFPPKPKDTLMISRTPEVTCVVVDVSHEDPE VKFNWYVDGVEVHNAKTKPREEQYNSTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 32 | SP-GG-His-VDDDDK-hKLK1-GSSGSSG-YTE IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL |
| | | QCVDLKILPNDECKKAHVQKVTDFMLCVGHLEGGK DTCVGDSGGPLMCDGVLQGVTSWGYVPCGTPNKPS VAVRVLSYVKWIEDTIAENSGSSGSSGTCPPCPAPELL GGPSVFLFPPKPKDTLYITREPEVTCVVVDVSHEDPE VKFNWYVDGVEVHNAKTKPREEQYNSTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 33 | SP-GG-His-VDDDDK-hKLK1-GSSGSSG-IgG4 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL QCVDLKILPNDECKKAHVQKVTDFMLCVGHLEGGK DTCVGDSGGPLMCDGVLQGVTSWGYVPCGTPNKPS VAVRVLSYVKWIEDTIAENSGSSGSSGGPPCPSCPAPE FLGGPSVFLFPPKPKDTLMISRTPEVTCVVVDVSQED PEVQFNWYVDGVEVHNAKTKPREEQFNSTYRVVSV LTVLHQDWLNGKEYKCKVSNKGLPSSIEKTISKAKG QPREPQVYTLPPSQEEMTKNQVSLTCLVKGFYPSDIA VEWESNGQPENNYKTTPPVLDSDGSFFLYSRLTVDKS RWQEGNVFSCSVMHEALHNHYTQKSLSLSLGK |
| 34 | SP-GG-His-VDDDDK-hKLK1-GSSGSSG-YTE IgG4 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL QCVDLKILPNDECKKAHVQKVTDFMLCVGHLEGGK DTCVGDSGGPLMCDGVLQGVTSWGYVPCGTPNKPS VAVRVLSYVKWIEDTIAENSGSSGSSGGPPCPSCPAPE FLGGPSVFLFPPKPKDTLYITREPEVTCVVVDVSQEDP EVQFNWYVDGVEVHNAKTKPREEQFNSTYRVVSVL TVLHQDWLNGKEYKCKVSNKGLPSSIEKTISKAKGQ PREPQVYTLPPSQEEMTKNQVSLTCLVKGFYPSDIAV EWESNGQPENNYKTTPPVLDSDGSFFLYSRLTVDKSR WQEGNVFSCSVMHEALHNHYTQKSLSLSLGK |
| 35 | SP-GG-His-VDDDDK-hKLK1-GGGGSGGGGS-IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTEEPEVGSTCLASGWGSIEPENFSFPDDL QCVDLKILPNDECKKAHVQKVTDFMLCVGHLEGGK DTCVGDSGGPLMCDGVLQGVTSWGYVPCGTPNKPS VAVRVLSYVKWIEDTIAENSGGGGSGGGGSTCPPCPA PELLGGPSVFLFPPKPKDTLMISRTPEVTCVVVDVSHE DPEVKFNWYVDGVEVHNAKTKPREEQYNSTYRVVS |
| | | VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGK |
| 36 | His-VDDDDK-hKLK1-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENST CPPCPAPELLGGPSVFLFPPKPKDTLMISRTPEVTCVV VDVSHEDPEVKFNWYVDGVEVHNAKTKPREEQYNS TYRVVSVLTVLHQDWLNGKEYKCKVSNKALPAPIEK TISKAKGQPREPQVYTLPPSRDELTKNQVSLTCLVKG FYPSDIAVEWESNGQPENNYKTTPPVLDSDGSFFLYS KLTVDKSRWQQGNVFSCSVMHEALHNHYTQKSLSL SPGK |
| 37 | His-VDDDDK-hKLK1-GGGSSSS-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSG GGSSSSTCPPCPAPELLGGPSVFLFPPKPKDTLMISRTP EVTCVVVDVSHEDPEVKFNWYVDGVEVHNAKTKPR EEQYNSTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSDG SFFLYSKLTVDKSRWQQGNVFSCSVMHEALHNHYTQ KSLSLSPGK |
| 38 | His-VDDDDK-hKLK1-EAAAK-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSE AAAKTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSRDELTKNQVSLT CLVKGFYPSDIAVEWESNGQPENNYKTTPPVLDSDGS FFLYSKLTVDKSRWQQGNVFSCSVMHEALHNHYTQ KSLSLSPGK |
| 39 | His-VDDDDK-hKLK1-GGGSSSS-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSG GGSSSSTCPPCPAPELLGGPSVFLFPPKPKDTLMISRTP EVTCVVVDVSHEDPEVKFNWYVDGVEVHNAKTKPR EEQYNSTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSDG SFFLYSKLTVDKSRWQQGNVFSCSVMHEALHNHYTQ KSLSLSPGK |
| 40 | His-VDDDDK-hKLK1-EAAAK-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSE AAAKTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSRDELTKNQVSLT CLVKGFYPSDIAVEWESNGQPENNYKTTPPVLDSDGS FFLYSKLTVDKSRWQQGNVFSCSVMHEALHNHYTQ KSLSLSPGK |
| 41 | His-VDDDDK-hKLK1-GSSGSSG-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSG SSGSSGTCPPCPAPELLGGPSVFLFPPKPKDTLMISRTP EVTCVVVDVSHEDPEVKFNWYVDGVEVHNAKTKPR EEQYNSTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSDG SFFLYSKLTVDKSRWQQGNVFSCSVMHEALHNHYTQ KSLSLSPGK |
| 42 | His-VDDDDK-hKLK1-GSSGSSG-YTE IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSG SSGSSGTCPPCPAPELLGGPSVFLFPPKPKDTLYITREP EVTCVVVDVSHEDPEVKFNWYVDGVEVHNAKTKPR EEQYNSTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSDG SFFLYSKLTVDKSRWQQGNVFSCSVMHEALHNHYTQ KSLSLSPGK |
| 43 | His-VDDDDK-hKLK1-GSSGSSG-IgG4 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSG SSGSSGGPPCPSCPAPEFLGGPSVFLFPPKPKDTLMISR TPEVTCVVVDVSQEDPEVQFNWYVDGVEVHNAKTK PREEQFNSTYRVVSVLTVLHQDWLNGKEYKCKVSN KGLPSSIEKTISKAKGQPREPQVYTLPPSQEEMTKNQ VSLTCLVKGFYPSDIAVEWESNGQPENNYKTTPPVLD SDGSFFLYSRLTVDKSRWQEGNVFSCSVMHEALHNH YTQKSLSLSLGK |
| 44 | His-VDDDDK-hKLK1-GSSGSSG-YTE IgG4 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSG SSGSSGGPPCPSCPAPEFLGGPSVFLFPPKPKDTLYITR EPEVTCVVVDVSQEDPEVQFNWYVDGVEVHNAKTK PREEQFNSTYRVVSVLTVLHQDWLNGKEYKCKVSN KGLPSSIEKTISKAKGQPREPQVYTLPPSQEEMTKNQ VSLTCLVKGFYPSDIAVEWESNGQPENNYKTTPPVLD SDGSFFLYSRLTVDKSRWQEGNVFSCSVMHEALHNH YTQKSLSLSLGK |
| 45 | His-VDDDDK-hKLK1-GGGGSGGGGS-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTEEPEVGSTCLAS GWGSIEPENFSFPDDLQCVDLKILPNDECKKAHVQK VTDFMLCVGHLEGGKDTCVGDSGGPLMCDGVLQG VTSWGYVPCGTPNKPSVAVRVLSYVKWIEDTIAENSG GGGSGGGGSTCPPCPAPELLGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALHN HYTQKSLSLSPGK |
| 46 | VDDDDK-hKLK1-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSTCPPCPAPE LLGGPSVFLFPPKPKDTLMISRTPEVTCVVVDVSHED PEVKFNWYVDGVEVHNAKTKPREEQYNSTYRVVSV LTVLHQDWLNGKEYKCKVSNKALPAPIEKTISKAKG QPREPQVYTLPPSRDELTKNQVSLTCLVKGFYPSDIAV EWESNGQPENNYKTTPPVLDSDGSFFLYSKLTVDKSR WQQGNVFSCSVMHEALHNHYTQKSLSLSPGK |
| 47 | VDDDDK-hKLK1-GGGSSSS-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSGGGSSSSTC PPCPAPELLGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLSP GK |
| 48 | VDDDDK-hKLK1-EAAAK-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSEAAAKTCP PCPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K |
| 49 | VDDDDK-hKLK1-GGGSSSS-IgG 1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSGGGSSSSTC PPCPAPELLGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLSP GK |
| 50 | VDDDDK-hKLK1-EAAAK-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSEAAAKTCP PCPAPELLGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K |
| 51 | VDDDDK-hKLK1-GSSGSSG-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSGSSGSSGTC PPCPAPELLGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLSP GK |
| 52 | VDDDDK-hKLK1-GSSGSSG-YTE IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSGSSGSSGTC PPCPAPELLGGPSVFLFPPKPKDTLYITREPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLSP GK |
| 53 | VDDDDK-hKLK1-GSSGSSG-IgG4 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSGSSGSSGGP PCPSCPAPEFLGGPSVFLFPPKPKDTLMISRTPEVTCV VVDVSQEDPEVQFNWYVDGVEVHNAKTKPREEQFN STYRVVSVLTVLHQDWLNGKEYKCKVSNKGLPSSIE KTISKAKGQPREPQVYTLPPSQEEMTKNQVSLTCLVK GFYPSDIAVEWESNGQPENNYKTTPPVLDSDGSFFLY SRLTVDKSRWQEGNVFSCSVMHEALHNHYTQKSLSL SLGK |
| 54 | VDDDDK-hKLK1-GSSGSSG-YTE IgG4 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSGSSGSSGGP PCPSCPAPEFLGGPSVFLFPPKPKDTLYITREPEVTCVV VDVSQEDPEVQFNWYVDGVEVHNAKTKPREEQFNS TYRVVSVLTVLHQDWLNGKEYKCKVSNKGLPSSIEK TISKAKGQPREPQVYTLPPSQEEMTKNQVSLTCLVKG FYPSDIAVEWESNGQPENNYKTTPPVLDSDGSFFLYS RLTVDKSRWQEGNVFSCSVMHEALHNHYTQKSLSLS LGK |
| 55 | VDDDDK-hKLK1-GGGGSGGGGS-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTEEPEVGSTCLASGWGSIEP ENFSFPDDLQCVDLKILPNDECKKAHVQKVTDFMLC VGHLEGGKDTCVGDSGGPLMCDGVLQGVTSWGYV PCGTPNKPSVAVRVLSYVKWIEDTIAENSGGGGSGGG GSTCPPCPAPELLGGPSVFLFPPKPKDTLMISRTPEVTC VVVDVSHEDPEVKFNWYVDGVEVHNAKTKPREEQY NSTYRVVSVLTVLHQDWLNGKEYKCKVSNKALPAPI EKTISKAKGQPREPQVYTLPPSRDELTKNQVSLTCLV KGFYPSDIAVEWESNGQPENNYKTTPPVLDSDGSFFL YSKLTVDKSRWQQGNVFSCSVMHEALHNHYTQKSL SLSPGK |
| 56 | hKLK1-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSTCPPCPAPELLGGPS VFLFPPKPKDTLMISRTPEVTCVVVDVSHEDPEVKFN WYVDGVEVHNAKTKPREEQYNSTYRVVSVLTVLHQ DWLNGKEYKCKVSNKALPAPIEKTISKAKGQPREPQ VYTLPPSRDELTKNQVSLTCLVKGFYPSDIAVEWESN GQPENNYKTTPPVLDSDGSFFLYSKLTVDKSRWQQG NVFSCSVMHEALHNHYTQKSLSLSPGK |
| 57 | hKLK1-GGGSSSS-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSGGGSSSSTCPPCPAP ELLGGPSVFLFPPKPKDTLMISRTPEVTCVVVDVSHE DPEVKFNWYVDGVEVHNAKTKPREEQYNSTYRVVS VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGK |
| 58 | hKLK1-EAAAK-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSEAAAKTCPPCPAPEL LGGPSVFLFPPKPKDTLMISRTPEVTCVVVDVSHEDP EVKFNWYVDGVEVHNAKTKPREEQYNSTYRVVSVL TVLHQDWLNGKEYKCKVSNKALPAPIEKTISKAKGQ PREPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 59 | hKLK1-GGGSSSS-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSGGGSSSTCPPCPAP ELLGGPSVFLFPPKPKDTLMISRTPEVTCVVVDVSHE DPEVKFNWYVDGVEVHNAKTKPREEQYNSTYRVVS VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGK |
| 60 | hKLK1-EAAAK-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSEAAAKTCPPCPAPEL LGGPSVFLFPPKPKDTLMISRTPEVTCVVVDVSHEDP |
| | | EVKFNWYVDGVEVHNAKTKPREEQYNSTYRVVSVL TVLHQDWLNGKEYKCKVSNKALPAPIEKTISKAKGQ PREPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 61 | hKLK1-GSSGSSG-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSGSSGSSGTCPPCPAP ELLGGPSVFLFPPKPKDTLMISRTPEVTCVVVDVSHE DPEVKFNWYVDGVEVHNAKTKPREEQYNSTYRVVS VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGK |
| 62 | hKLK1-GSSGSSG-Fc (YTE IgG1) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSGSSGSSGTCPPCPAP ELLGGPSVFLFPPKPKDTLYITREPEVTCVVVDVSHED PEVKFNWYVDGVEVHNAKTKPREEQYNSTYRVVSV LTVLHQDWLNGKEYKCKVSNKALPAPIEKTISKAKG QPREPQVYTLPPSRDELTKNQVSLTCLVKGFYPSDIAV EWESNGQPENNYKTTPPVLDSDGSFFLYSKLTVDKSR WQQGNVFSCSVMHEALHNHYTQKSLSLSPGK |
| 63 | hKLK1-GSSGSSG-IgG4 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSGSSGSSGGPPCPSCP APEFLGGPSVFLFPPKPKDTLMISRTPEVTCVVVDVS QEDPEVQFNWYVDGVEVHNAKTKPREEQFNSTYRV VSVLTVLHQDWLNGKEYKCKVSNKGLPSSIEKTISKA KGQPREPQVYTLPPSQEEMTKNQVSLTCLVKGFYPSD IAVEWESNGQPENNYKTTPPVLDSDGSFFLYSRLTVD KSRWQEGNVFSCSVMHEALHNHYTQKSLSLSLGK |
| 64 | hKLK1-GSSGSSG-Fc (YTE IgG4) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSGSSGSSGGPPCPSCP APEFLGGPSVFLFPPKPKDTLYITREPEVTCVVVDVSQ EDPEVQFNWYVDGVEVHNAKTKPREEQFNSTYRVV SVLTVLHQDWLNGKEYKCKVSNKGLPSSIEKTISKAK GQPREPQVYTLPPSQEEMTKNQVSLTCLVKGFYPSDI AVEWESNGQPENNYKTTPPVLDSDGSFFLYSRLTVDK SRWQEGNVFSCSVMHEALHNHYTQKSLSLSLGK |
| 65 | hKLK1-GGGGSGGGGS-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKAHVQKVTDFMLCVGHLE GGKDTCVGDSGGPLMCDGVLQGVTSWGYVPCGTPN KPSVAVRVLSYVKWIEDTIAENSGGGGSGGGGSTCPP CPAPELLGGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K |
| 66 | SP-GG-His-VDDDDK-hKLK1-IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA |
| | | AGCCTTCTGTCGCCGTCAGAGTGCTGTCTTATGTGA AGTGGATCGAGGACACCATAGCGGAGAACTCCACA TGCCCACCGTGCCCAGCACCTGAACTCCTGGGGGG ACCGTCAGTCTTCCTCTTCCCCCCAAAACCCAAGG ACACCCTCATGATCTCCCGGACCCCTGAGGTCACAT GCGTGGTGGTGGACGTGAGCCACGAAGACCCTGA GGTCAAGTTCAACTGGTACGTGGACGGCGTGGAGG TGCATAATGCCAAGACAAAGCCGCGGGAGGAGCA GTACAACAGCACGTACCGGGTGGTCAGCGTCCTCA CCGTCCTGCACCAGGACTGGCTGAATGGCAAGGAG TACAAGTGCAAGGTCTCCAACAAAGCCCTCCCAGC CCCCATCGAGAAAACCATCTCCAAAGCCAAAGGGC AGCCCCGAGAACCACAGGTGTACACCCTGCCCCCA TCCCGGGATGAGCTGACCAAGAACCAGGTCAGCCT GACCTGCCTGGTCAAAGGCTTCTATCCCAGCGACA TCGCCGTGGAGTGGGAGAGCAATGGGCAGCCGGA GAACAACTACAAGACCACGCCTCCCGTGCTGGACT CCGACGGCTCCTTCTTCCTCTACAGCAAGCTCACC GTGGACAAGAGCAGGTGGCAGCAGGGGAACGTCT TCTCATGCTCCGTGATGCATGAGGCTCTGCACAACC ACTACACGCAGAAGAGCCTCTCCCTGTCTCCGGGT AAATGA |
| 67 | SP-GG-His-VDDDDK-hKLK1-GGGSSSS-IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA GGCGGTTCAAGTTCTAGCACATGCCCACCGTGCCC AGCACCTGAACTCCTGGGGGGACCGTCAGTCTTCC TCTTCCCCCCAAAACCCAAGGACACCCTCATGATCT CCCGGACCCCTGAGGTCACATGCGTGGTGGTGGAC |
| | | GTGAGCCACGAAGACCCTGAGGTCAAGTTCAACTG GTACGTGGACGGCGTGGAGGTGCATAATGCCAAGA CAAAGCCGCGGGAGGAGCAGTACAACAGCACGTA CCGGGTGGTCAGCGTCCTCACCGTCCTGCACCAGG ACTGGCTGAATGGCAAGGAGTACAAGTGCAAGGTC TCCAACAAAGCCCTCCCAGCCCCCATCGAGAAAAC CATCTCCAAAGCCAAAGGGCAGCCCCGAGAACCA CAGGTGTACACCCTGCCCCCATCCCGGGATGAGCT GACCAAGAACCAGGTCAGCCTGACCTGCCTGGTCA AAGGCTTCTATCCCAGCGACATCGCCGTGGAGTGG GAGAGCAATGGGCAGCCGGAGAACAACTACAAGA CCACGCCTCCCGTGCTGGACTCCGACGGCTCCTTC TTCCTCTACAGCAAGCTCACCGTGGACAAGAGCAG GTGGCAGCAGGGGAACGTCTTCTCATGCTCCGTGA TGCATGAGGCTCTGCACAACCACTACACGCAGAAG AGCCTCTCCCTGTCTCCGGGTAAATGA |
| 68 | SP-GG-His-VDDDDK-hKLK1-EAAAK-IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGAG GCAGCGGCTAAGACATGCCCACCGTGCCCAGCACC TGAACTCCTGGGGGGACCGTCAGTCTTCCTCTTCC CCCCAAAACCCAAGGACACCCTCATGATCTCCCGG ACCCCTGAGGTCACATGCGTGGTGGTGGACGTGAG |
| | | CCACGAAGACCCTGAGGTCAAGTTCAACTGGTACG TGGACGGCGTGGAGGTGCATAATGCCAAGACAAAG CCGCGGGAGGAGCAGTACAACAGCACGTACCGGG TGGTCAGCGTCCTCACCGTCCTGCACCAGGACTGG CTGAATGGCAAGGAGTACAAGTGCAAGGTCTCCAA CAAAGCCCTCCCAGCCCCCATCGAGAAAACCATCT CCAAAGCCAAAGGGCAGCCCCGAGAACCACAGGT GTACACCCTGCCCCCATCCCGGGATGAGCTGACCA AGAACCAGGTCAGCCTGACCTGCCTGGTCAAAGGC TTCTATCCCAGCGACATCGCCGTGGAGTGGGAGAG CAATGGGCAGCCGGAGAACAACTACAAGACCACG CCTCCCGTGCTGGACTCCGACGGCTCCTTCTTCCTC TACAGCAAGCTCACCGTGGACAAGAGCAGGTGGC AGCAGGGGAACGTCTTCTCATGCTCCGTGATGCAT GAGGCTCTGCACAACCACTACACGCAGAAGAGCCT CTCCCTGTCTCCGGGTAAATGA |
| 69 | IL2-GG-His-VDDDDK-hKLK1-GGGSSSS-IgG1 Fc (base sequence) | ATGTACAGGATGCAACTCCTGTCTTGCATTGCACTA AGTCTTGCACTTGTCACAAACAGTGGTGGCCACCA TCATCATCATCATGTAGATGACGATGACAAGATTGT GGGAGGCTGGGAGTGTGAGCAGCATTCCCAGCCCT GGCAGGCGGCTCTGTACCATTTCAGCACTTTCCAGT GTGGGGGCATCCTGGTGCACCGCCAGTGGGTGCTC ACAGCTGCTCATTGCATCAGCGACAATTACCAGCTC TGGCTGGGTCGCCACAACTTGTTTGACGACGAAAA CACAGCCCAGTTTGTTCATGTCAGTGAGAGCTTCC CACACCCTGGCTTCAACATGAGCCTCCTGGAGAAC CACACCCGCCAAGCAGACGAGGACTACAGCCACG ACCTCATGCTGCTCCGCCTGACAGAGCCTGCTGATA CCATCACAGATGCTGTGAAGGTCGTGGAGTTGCCC ACCGAGGAACCCGAAGTGGGGAGCACCTGTTTGG CTTCCGGCTGGGGCAGCATCGAACCAGAGAATTTC TCATTTCCAGATGATCTCCAGTGTGTGGACCTCAAA ATCCTGCCTAATGATGAGTGCAAAAAAGCCCACGT CCAGAAGGTGACAGACTTCATGCTGTGTGTCGGAC ACCTGGAAGGTGGCAAAGACACCTGTGTGGGTGAT TCAGGGGGCCCGCTGATGTGTGATGGTGTGCTCCA AGGTGTCACATCATGGGGCTACGTCCCTTGTGGCA CCCCCAATAAGCCTTCTGTCGCCGTCAGAGTGCTGT CTTATGTGAAGTGGATCGAGGACACCATAGCGGAG AACTCCGGAGGCGGTTCAAGTTCTAGCACATGCCC ACCGTGCCCAGCACCTGAACTCCTGGGGGGACCGT CAGTCTTCCTCTTCCCCCCAAAACCCAAGGACACC CTCATGATCTCCCGGACCCCTGAGGTCACATGCGTG |
| | | GTGGTGGACGTGAGCCACGAAGACCCTGAGGTCA AGTTCAACTGGTACGTGGACGGCGTGGAGGTGCAT AATGCCAAGACAAAGCCGCGGGAGGAGCAGTACA ACAGCACGTACCGGGTGGTCAGCGTCCTCACCGTC CTGCACCAGGACTGGCTGAATGGCAAGGAGTACAA GTGCAAGGTCTCCAACAAAGCCCTCCCAGCCCCCA TCGAGAAAACCATCTCCAAAGCCAAAGGGCAGCC CCGAGAACCACAGGTGTACACCCTGCCCCCATCCC GGGATGAGCTGACCAAGAACCAGGTCAGCCTGAC CTGCCTGGTCAAAGGCTTCTATCCCAGCGACATCGC CGTGGAGTGGGAGAGCAATGGGCAGCCGGAGAAC AACTACAAGACCACGCCTCCCGTGCTGGACTCCGA CGGCTCCTTCTTCCTCTACAGCAAGCTCACCGTGG ACAAGAGCAGGTGGCAGCAGGGGAACGTCTTCTC ATGCTCCGTGATGCATGAGGCTCTGCACAACCACTA CACGCAGAAGAGCCTCTCCCTGTCTCCGGGTAAAT GA |
| 70 | IL2-GG-His-VDDDDK-hKLK1-EAAAK-IgG1 Fc (base sequence) | ATGTACAGGATGCAACTCCTGTCTTGCATTGCACTA AGTCTTGCACTTGTCACAAACAGTGGTGGCCACCA TCATCATCATCATGTAGATGACGATGACAAGATTGT GGGAGGCTGGGAGTGTGAGCAGCATTCCCAGCCCT GGCAGGCGGCTCTGTACCATTTCAGCACTTTCCAGT GTGGGGGCATCCTGGTGCACCGCCAGTGGGTGCTC ACAGCTGCTCATTGCATCAGCGACAATTACCAGCTC TGGCTGGGTCGCCACAACTTGTTTGACGACGAAAA CACAGCCCAGTTTGTTCATGTCAGTGAGAGCTTCC CACACCCTGGCTTCAACATGAGCCTCCTGGAGAAC CACACCCGCCAAGCAGACGAGGACTACAGCCACG ACCTCATGCTGCTCCGCCTGACAGAGCCTGCTGATA CCATCACAGATGCTGTGAAGGTCGTGGAGTTGCCC ACCGAGGAACCCGAAGTGGGGAGCACCTGTTTGG CTTCCGGCTGGGGCAGCATCGAACCAGAGAATTTC TCATTTCCAGATGATCTCCAGTGTGTGGACCTCAAA ATCCTGCCTAATGATGAGTGCAAAAAAGCCCACGT CCAGAAGGTGACAGACTTCATGCTGTGTGTCGGAC ACCTGGAAGGTGGCAAAGACACCTGTGTGGGTGAT TCAGGGGGCCCGCTGATGTGTGATGGTGTGCTCCA AGGTGTCACATCATGGGGCTACGTCCCTTGTGGCA CCCCCAATAAGCCTTCTGTCGCCGTCAGAGTGCTGT CTTATGTGAAGTGGATCGAGGACACCATAGCGGAG AACTCCGAGGCAGCGGCTAAGACATGCCCACCGTG CCCAGCACCTGAACTCCTGGGGGGACCGTCAGTCT TCCTCTTCCCCCCAAAACCCAAGGACACCCTCATG ATCTCCCGGACCCCTGAGGTCACATGCGTGGTGGT |
| | | GGACGTGAGCCACGAAGACCCTGAGGTCAAGTTC AACTGGTACGTGGACGGCGTGGAGGTGCATAATGC CAAGACAAAGCCGCGGGAGGAGCAGTACAACAGC ACGTACCGGGTGGTCAGCGTCCTCACCGTCCTGCA CCAGGACTGGCTGAATGGCAAGGAGTACAAGTGC AAGGTCTCCAACAAAGCCCTCCCAGCCCCCATCGA GAAAACCATCTCCAAAGCCAAAGGGCAGCCCCGA GAACCACAGGTGTACACCCTGCCCCCATCCCGGGA TGAGCTGACCAAGAACCAGGTCAGCCTGACCTGCC TGGTCAAAGGCTTCTATCCCAGCGACATCGCCGTG GAGTGGGAGAGCAATGGGCAGCCGGAGAACAACT ACAAGACCACGCCTCCCGTGCTGGACTCCGACGGC TCCTTCTTCCTCTACAGCAAGCTCACCGTGGACAA GAGCAGGTGGCAGCAGGGGAACGTCTTCTCATGCT CCGTGATGCATGAGGCTCTGCACAACCACTACACG CAGAAGAGCCTCTCCCTGTCTCCGGGTAAATGA |
| 71 | SP-GG-His-VDDDDK-hKLK1-GSSGSSG-IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA TCAAGTGGCTCTAGCGGTACATGCCCACCGTGCCC AGCACCTGAACTCCTGGGGGGACCGTCAGTCTTCC TCTTCCCCCCAAAACCCAAGGACACCCTCATGATCT CCCGGACCCCTGAGGTCACATGCGTGGTGGTGGAC |
| | | GTGAGCCACGAAGACCCTGAGGTCAAGTTCAACTG GTACGTGGACGGCGTGGAGGTGCATAATGCCAAGA CAAAGCCGCGGGAGGAGCAGTACAACAGCACGTA CCGGGTGGTCAGCGTCCTCACCGTCCTGCACCAGG ACTGGCTGAATGGCAAGGAGTACAAGTGCAAGGTC TCCAACAAAGCCCTCCCAGCCCCCATCGAGAAAAC CATCTCCAAAGCCAAAGGGCAGCCCCGAGAACCA CAGGTGTACACCCTGCCCCCATCCCGGGATGAGCT GACCAAGAACCAGGTCAGCCTGACCTGCCTGGTCA AAGGCTTCTATCCCAGCGACATCGCCGTGGAGTGG GAGAGCAATGGGCAGCCGGAGAACAACTACAAGA CCACGCCTCCCGTGCTGGACTCCGACGGCTCCTTC TTCCTCTACAGCAAGCTCACCGTGGACAAGAGCAG GTGGCAGCAGGGGAACGTCTTCTCATGCTCCGTGA TGCATGAGGCTCTGCACAACCACTACACGCAGAAG AGCCTCTCCCTGTCTCCGGGTAAATGA |
| 72 | SP-GG-His-VDDDDK-hKLK1-GSSGSSG-YTE IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA TCAAGTGGCTCTAGCGGTACATGCCCACCGTGCCC AGCACCTGAACTCCTGGGGGGACCGTCAGTCTTCC TCTTCCCCCCAAAACCCAAGGACACCCTCTACATC ACCCGGGAACCTGAGGTCACATGCGTGGTGGTGGA |
| | | CGTGAGCCACGAAGACCCTGAGGTCAAGTTCAACT GGTACGTGGACGGCGTGGAGGTGCATAATGCCAAG ACAAAGCCGCGGGAGGAGCAGTACAACAGCACGT ACCGGGTGGTCAGCGTCCTCACCGTCCTGCACCAG GACTGGCTGAATGGCAAGGAGTACAAGTGCAAGG TCTCCAACAAAGCCCTCCCAGCCCCCATCGAGAAA ACCATCTCCAAAGCCAAAGGGCAGCCCCGAGAAC CACAGGTGTACACCCTGCCCCCATCCCGGGATGAG CTGACCAAGAACCAGGTCAGCCTGACCTGCCTGGT CAAAGGCTTCTATCCCAGCGACATCGCCGTGGAGT GGGAGAGCAATGGGCAGCCGGAGAACAACTACAA GACCACGCCTCCCGTGCTGGACTCCGACGGCTCCT TCTTCCTCTACAGCAAGCTCACCGTGGACAAGAGC AGGTGGCAGCAGGGGAACGTCTTCTCATGCTCCGT GATGCATGAGGCTCTGCACAACCACTACACGCAGA AGAGCCTCTCCCTGTCTCCGGGTAAATGA |
| 73 | SP-GG-His-VDDDDK-hKLK1-GSSGSSG-IgG4 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA TCAAGTGGCTCTAGCGGTGGTCCCCCATGCCCATCA TGCCCAGCACCTGAGTTCCTGGGGGGACCATCAGT CTTCCTGTTCCCCCCAAAACCCAAGGACACTCTCAT GATCTCCCGGACCCCTGAGGTCACGTGCGTGGTGG |
| | | TGGACGTGAGCCAGGAAGACCCCGAGGTCCAGTT CAACTGGTACGTGGATGGCGTGGAGGTGCATAATG CCAAGACAAAGCCGCGGGAGGAGCAGTTCAACAG CACGTACCGTGTGGTCAGCGTCCTCACCGTCCTGC ACCAGGACTGGCTGAACGGCAAGGAGTACAAGTG CAAGGTCTCCAACAAAGGCCTCCCGTCCTCCATCG AGAAAACCATCTCCAAAGCCAAAGGGCAGCCCCG AGAGCCACAGGTGTACACCCTGCCCCCATCCCAGG AGGAGATGACCAAGAACCAGGTCAGCCTGACCTG CCTGGTCAAAGGCTTCTACCCCAGCGACATCGCCG TGGAGTGGGAGAGCAATGGGCAGCCGGAGAACAA CTACAAGACCACGCCTCCCGTGCTGGACTCCGACG GCTCCTTCTTCCTCTACAGCAGGCTCACCGTGGAC AAGAGCAGGTGGCAGGAGGGGAATGTCTTCTCATG CTCCGTGATGCATGAGGCTCTGCACAACCACTACA CACAGAAGAGCCTCTCCCTGTCTCTGGGTAAATGA |
| 74 | SP-GG-His-VDDDDK-hKLK1-GSSGSSG-YTE IgG4 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA TCAAGTGGCTCTAGCGGTGGTCCCCCATGCCCATCA TGCCCAGCACCTGAGTTCCTGGGGGGACCATCAGT CTTCCTGTTCCCCCCAAAACCCAAGGACACTCTCT ACATCACCCGGGAGCCTGAGGTCACGTGCGTGGTG |
| | | GTGGACGTGAGCCAGGAAGACCCCGAGGTCCAGT TCAACTGGTACGTGGATGGCGTGGAGGTGCATAAT GCCAAGACAAAGCCGCGGGAGGAGCAGTTCAACA GCACGTACCGTGTGGTCAGCGTCCTCACCGTCCTG CACCAGGACTGGCTGAACGGCAAGGAGTACAAGT GCAAGGTCTCCAACAAAGGCCTCCCGTCCTCCATC GAGAAAACCATCTCCAAAGCCAAAGGGCAGCCCC GAGAGCCACAGGTGTACACCCTGCCCCCATCCCAG GAGGAGATGACCAAGAACCAGGTCAGCCTGACCT GCCTGGTCAAAGGCTTCTACCCCAGCGACATCGCC GTGGAGTGGGAGAGCAATGGGCAGCCGGAGAACA ACTACAAGACCACGCCTCCCGTGCTGGACTCCGAC GGCTCCTTCTTCCTCTACAGCAGGCTCACCGTGGA CAAGAGCAGGTGGCAGGAGGGGAATGTCTTCTCAT GCTCCGTGATGCATGAGGCTCTGCACAACCACTAC ACACAGAAGAGCCTCTCCCTGTCTCTGGGTAAATG A |
| 75 | SP-GG-His-VDDDDK-hKLK1-GGGGSGGGGS-IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCGAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGCCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA GGCGGTGGATCAGGAGGCGGTGGATCAACATGCCC ACCGTGCCCAGCACCTGAACTCCTGGGGGGACCGT CAGTCTTCCTCTTCCCCCCAAAACCCAAGGACACC CTCATGATCTCCCGGACCCCTGAGGTCACATGCGTG |
| | | GTGGTGGACGTGAGCCACGAAGACCCTGAGGTCA AGTTCAACTGGTACGTGGACGGCGTGGAGGTGCAT AATGCCAAGACAAAGCCGCGGGAGGAGCAGTACA ACAGCACGTACCGGGTGGTCAGCGTCCTCACCGTC CTGCACCAGGACTGGCTGAATGGCAAGGAGTACAA GTGCAAGGTCTCCAACAAAGCCCTCCCAGCCCCCA TCGAGAAAACCATCTCCAAAGCCAAAGGGCAGCC CCGAGAACCACAGGTGTACACCCTGCCCCCATCCC GGGATGAGCTGACCAAGAACCAGGTCAGCCTGAC CTGCCTGGTCAAAGGCTTCTATCCCAGCGACATCGC CGTGGAGTGGGAGAGCAATGGGCAGCCGGAGAAC AACTACAAGACCACGCCTCCCGTGCTGGACTCCGA CGGCTCCTTCTTCCTCTACAGCAAGCTCACCGTGG ACAAGAGCAGGTGGCAGCAGGGGAACGTCTTCTC ATGCTCCGTGATGCATGAGGCTCTGCACAACCACTA CACGCAGAAGAGCCTCTCCCTGTCTCCGGGTAAAT GA |
| 76 | EK recognition sequence (base sequence) | gtagatgacgatgacaag |
| 77 | Human mature KLK1 protein (AA25-262) (base sequence) | ATTGTGGGAGGCTGGGAGTGTGAGCAGCATTCCCA GCCCTGGCAGGCGGCTCTGTACCATTTCAGCACTTT CCAGTGTGGGGGCATCCTGGTGCACCGCCAGTGGG TGCTCACAGCTGCTCATTGCATCAGCGACAATTACC AGCTCTGGCTGGGTCGCCACAACTTGTTTGACGAC GAAAACACAGCCCAGTTTGTTCATGTCAGTGAGAG CTTCCCACACCCTGGCTTCAACATGAGCCTCCTGG AGAACCACACCCGCCAAGCAGACGAGGACTACAG CCACGACCTCATGCTGCTCCGCCTGACAGAGCCTG CTGATACCATCACAGATGCTGTGAAGGTCGTGGAG TTGCCCACCGAGGAACCCGAAGTGGGGAGCACCT GTTTGGCTTCCGGCTGGGGCAGCATCGAACCAGAG AATTTCTCATTTCCAGATGATCTCCAGTGTGTGGAC CTCAAAATCCTGCCTAATGATGAGTGCAAAAAAGC CCACGTCCAGAAGGTGACAGACTTCATGCTGTGTG TCGGACACCTGGAAGGTGGCAAAGACACCTGTGT GGGTGATTCAGGGGGCCCGCTGATGTGTGATGGTG TGCTCCAAGGTGTCACATCATGGGGCTACGTCCCTT GTGGCACCCCCAATAAGCCTTCTGTCGCCGTCAGA GTGCTGTCTTATGTGAAGTGGATCGAGGACACCATA GCGGAGAACTCCTGA |
| 78 | IgG1 Fc (base sequence) | acatgcccaccgtgcccagcacctgaactcctggggggaccgtcagtcttcctcttc cccccaaaacccaaggacaccctcatgatctcccggacccctgaggtcacatgcgt ggtggtggacgtgagccacgaagaccctgaggtcaagttcaactggtacgtggac ggcgtggaggtgcataatgccaagacaaagccgcgggaggagcagtacaacag cacgtaccgggtggtcagcgtcctcaccgtcctgcaccaggactggctgaatggc aaggagtacaagtgcaaggtctccaacaaagccctcccagcccccatcgagaaaa ccatctccaaagccaaagggcagccccgagaaccacaggtgtacaccctgcccc catcccgggatgagctgaccaagaaccaggtcagcctgacctgcctggtcaaagg cttctatcccagcgacatcgccgtggagtgggagagcaatgggcagccggagaa caactacaagaccacgcctcccgtgctggactccgacggctccttcttcctctacag caagctcaccgtggacaagagcaggtggcagcaggggaacgtcttctcatgctcc gtgatgcatgaggctctgcacaaccactacacgcagaagagcctctccctgtctcc gggtaaatga |
| 79 | IgG4 Fc (base sequence) | ggtcccccatgcccatcatgcccagcacctgagttcctggggggaccatcagtcttc ctgttccccccaaaacccaaggacactctcatgatctcccggacccctgaggtcac gtgcgtggtggtggacgtgagccaggaagaccccgaggtccagttcaactggtac gtggatggcgtggaggtgcataatgccaagacaaagccgcgggaggagcagttc aacagcacgtaccgtgtggtcagcgtcctcaccgtcctgcaccaggactggctgaa cggcaaggagtacaagtgcaaggtctccaacaaaggcctcccgtcctccatcgag aaaaccatctccaaagccaaagggcagccccgagagccacaggtgtacaccctg cccccatcccaggaggagatgaccaagaaccaggtcagcctgacctgcctggtca aaggcttctaccccagcgacatcgccgtggagtgggagagcaatgggcagccgg agaacaactacaagaccacgcctcccgtgctggactccgacggctccttcttcctct acagcaggctcaccgtggacaagagcaggtggcaggaggggaatgtcttctcatg ctccgtgatgcatgaggctctgcacaaccactacacacagaagagcctctccctgtc tctgggtaaatga |
| 80 | IgG1 Fc YTE (base sequence) | acatgcccaccgtgcccagcacctgaactcctggggggaccgtcagtcttcctcttc cccccaaaacccaaggacaccctcatgatctcccggacccctgaggtcacatgcg tggtggtggacgtgagccacgaagaccctgaggtcaagttcaactggtacgtgga cggcgtggaggtgcataatgccaagacaaagccgcgggaggagcagtacaaca gcacgtaccgggtggtcagcgtcctcaccgtcctgcaccaggactggctgaatgg caaggagtacaagtgcaaggtctccaacaaagccctcccagcccccatcgagaaa accatctccaaagccaaagggcagccccgagaaccacaggtgtacaccctgccc ccatcccgggatgagctgaccaagaaccaggtcagcctgacctgcctggtcaaag gcttctatcccagcgacatcgccgtggagtgggagagcaatgggcagccggaga acaactacaagaccacgcctcccgtgctggactccgacggctccttcttcctctaca gcaagctcaccgtggacaagagcaggtggcagcaggggaacgtcttctcatgctc cgtgatgcatgaggctctgcacaaccactacacgcagaagagcctctccctgtctc cgggtaaatga |
| 81 | IgG4 Fc YTE (base sequence) | ggtcccccatgcccatcatgcccagcacctgagttcctggggggaccatcagtcttc ctgttccccccaaaacccaaggacactctcatgatctcccggacccctgaggtcac gtgcgtggtggtggacgtgagccaggaagaccccgaggtccagttcaactggtac gtggatggcgtggaggtgcataatgccaagacaaagccgcgggaggagcagttc aacagcacgtaccgtgtggtcagcgtcctcaccgtcctgcaccaggactggctgaa cggcaaggagtacaagtgcaaggtctccaacaaaggcctcccgtcctccatcgag aaaaccatctccaaagccaaagggcagccccgagagccacaggtgtacaccctg cccccatcccaggaggagatgaccaagaaccaggtcagcctgacctgcctggtca |
| | | aaggcttctaccccagcgacatcgccgtggagtgggagagcaatgggcagccgg agaacaactacaagaccacgcctcccgtgctggactccgacggctccttcttcctct acagcaggctcaccgtggacaagagcaggtggcaggaggggaatgtcttctcatg ctccgtgatgcatgaggctctgcacaaccactacacacagaagagcctctccctgtc tctgggtaaatga |
| 82 | 6xHis tag (base sequence) | caccatcatcatcatcat |
| 83 | GGGGS linker (base sequence) | ggaggcggtggatca |
| 84 | GSSGSSG linker (base sequence) | ggatcaagtggctctagcggt |
| 85 | GGGSSSS linker (base sequence) | ggaggcggttcaagttctagc |
| 86 | EAAAK linker (base sequence) | gaggcagcggctaag |
| 87 | Signal peptide (base sequence) | atgtggttcctggttctgtgcctcgccctgtccctgggggggactggtgctgcg |
| 88 | IL-2 signal peptide (base sequence) | atgtacaggatgcaactcctgtcttgcattgcactaagtcttgcacttgtcacaaacag t |
| 89 | SP-prepro-hKLK1-EAAAK-IgG1 Fc | MWFLVLCLALSLGGTGAAPPIQSRIVGGWECEQHSQ PWQAALYHFSTFQCGGILVHRQWVLTAAHCISDNYQ LWLGRHNLFDDENTAQFVHVSESFPHPGFNMSLLEN HTRQADEDYSHDLMLLRLTEPADTITDAVKVVELPTE EPEVGSTCLASGWGSIEPENFSFPDDLQCVDLKILPND ECKKAHVQKVTDFMLCVGHLEGGKDTCVGDSGGPL MCDGVLQGVTSWGYVPCGTPNKPSVAVRVLSYVKW IEDTIAENSEAAAKTCPPCPAPELLGGPSVFLFPPKPK DTLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSRDE LTKNQVSLTCLVKGFYPSDIAVEWESNGQPENNYKTT PPVLDSDGSFFLYSKLTVDKSRWQQGNVFSCSVMHE ALHNHYTQKSLSLSPGK |
| 91 | SP-prepro-VDDDDK-hKLK1 | MWFLVLCLALSLGGTGAAPPIQSRVDDDDKIVGGWE CEQHSQPWQAALYHFSTFQCGGILVHRQWVLTAAHC ISDNYQLWLGRHNLFDDENTAQFVHVSESFPHPGFN MSLLENHTRQADEDYSHDLMLLRLTEPADTITDAVK VVELPTEEPEVGSTCLASGWGSIEPENFSFPDDLQCV DLKILPNDECKKAHVQKVTDFMLCVGHLEGGKDTC |
| | | VGDSGGPLMCDGVLQGVTSWGYVPCGTPNKPSVAV RVLSYVKWIEDTIAENS |
| 92 | SP-prepro-VDDDDK-hKLK1-EAAAK-IgG1 Fc | MWFLVLCLALSLGGTGAAPPIQSRVDDDDKIVGGWE CEQHSQPWQAALYHFSTFQCGGILVHRQWVLTAAHC ISDNYQLWLGRHNLFDDENTAQFVHVSESFPHPGFN MSLLENHTRQADEDYSHDLMLLRLTEPADTITDAVK VVELPTEEPEVGSTCLASGWGSIEPENFSFPDDLQCV DLKILPNDECKKAHVQKVTDFMLCVGHLEGGKDTC VGDSGGPLMCDGVLQGVTSWGYVPCGTPNKPSVAV RVLSYVKWIEDTIAENSEAAAKTCPPCPAPELLGGPS VFLFPPKPKDTLMISRTPEVTCVVVDVSHEDPEVKFN WYVDGVEVHNAKTKPREEQYNSTYRVVSVLTVLHQ DWLNGKEYKCKVSNKALPAPIEKTISKAKGQPREPQ VYTLPPSRDELTKNQVSLTCLVKGFYPSDIAVEWESN GQPENNYKTTPPVLDSDGSFFLYSKLTVDKSRWQQG NVFSCSVMHEALHNHYTQKSLSLSPGK |
| 93 | prepro-hKLK1-EAAAK-IgG1 Fc | PPIQSRIVGGWECEQHSQPWQAALYHFSTFQCGGILV HRQWVLTAAHCISDNYQLWLGRHNLFDDENTAQFV HVSESFPHPGFNMSLLENHTRQADEDYSHDLMLLRL TEPADTITDAVKVVELPTEEPEVGSTCLASGWGSIEPE NFSFPDDLQCVDLKILPNDECKKAHVQKVTDFMLCV GHLEGGKDTCVGDSGGPLMCDGVLQGVTSWGYVP CGTPNKPSVAVRVLSYVKWIEDTIAENSEAAAKTCPP CPAPELLGGPSVFLFPPKPKDTLMISRTPEVTCVVVDV SHEDPEVKFNWYVDGVEVHNAKTKPREEQYNSTYR VVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLT VDKSRWQQGNVFSCSVMHEALHNHYTQKSLSLSPG K |
| 94 | prepro-hKLK1 | PPIQSRIVGGWECEQHSQPWQAALYHFSTFQCGGILV HRQWVLTAAHCISDNYQLWLGRHNLFDDENTAQFV HVSESFPHPGFNMSLLENHTRQADEDYSHDLMLLRL TEPADTITDAVKVVELPTEEPEVGSTCLASGWGSIEPE NFSFPDDLQCVDLKILPNDECKKAHVQKVTDFMLCV GHLEGGKDTCVGDSGGPLMCDGVLQGVTSWGYVP CGTPNKPSVAVRVLSYVKWIEDTIAENS |
| 95 | prepro-VDDDDK-hKLK1 | PPIQSRVDDDDKIVGGWECEQHSQPWQAALYHFSTF QCGGILVHRQWVLTAAHCISDNYQLWLGRHNLFDDE NTAQFVHVSESFPHPGFNMSLLENHTRQADEDYSHD LMLLRLTEPADTITDAVKVVELPTEEPEVGSTCLASG WGSIEPENFSFPDDLQCVDLKILPNDECKKAHVQKVT DFMLCVGHLEGGKDTCVGDSGGPLMCDGVLQGVTS WGYVPCGTPNKPSVAVRVLSYVKWIEDTIAENS |
| 96 | prepro-VDDDDK-hKLK1-EAAAK-IgG1 Fc | PPIQSRVDDDDKIVGGWECEQHSQPWQAALYHFSTF QCGGILVHRQWVLTAAHCISDNYQLWLGRHNLFDDE NTAQFVHVSESFPHPGFNMSLLENHTRQADEDYSHD LMLLRLTEPADTITDAVKVVELPTEEPEVGSTCLASG WGSIEPENFSFPDDLQCVDLKILPNDECKKAHVQKVT DFMLCVGHLEGGKDTCVGDSGGPLMCDGVLQGVTS WGYVPCGTPNKPSVAVRVLSYVKWIEDTIAENSEAA AKTCPPCPAPELLGGPSVFLFPPKPKDTLMISRTPEVT CVVVDVSHEDPEVKFNWYVDGVEVHNAKTKPREEQ YNSTYRVVSVLTVLHQDWLNGKEYKCKVSNKALPA PIEKTISKAKGQPREPQVYTLPPSRDELTKNQVSLTCL VKGFYPSDIAVEWESNGQPENNYKTTPPVLDSDGSFF LYSKLTVDKSRWQQGNVFSCSVMHEALHNHYTQKS LSLSPGK |
| 97 | SP-prepro-hKLK1-EAAAK-IgG1 Fc (base sequence) | ATGTGGTTCCTGGTTCTGTGCCTCGCCCTGTCCCTG GGGGGGACTGGTGCTGCGCCCCCGATTCAGTCCCG GATTGTGGGAGGCTGGGAGTGTGAGCAGCATTCCC AGCCCTGGCAGGCGGCTCTGTACCATTTCAGCACT TTCCAGTGTGGGGGCATCCTGGTGCACCGCCAGTG GGTGCTCACAGCTGCTCATTGCATCAGCGACAATTA CCAGCTCTGGCTGGGTCGCCACAACTTGTTTGACG ACGAAAACACAGCCCAGTTTGTTCATGTCAGTGAG AGCTTCCCACACCCTGGCTTCAACATGAGCCTCCT GGAGAACCACACCCGCCAAGCAGACGAGGACTAC AGCCACGACCTCATGCTGCTCCGCCTGACAGAGCC TGCTGATACCATCACAGATGCTGTGAAGGTCGTGG AGTTGCCCACCGAGGAACCCGAAGTGGGGAGCAC CTGTTTGGCTTCCGGCTGGGGCAGCATCGAACCAG AGAATTTCTCATTTCCAGATGATCTCCAGTGTGTGG ACCTCAAAATCCTGCCTAATGATGAGTGCAAAAAA GCCCACGTCCAGAAGGTGACAGACTTCATGCTGTG TGTCGGACACCTGGAAGGTGGCAAAGACACCTGT GTGGGTGATTCAGGGGGCCCGCTGATGTGTGATGG TGTGCTCCAAGGTGTCACATCATGGGGCTACGTCCC TTGTGGCACCCCCAATAAGCCTTCTGTCGCCGTCAG AGTGCTGTCTTATGTGAAGTGGATCGAGGACACCA TAGCGGAGAACTCCGAGGCAGCGGCTAAGACATGC CCACCGTGCCCAGCACCTGAACTCCTGGGGGGACC GTCAGTCTTCCTCTTCCCCCCAAAACCCAAGGACA CCCTCATGATCTCCCGGACCCCTGAGGTCACATGCG TGGTGGTGGACGTGAGCCACGAAGACCCTGAGGT CAAGTTCAACTGGTACGTGGACGGCGTGGAGGTGC ATAATGCCAAGACAAAGCCGCGGGAGGAGCAGTAC AACAGCACGTACCGGGTGGTCAGCGTCCTCACCGT CCTGCACCAGGACTGGCTGAATGGCAAGGAGTACA AGTGCAAGGTCTCCAACAAAGCCCTCCCAGCCCCC ATCGAGAAAACCATCTCCAAAGCCAAAGGGCAGCC CCGAGAACCACAGGTGTACACCCTGCCCCCATCCC GGGATGAGCTGACCAAGAACCAGGTCAGCCTGAC |
| | | CTGCCTGGTCAAAGGCTTCTATCCCAGCGACATCGC CGTGGAGTGGGAGAGCAATGGGCAGCCGGAGAAC AACTACAAGACCACGCCTCCCGTGCTGGACTCCGA CGGCTCCTTCTTCCTCTACAGCAAGCTCACCGTGG ACAAGAGCAGGTGGCAGCAGGGGAACGTCTTCTC ATGCTCCGTGATGCATGAGGCTCTGCACAACCACTA CACGCAGAAGAGCCTCTCCCTGTCTCCGGGTAAAT GACTCGAGTGC |
| 98 | SP-prepro-hKLK1 (base sequence) | ATGTGGTTCCTGGTTCTGTGCCTCGCCCTGTCCCTG GGGGGGACTGGTGCTGCGCCCCCGATTCAGTCCCG GATTGTGGGAGGCTGGGAGTGTGAGCAGCATTCCC AGCCCTGGCAGGCGGCTCTGTACCATTTCAGCACT TTCCAGTGTGGGGGCATCCTGGTGCACCGCCAGTG GGTGCTCACAGCTGCTCATTGCATCAGCGACAATTA CCAGCTCTGGCTGGGTCGCCACAACTTGTTTGACG ACGAAAACACAGCCCAGTTTGTTCATGTCAGTGAG AGCTTCCCACACCCTGGCTTCAACATGAGCCTCCT GGAGAACCACACCCGCCAAGCAGACGAGGACTAC AGCCACGACCTCATGCTGCTCCGCCTGACAGAGCC TGCTGATACCATCACAGATGCTGTGAAGGTCGTGG AGTTGCCCACCGAGGAACCCGAAGTGGGGAGCAC CTGTTTGGCTTCCGGCTGGGGCAGCATCGAACCAG AGAATTTCTCATTTCCAGATGATCTCCAGTGTGTGG ACCTCAAAATCCTGCCTAATGATGAGTGCAAAAAA GCCCACGTCCAGAAGGTGACAGACTTCATGCTGTG TGTCGGACACCTGGAAGGTGGCAAAGACACCTGT GTGGGTGATTCAGGGGGCCCGCTGATGTGTGATGG TGTGCTCCAAGGTGTCACATCATGGGGCTACGTCCC TTGTGGCACCCCCAATAAGCCTTCTGTCGCCGTCAG AGTGCTGTCTTATGTGAAGTGGATCGAGGACACCA TAGCGGAGAACTCCTGA |
| 99 | SP-prepro-VDDDDK-hKLK1 (base sequence) | ATGTGGTTCCTGGTTCTGTGCCTCGCCCTGTCCCTG GGGGGGACTGGTGCTGCGCCCCCGATTCAGTCCCG GGTAGATGACGATGACAAGATTGTGGGAGGCTGGG AGTGTGAGCAGCATTCCCAGCCCTGGCAGGCGGCT CTGTACCATTTCAGCACTTTCCAGTGTGGGGGCATC CTGGTGCACCGCCAGTGGGTGCTCACAGCTGCTCA TTGCATCAGCGACAATTACCAGCTCTGGCTGGGTC GCCACAACTTGTTTGACGACGAAAACACAGCCCAG TTTGTTCATGTCAGTGAGAGCTTCCCACACCCTGGC TTCAACATGAGCCTCCTGGAGAACCACACCCGCCA AGCAGACGAGGACTACAGCCACGACCTCATGCTGC TCCGCCTGACAGAGCCTGCTGATACCATCACAGAT GCTGTGAAGGTCGTGGAGTTGCCCACCGAGGAACC CGAAGTGGGGAGCACCTGTTTGGCTTCCGGCTGGG GCAGCATCGAACCAGAGAATTTCTCATTTCCAGATG ATCTCCAGTGTGTGGACCTCAAAATCCTGCCTAATG ATGAGTGCAAAAAAGCCCACGTCCAGAAGGTGAC |
| | | AGACTTCATGCTGTGTGTCGGACACCTGGAAGGTG GCAAAGACACCTGTGTGGGTGATTCAGGGGGCCCG CTGATGTGTGATGGTGTGCTCCAAGGTGTCACATCA TGGGGCTACGTCCCTTGTGGCACCCCCAATAAGCCT TCTGTCGCCGTCAGAGTGCTGTCTTATGTGAAGTGG ATCGAGGACACCATAGCGGAGAACTCCTGA |
| 90 | SP-prepro-VDDDDK-hKLK1-Fc (IgG1) (base sequence) | ATGTGGTTCCTGGTTCTGTGCCTCGCCCTGTCCCTG GGGGGGACTGGTGCTGCGCCCCCGATTCAGTCCCG GGTAGATGACGATGACAAGATTGTGGGAGGCTGGG AGTGTGAGCAGCATTCCCAGCCCTGGCAGGCGGCT CTGTACCATTTCAGCACTTTCCAGTGTGGGGGCATC CTGGTGCACCGCCAGTGGGTGCTCACAGCTGCTCA TTGCATCAGCGACAATTACCAGCTCTGGCTGGGTC GCCACAACTTGTTTGACGACGAAAACACAGCCCAG TTTGTTCATGTCAGTGAGAGCTTCCCACACCCTGGC TTCAACATGAGCCTCCTGGAGAACCACACCCGCCA AGCAGACGAGGACTACAGCCACGACCTCATGCTGC TCCGCCTGACAGAGCCTGCTGATACCATCACAGAT GCTGTGAAGGTCGTGGAGTTGCCCACCGAGGAACC CGAAGTGGGGAGCACCTGTTTGGCTTCCGGCTGGG GCAGCATCGAACCAGAGAATTTCTCATTTCCAGATG ATCTCCAGTGTGTGGACCTCAAAATCCTGCCTAATG ATGAGTGCAAAAAAGCCCACGTCCAGAAGGTGAC AGACTTCATGCTGTGTGTCGGACACCTGGAAGGTG GCAAAGACACCTGTGTGGGTGATTCAGGGGGCCCG CTGATGTGTGATGGTGTGCTCCAAGGTGTCACATCA TGGGGCTACGTCCCTTGTGGCACCCCCAATAAGCCT TCTGTCGCCGTCAGAGTGCTGTCTTATGTGAAGTGG ATCGAGGACACCATAGCGGAGAACTCCGAGGCAGC GGCTAAGACATGCCCACCGTGCCCAGCACCTGAAC TCCTGGGGGGACCGTCAGTCTTCCTCTTCCCCCCA AAACCCAAGGACACCCTCATGATCTCCCGGACCCC TGAGGTCACATGCGTGGTGGTGGACGTGAGCCACG AAGACCCTGAGGTCAAGTTCAACTGGTACGTGGAC GGCGTGGAGGTGCATAATGCCAAGACAAAGCCGCG GGAGGAGCAGTACAACAGCACGTACCGGGTGGTC AGCGTCCTCACCGTCCTGCACCAGGACTGGCTGAA TGGCAAGGAGTACAAGTGCAAGGTCTCCAACAAA GCCCTCCCAGCCCCCATCGAGAAAACCATCTCCAA AGCCAAAGGGCAGCCCCGAGAACCACAGGTGTAC ACCCTGCCCCCATCCCGGGATGAGCTGACCAAGAA CCAGGTCAGCCTGACCTGCCTGGTCAAAGGCTTCT ATCCCAGCGACATCGCCGTGGAGTGGGAGAGCAAT GGGCAGCCGGAGAACAACTACAAGACCACGCCTC CCGTGCTGGACTCCGACGGCTCCTTCTTCCTCTACA GCAAGCTCACCGTGGACAAGAGCAGGTGGCAGCA GGGGAACGTCTTCTCATGCTCCGTGATGCATGAGG CTCTGCACAACCACTACACGCAGAAGAGCCTCTCC CTGTCTCCGGGTAAATGACTCGAGTGC |
| 100 | GG-His-VDDDDK-hKLK1-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSTCPPCPAPELLGGPSVFLFPPKPKDTLMISRTPEVTC VVVDVSHEDPEVKFNWYVDGVEVHNAKTKPREEQY NSTYRVVSVLTVLHQDWLNGKEYKCKVSNKALPAPI EKTISKAKGQPREPQVYTLPPSRDELTKNQVSLTCLV KGFYPSDIAVEWESNGQPENNYKTTPPVLDSDGSFFL YSKLTVDKSRWQQGNVFSCSVMHEALHNHYTQKSL SLSPGK |
| 101 | GG-His-VDDDDK-hKLK1-GGGSSSS-IgG 1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSGGGSSSSTCPPCPAPELLGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALHN HYTQKSLSLSPGK |
| 102 | GG-His-VDDDDK-hKLK1-EAAAK-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSEAAAKTCPPCPAPELLGGPSVFLFPPKPKDTLMISR TPEVTCVVVDVSHEDPEVKFNWYVDGVEVHNAKTK PREEQYNSTYRVVSVLTVLHQDWLNGKEYKCKVSN KALPAPIEKTISKAKGQPREPQVYTLPPSRDELTKNQV SLTCLVKGFYPSDIAVEWESNGQPENNYKTTPPVLDS DGSFFLYSKLTVDKSRWQQGNVFSCSVMHEALHNH YTQKSLSLSPGK |
| 103 | GG-His-VDDDDK-hKLK1-GGGSSSS-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSGGGSSSSTCPPCPAPELLGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALHN HYTQKSLSLSPGK |
| 104 | GG-His-VDDDDK-hKLK1-EAAAK-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSEAAAKTCPPCPAPELLGGPSVFLFPPKPKDTLMISR TPEVTCVVVDVSHEDPEVKFNWYVDGVEVHNAKTK PREEQYNSTYRVVSVLTVLHQDWLNGKEYKCKVSN KALPAPIEKTISKAKGQPREPQVYTLPPSRDELTKNQV SLTCLVKGFYPSDIAVEWESNGQPENNYKTTPPVLDS DGSFFLYSKLTVDKSRWQQGNVFSCSVMHEALHNH YTQKSLSLSPGK |
| 105 | GG-His-VDDDDK-hKLK1-GSSGSSG-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSGSSGSSGTCPPCPAPELLGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALHN HYTQKSLSLSPGK |
| 106 | GG-His-VDDDDK-hKLK1-GSSGSSG-YTE IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSGSSGSSGTCPPCPAPELLGGPSVFLFPPKPKDTLYIT REPEVTCVVVDVSHEDPEVKFNWYVDGVEVHNAKT KPREEQYNSTYRVVSVLTVLHQDWLNGKEYKCKVS NKALPAPIEKTISKAKGQPREPQVYTLPPSRDELTKNQ VSLTCLVKGFYPSDIAVEWESNGQPENNYKTTPPVLD SDGSFFLYSKLTVDKSRWQQGNVFSCSVMHEALHNH YTQKSLSLSPGK |
| 107 | GG-His-VDDDDK-hKLK1-GSSGSSG-IgG4 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSGSSGSSGGPPCPSCPAPEFLGGPSVFLFPPKPKDTL MISRTPEVTCVVVDVSQEDPEVQFNWYVDGVEVHN AKTKPREEQFNSTYRVVSVLTVLHQDWLNGKEYKC KVSNKGLPSSIEKTISKAKGQPREPQVYTLPPSQEEMT KNQVSLTCLVKGFYPSDIAVEWESNGQPENNYKTTPP VLDSDGSFFLYSRLTVDKSRWQEGNVFSCSVMHEAL HNHYTQKSLSLSLGK |
| 108 | GG-His-VDDDDK-hKLK1-GSSGSSG-YTE IgG4 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSGSSGSSGGPPCPSCPAPEFLGGPSVFLFPPKPKDTL YITREPEVTCVVVDVSQEDPEVQFNWYVDGVEVHN AKTKPREEQFNSTYRVVSVLTVLHQDWLNGKEYKC KVSNKGLPSSIEKTISKAKGQPREPQVYTLPPSQEEMT KNQVSLTCLVKGFYPSDIAVEWESNGQPENNYKTTPP VLDSDGSFFLYSRLTVDKSRWQEGNVFSCSVMHEAL HNHYTQKSLSLSLGK |
| 109 | GG-His-VDDDDK-hKLK1-GGGGSGGGGS-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTEEPEVGSTC LASGWGSIEPENFSFPDDLQCVDLKILPNDECKKAHV QKVTDFMLCVGHLEGGKDTCVGDSGGPLMCDGVL QGVTSWGYVPCGTPNKPSVAVRVLSYVKWIEDTIAE NSGGGGSGGGGSTCPPCPAPELLGGPSVFLFPPKPKD TLMISRTPEVTCVVVDVSHEDPEVKFNWYVDGVEVH NAKTKPREEQYNSTYRVVSVLTVLHQDWLNGKEYK CKVSNKALPAPIEKTISKAKGQPREPQVYTLPPSRDEL TKNQVSLTCLVKGFYPSDIAVEWESNGQPENNYKTTP PVLDSDGSFFLYSKLTVDKSRWQQGNVFSCSVMHEA LHNHYTQKSLSLSPGK |
| 110 | SP-GG-His-VDDDDK-hKLK1 (E121Q+A164V)-GSSGSSG-IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTQEPEVGSTCLASGWGSIEPENFSFPDDL QCVDLKILPNDECKKVHVQKVTDFMLCVGHLEGGK DTCVGDSGGPLMCDGVLQGVTSWGYVPCGTPNKPS VAVRVLSYVKWIEDTIAENSGSSGSSGTCPPCPAPELL GGPSVFLFPPKPKDTLMISRTPEVTCVVVDVSHEDPE VKFNWYVDGVEVHNAKTKPREEQYNSTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 111 | GG-His-VDDDDK-hKLK1 (E121Q+A164V)-GSSGSSG-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTQEPEVGSTC LASGWGSIEPENFSFPDDLQCVDLKILPNDECKKVHV QKVTDFMLCVGHLEGGKDTCVGDSGGPLMCDGVL QGVTSWGYVPCGTPNKPSVAVRVLSYVKWIEDTIAE NSGSSGSSGTCPPCPAPELLGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALHN HYTQKSLSLSPGK |
| 112 | His-VDDDDK-hKLK1 (E121Q+A164V)-GSSGSSG-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTQEPEVGSTCLAS GWGSIEPENFSFPDDLQCVDLKILPNDECKKVHVQK VTDFMLCVGHLEGGKDTCVGDSGGPLMCDGVLQG VTSWGYVPCGTPNKPSVAVRVLSYVKWIEDTIAENSG SSGSSGTCPPCPAPELLGGPSVFLFPPKPKDTLMISRTP EVTCVVVDVSHEDPEVKFNWYVDGVEVHNAKTKPR EEQYNSTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSDG SFFLYSKLTVDKSRWQQGNVFSCSVMHEALHNHYTQ KSLSLSPGK |
| 113 | VDDDDK-hKLK1 (E121Q+A164V)-GSSGSSG-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTQEPEVGSTCLASGWGSIEP ENFSFPDDLQCVDLKILPNDECKKVHVQKVTDFMLC VGHLEGGKDTCVGDSGGPLMCDGVLQGVTSWGYV PCGTPNKPSVAVRVLSYVKWIEDTIAENSGSSGSSGTC PPCPAPELLGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLSP GK |
| 114 | hKLK1 (E121Q+A164V)-GSSGSSG-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTQEPEVGSTCLASGWGSIEPENFSFP DDLQCVDLKILPNDECKKVHVQKVTDFMLCVGHLE GGKDTCVGDSGGPLMCDGVLQGVTSWGYVPCGTPN KPSVAVRVLSYVKWIEDTIAENSGSSGSSGTCPPCPAP ELLGGPSVFLFPPKPKDTLMISRTPEVTCVVVDVSHE DPEVKFNWYVDGVEVHNAKTKPREEQYNSTYRVVS VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGK |
| 115 | hKLK1 (E121Q+A164V) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTQEPEVGSTCLASGWGSIEPENFSFP DDLQCVDLKILPNDECKKVHVQKVTDFMLCVGHLE GGKDTCVGDSGGPLMCDGVLQGVTSWGYVPCGTPN KPSVAVRVLSYVKWIEDTIAENS |
| 116 | SP-GG-His-VDDDDK-hKLK1 (E121Q+A164V)-GSSGSSG-IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCCAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCAAAAAAGTCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA TCAAGTGGCTCTAGCGGTACATGCCCACCGTGCCC AGCACCTGAACTCCTGGGGGGACCGTCAGTCTTCC TCTTCCCCCCAAAACCCAAGGACACCCTCATGATCT CCCGGACCCCTGAGGTCACATGCGTGGTGGTGGAC GTGAGCCACGAAGACCCTGAGGTCAAGTTCAACTG GTACGTGGACGGCGTGGAGGTGCATAATGCCAAGA CAAAGCCGCGGGAGGAGCAGTACAACAGCACGTA CCGGGTGGTCAGCGTCCTCACCGTCCTGCACCAGG ACTGGCTGAATGGCAAGGAGTACAAGTGCAAGGTC TCCAACAAAGCCCTCCCAGCCCCCATCGAGAAAAC CATCTCCAAAGCCAAAGGGCAGCCCCGAGAACCA CAGGTGTACACCCTGCCCCCATCCCGGGATGAGCT GACCAAGAACCAGGTCAGCCTGACCTGCCTGGTCA AAGGCTTCTATCCCAGCGACATCGCCGTGGAGTGG GAGAGCAATGGGCAGCCGGAGAACAACTACAAGA CCACGCCTCCCGTGCTGGACTCCGACGGCTCCTTC TTCCTCTACAGCAAGCTCACCGTGGACAAGAGCAG GTGGCAGCAGGGGAACGTCTTCTCATGCTCCGTGA |
| | | TGCATGAGGCTCTGCACAACCACTACACGCAGAAG AGCCTCTCCCTGTCTCCGGGTAAATGA |
| 117 | hKLK1 (E121Q) | |
| 118 | hKLK1 (K162E) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECEKAHVQKVTDFMLCVGHLE GGKDTCVGDSGGPLMCDGVLQGVTSWGYVPCGTPN KPSVAVRVLSYVKWIEDTIAENS |
| 119 | hKLK1 (A164V) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECKKVHVQKVTDFMLCVGHLE GGKDTCVGDSGGPLMCDGVLQGVTSWGYVPCGTPN KPSVAVRVLSYVKWIEDTIAENS |
| 120 | hKLK1 (E121Q+K162E) | |
| 121 | hKLK1 (K162E+A164V) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTEEPEVGSTCLASGWGSIEPENFSFP DDLQCVDLKILPNDECEKVHVQKVTDFMLCVGHLE GGKDTCVGDSGGPLMCDGVLQGVTSWGYVPCGTPN KPSVAVRVLSYVKWIEDTIAENS |
| 122 | hKLK1 (E121Q+K162E+A 164V) | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTQEPEVGSTCLASGWGSIEPENFSFP DDLQCVDLKILPNDECEKVHVQKVTDFMLCVGHLE GGKDTCVGDSGGPLMCDGVLQGVTSWGYVPCGTPN KPSVAVRVLSYVKWIEDTIAENS |
| 123 | hKLK1 (E121Q+A164V) base sequence | ATTGTGGGAGGCTGGGAGTGTGAGCAGCATTCCCA GCCCTGGCAGGCGGCTCTGTACCATTTCAGCACTTT CCAGTGTGGGGGCATCCTGGTGCACCGCCAGTGGG TGCTCACAGCTGCTCATTGCATCAGCGACAATTACC AGCTCTGGCTGGGTCGCCACAACTTGTTTGACGAC GAAAACACAGCCCAGTTTGTTCATGTCAGTGAGAG CTTCCCACACCCTGGCTTCAACATGAGCCTCCTGG AGAACCACACCCGCCAAGCAGACGAGGACTACAG CCACGACCTCATGCTGCTCCGCCTGACAGAGCCTG CTGATACCATCACAGATGCTGTGAAGGTCGTGGAG TTGCCCACCCAGGAACCCGAAGTGGGGAGCACCT GTTTGGCTTCCGGCTGGGGCAGCATCGAACCAGAG AATTTCTCATTTCCAGATGATCTCCAGTGTGTGGAC CTCAAAATCCTGCCTAATGATGAGTGCAAAAAAGT CCACGTCCAGAAGGTGACAGACTTCATGCTGTGTG TCGGACACCTGGAAGGTGGCAAAGACACCTGTGT GGGTGATTCAGGGGGCCCGCTGATGTGTGATGGTG TGCTCCAAGGTGTCACATCATGGGGCTACGTCCCTT GTGGCACCCCCAATAAGCCTTCTGTCGCCGTCAGA GTGCTGTCTTATGTGAAGTGGATCGAGGACACCATA GCGGAGAACTCC |
| 124 | SP-GG-His-VDDDDK-hKLK1 (E121Q+K162E+A 164V)-GSSGSSG-IgG1 Fc | MWFLVLCLALSLGGTGAAGGHHHHHHVDDDDKIVG GWECEQHSQPWQAALYHFSTFQCGGILVHRQWVLT AAHCISDNYQLWLGRHNLFDDENTAQFVHVSESFPH PGFNMSLLENHTRQADEDYSHDLMLLRLTEPADTITD AVKVVELPTQEPEVGSTCLASGWGSIEPENFSFPDDL QCVDLKILPNDECEKVHVQKVTDFMLCVGHLEGGK DTCVGDSGGPLMCDGVLQGVTSWGYVPCGTPNKPS VAVRVLSYVKWIEDTIAENSGSSGSSGTCPPCPAPELL GGPSVFLFPPKPKDTLMISRTPEVTCVVVDVSHEDPE VKFNWYVDGVEVHNAKTKPREEQYNSTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 125 | GG-His-VDDDDK-hKLK1 (E121Q+K162E+A 164V)-GSSGSSG-IgG1 Fc | GGHHHHHHVDDDDKIVGGWECEQHSQPWQAALYH FSTFQCGGILVHRQWVLTAAHCISDNYQLWLGRHNL FDDENTAQFVHVSESFPHPGFNMSLLENHTRQADED YSHDLMLLRLTEPADTITDAVKVVELPTQEPEVGSTC LASGWGSIEPENFSFPDDLQCVDLKILPNDECEKVHV QKVTDFMLCVGHLEGGKDTCVGDSGGPLMCDGVL QGVTSWGYVPCGTPNKPSVAVRVLSYVKWIEDTIAE NSGSSGSSGTCPPCPAPELLGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALHN HYTQKSLSLSPGK |
| 126 | His-VDDDDK-hKLK1 (E121Q+K162E+A 164V)-GSSGSSG-IgG1 Fc | HHHHHHVDDDDKIVGGWECEQHSQPWQAALYHFST FQCGGILVHRQWVLTAAHCISDNYQLWLGRHNLFDD ENTAQFVHVSESFPHPGFNMSLLENHTRQADEDYSH DLMLLRLTEPADTITDAVKVVELPTQEPEVGSTCLAS GWGSIEPENFSFPDDLQCVDLKILPNDECEKVHVQKV TDFMLCVGHLEGGKDTCVGDSGGPLMCDGVLQGVT SWGYVPCGTPNKPSVAVRVLSYVKWIEDTIAENSGSS GSSGTCPPCPAPELLGGPSVFLFPPKPKDTLMISRTPEV TCVVVDVSHEDPEVKFNWYVDGVEVHNAKTKPREE QYNSTYRVVSVLTVLHQDWLNGKEYKCKVSNKALP APIEKTISKAKGQPREPQVYTLPPSRDELTKNQVSLTC LVKGFYPSDIAVEWESNGQPENNYKTTPPVLDSDGSF FLYSKLTVDKSRWQQGNVFSCSVMHEALHNHYTQK SLSLSPGK |
| 127 | VDDDDK-hKLK1 (E121Q+K162E+A 164V)-GSSGSSG-IgG1 Fc | VDDDDKIVGGWECEQHSQPWQAALYHFSTFQCGGIL VHRQWVLTAAHCISDNYQLWLGRHNLFDDENTAQF VHVSESFPHPGFNMSLLENHTRQADEDYSHDLMLLR LTEPADTITDAVKVVELPTQEPEVGSTCLASGWGSIEP ENFSFPDDLQCVDLKILPNDECEKVHVQKVTDFMLC VGHLEGGKDTCVGDSGGPLMCDGVLQGVTSWGYV PCGTPNKPSVAVRVLSYVKWIEDTIAENSGSSGSSGTC PPCPAPELLGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLSP GK |
| 128 | hKLK1 (E121Q+K162E+A 164V)-GSSGSSG-IgG1 Fc | IVGGWECEQHSQPWQAALYHFSTFQCGGILVHRQW VLTAAHCISDNYQLWLGRHNLFDDENTAQFVHVSES FPHPGFNMSLLENHTRQADEDYSHDLMLLRLTEPAD TITDAVKVVELPTQEPEVGSTCLASGWGSIEPENFSFP DDLQCVDLKILPNDECEKVHVQKVTDFMLCVGHLE GGKDTCVGDSGGPLMCDGVLQGVTSWGYVPCGTPN KPSVAVRVLSYVKWIEDTIAENSGSSGSSGTCPPCPAP ELLGGPSVFLFPPKPKDTLMISRTPEVTCVVVDVSHE DPEVKFNWYVDGVEVHNAKTKPREEQYNSTYRVVS VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGK |
| 129 | SP-GG-His-VDDDDK-hKLK1 (E121Q+K162E+A 164V)-GSSGSSG-IgG1 Fc (base sequence) | ATGTGGTTCCTGGTTCTGTGCCTCGCCCTGTCCCTG GGGGGGACTGGTGCTGCGGGTGGCCACCATCATCA TCATCATGTAGATGACGATGACAAGATTGTGGGAG GCTGGGAGTGTGAGCAGCATTCCCAGCCCTGGCAG GCGGCTCTGTACCATTTCAGCACTTTCCAGTGTGGG GGCATCCTGGTGCACCGCCAGTGGGTGCTCACAGC TGCTCATTGCATCAGCGACAATTACCAGCTCTGGCT GGGTCGCCACAACTTGTTTGACGACGAAAACACAG CCCAGTTTGTTCATGTCAGTGAGAGCTTCCCACACC CTGGCTTCAACATGAGCCTCCTGGAGAACCACACC CGCCAAGCAGACGAGGACTACAGCCACGACCTCAT GCTGCTCCGCCTGACAGAGCCTGCTGATACCATCA CAGATGCTGTGAAGGTCGTGGAGTTGCCCACCCAG GAACCCGAAGTGGGGAGCACCTGTTTGGCTTCCGG CTGGGGCAGCATCGAACCAGAGAATTTCTCATTTCC AGATGATCTCCAGTGTGTGGACCTCAAAATCCTGCC TAATGATGAGTGCGAAAAAGTCCACGTCCAGAAGG TGACAGACTTCATGCTGTGTGTCGGACACCTGGAA GGTGGCAAAGACACCTGTGTGGGTGATTCAGGGGG CCCGCTGATGTGTGATGGTGTGCTCCAAGGTGTCA CATCATGGGGCTACGTCCCTTGTGGCACCCCCAATA AGCCTTCTGTCGCCGTCAGAGTGCTGTCTTATGTGA AGTGGATCGAGGACACCATAGCGGAGAACTCCGGA TCAAGTGGCTCTAGCGGTACATGCCCACCGTGCCC AGCACCTGAACTCCTGGGGGGACCGTCAGTCTTCC TCTTCCCCCCAAAACCCAAGGACACCCTCATGATCT CCCGGACCCCTGAGGTCACATGCGTGGTGGTGGAC GTGAGCCACGAAGACCCTGAGGTCAAGTTCAACTG GTACGTGGACGGCGTGGAGGTGCATAATGCCAAGA CAAAGCCGCGGGAGGAGCAGTACAACAGCACGTA CCGGGTGGTCAGCGTCCTCACCGTCCTGCACCAGG ACTGGCTGAATGGCAAGGAGTACAAGTGCAAGGTC TCCAACAAAGCCCTCCCAGCCCCCATCGAGAAAAC CATCTCCAAAGCCAAAGGGCAGCCCCGAGAACCA CAGGTGTACACCCTGCCCCCATCCCGGGATGAGCT GACCAAGAACCAGGTCAGCCTGACCTGCCTGGTCA |
| | | AAGGCTTCTATCCCAGCGACATCGCCGTGGAGTGG GAGAGCAATGGGCAGCCGGAGAACAACTACAAGA CCACGCCTCCCGTGCTGGACTCCGACGGCTCCTTC TTCCTCTACAGCAAGCTCACCGTGGACAAGAGCAG GTGGCAGCAGGGGAACGTCTTCTCATGCTCCGTGA TGCATGAGGCTCTGCACAACCACTACACGCAGAAG AGCCTCTCCCTGTCTCCGGGTAAATGACTCGAGTG C |
| 130 | hKLK1 (E121Q+K162E+A 164V) base sequence | ATTGTGGGAGGCTGGGAGTGTGAGCAGCATTCCCA GCCCTGGCAGGCGGCTCTGTACCATTTCAGCACTTT CCAGTGTGGGGGCATCCTGGTGCACCGCCAGTGGG TGCTCACAGCTGCTCATTGCATCAGCGACAATTACC AGCTCTGGCTGGGTCGCCACAACTTGTTTGACGAC GAAAACACAGCCCAGTTTGTTCATGTCAGTGAGAG CTTCCCACACCCTGGCTTCAACATGAGCCTCCTGG AGAACCACACCCGCCAAGCAGACGAGGACTACAG CCACGACCTCATGCTGCTCCGCCTGACAGAGCCTG CTGATACCATCACAGATGCTGTGAAGGTCGTGGAG TTGCCCACCCAGGAACCCGAAGTGGGGAGCACCT GTTTGGCTTCCGGCTGGGGCAGCATCGAACCAGAG AATTTCTCATTTCCAGATGATCTCCAGTGTGTGGAC CTCAAAATCCTGCCTAATGATGAGTGCGAAAAAGT CCACGTCCAGAAGGTGACAGACTTCATGCTGTGTG TCGGACACCTGGAAGGTGGCAAAGACACCTGTGT GGGTGATTCAGGGGGCCCGCTGATGTGTGATGGTG TGCTCCAAGGTGTCACATCATGGGGCTACGTCCCTT GTGGCACCCCCAATAAGCCTTCTGTCGCCGTCAGA GTGCTGTCTTATGTGAAGTGGATCGAGGACACCATA GCGGAGAACTCC |

### Examples

The biological materials involved in all the examples, such as strains, various cloning and expression plasmids, culture media, tool enzymes, and buffers, as well as various culture methods, protein extraction and purification methods, and other molecular biology protocols are familiar to those skilled in the art, and can be made reference to "Molecular Cloning" edited by Sambrook et al. (Laboratory Manual, Cold Spring Harbor, 1989) and "Short Protocols in Molecular Biology" (edited by U.S./F. M. Ausubel et al., translated by Yan Ziying et al., Beijing, Science Press, 1998).

### Example 1: Plasmid construction and expression of KLK1 fusion protein

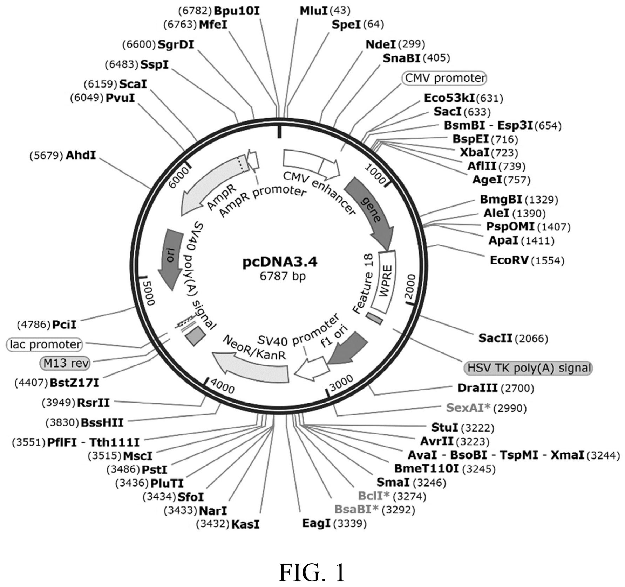

The target gene encoding KLK1 fusion protein was synthesized, and then inserted into the AgeI and EcoRV sites in the pcDNA3.4 vector (as shown in FIG. 1) to construct the plasmid pcDNA3.4-KLK1-Fc.

The constructed plasmid pcDNA3.4-KLK1-Fc was transformed into *E. coli* DH5α competent cells, and then selected on LB agar plate comprising ampicillin (Amp+). Then, a single colony was picked up and cultured in LB culture medium comprising a corresponding antibiotic with shaking, and the plasmid was extracted. Then the extracted plasmid was transfected into HEK293 cells and the target protein was expressed in Expi293^{™} expression system.

The constructed plasmids, the inserted target genes and the expressed fusion proteins are shown below, where SP in the name of each plasmid represents a signal peptide; GG represents two glycines; His represents a histidine tag; VDDDDK represents an enterokinase recognition sequence; hKLK1 represents human KLK1; GGGSSSS, GSSGSSG, GGGGSGGGGS, and EAAAK represent linkers; IgG1 Fc represents an Fc domain derived from human IgG1; YTE IgG1 Fc represents an Fc domain derived from human IgG1 with the mutations M252Y, S254T, and T256E; IgG4 Fc represents an Fc domain derived from human IgG4; and YTE IgG4 Fc represents an Fc domain derived from human IgG4 with the mutations M252Y, S254T, and T256E.
- The target gene inserted into the plasmid pcDNA3.4-SP-GG-His-VDDDDK-hKLK1-IgG1 Fc is as shown in SEQ ID NO: 66, and the expressed fusion protein (referred to as "intermediate of fusion protein 1" hereinafter) comprises an amino acid sequence as shown in SEQ ID NO: 100.
- The target gene inserted into the plasmid pcDNA3.4-SP-GG-His-VDDDDK-hKLK1-GGGSSSS-IgG1 Fc is as shown in SEQ ID NO: 67, and the expressed fusion protein (referred to as "intermediate of fusion protein 2.1" hereinafter) comprises an amino acid sequence as shown in SEQ ID NO: 101.
- The target gene inserted into the plasmid pcDNA3.4-SP-GG-His-VDDDDK-hKLK1-EAAAK-IgG1 Fc is as shown in SEQ ID NO: 68, and the expressed fusion protein (referred to as "intermediate of fusion protein 2.2" hereinafter) comprises an amino acid sequence as shown in SEQ ID NO: 102.
- The target gene inserted into the plasmid pcDNA3.4-IL2-GG-His-VDDDDK-hKLK1-GGGSSSS-IgG1 Fc is as shown in SEQ ID NO: 69, and the expressed fusion protein (referred to as "intermediate of fusion protein 2.3" hereinafter) comprises an amino acid sequence as shown in SEQ ID NO: 103.
- The target gene inserted into the plasmid pcDNA3.4-IL2-GG-His-VDDDDK-hKLK1-EAAAK-IgG1 Fc is as shown in SEQ ID NO: 70, and the expressed fusion protein (referred to as "intermediate of fusion protein 2.4" hereinafter) comprises an amino acid sequence as shown in SEQ ID NO: 104.
- The target gene inserted into the plasmid pcDNA3.4-SP-GG-His-VDDDDK-hKLK1-GSSGSSG-IgG1 Fc is as shown in SEQ ID NO: 71, and the expressed fusion protein (referred to as "intermediate of fusion protein 3.1" hereinafter) comprises an amino acid sequence as shown in SEQ ID NO: 105.
- The target gene inserted into the plasmid pcDNA3.4-SP-GG-His-VDDDDK-hKLK1-GSSGSSG-YTE IgG1 Fc is as shown in SEQ ID NO: 72, and the expressed fusion protein (referred to as "intermediate of fusion protein 3.2" hereinafter) comprises an amino acid sequence as shown in SEQ ID NO: 106.
- The target gene inserted into the plasmid pcDNA3.4-SP-GG-His-VDDDDK-hKLKI-GSSGSSG-IgG4 Fc is as shown in SEQ ID NO: 73, and the expressed fusion protein (referred to as "intermediate of fusion protein 3.3" hereinafter) comprises an amino acid sequence as shown in SEQ ID NO: 107.
- The target gene inserted into the plasmid pcDNA3.4-SP-GG-His-VDDDDK-hKLK1-GSSGSSG-YTE IgG4 Fc is as shown in SEQ ID NO: 74, and the expressed fusion protein (referred to as "intermediate of fusion protein 3.4" hereinafter) comprises an amino acid sequence as shown in SEQ ID NO: 108.
- The target gene inserted into the plasmid pcDNA3.4-SP-GG-His-VDDDDK-hKLK1-GGGGSGGGGS-IgG1 Fc is as shown in SEQ ID NO: 75, and the expressed fusion protein (referred to as "intermediate of fusion protein 3.5" hereinafter) comprises an amino acid sequence as shown in SEQ ID NO: 109.
- The target gene inserted into the plasmid pcDNA3.4-SP-prepro-hKLK1-EAAAK-IgG1 Fc is as shown in SEQ ID NO: 97, and the expressed fusion protein (referred to as "fusion protein 3.6" hereinafter) comprises an amino acid sequence as shown in SEQ ID NO: 93.
- The target gene inserted into the plasmid pcDNA3.4-SP-prepro-hKLK1 is as shown in SEQ ID NO: 98, and the expressed protein (referred to as "protein 4.1" hereinafter) comprises an amino acid sequence as shown in SEQ ID NO: 94.
- The target gene inserted into the plasmid pcDNA3.4-SP-prepro-VDDDDK-hKLK1 is as shown in SEQ ID NO: 99, and the expressed protein (referred to as "intermediate of protein 4.2" hereinafter) comprises an amino acid sequence as shown in SEQ ID NO: 95.
- The target gene inserted into the plasmid pcDNA3.4-SP-prepro-VDDDDK-hKLK1-EAAAK-IgG1 Fc is as shown in SEQ ID NO: 90, and the expressed fusion protein (referred to as "intermediate of fusion protein 4.3" hereinafter) comprises an amino acid sequence as shown in SEQ ID NO: 96.
- The target gene inserted into the plasmid pcDNA3.4-SP-GG-His-VDDDDK-hKLK1 (E121Q+A164V)-GSSGSSG-IgG1 Fc is as shown in SEQ ID NO: 116, and the expressed fusion protein (referred to as "intermediate of fusion protein 5.1" hereinafter) comprises an amino acid sequence as shown in SEQ ID NO: 111.
- The target gene inserted into the plasmid pcDNA3.4-SP-GG-His-VDDDDK-hKLK1 (E121Q+K162E+A164V)-GSSGSSG-IgG1 Fc is as shown in SEQ ID NO: 129, and the expressed fusion protein (referred to as "intermediate of fusion protein 6.1" hereinafter) comprises an amino acid sequence as shown in SEQ ID NO: 125.

### Example 2: Purification of KLK1 fusion proteins

### 2.1. Preliminary purification of KLK1 fusion proteins

Several proteins (for example: intermediate of fusion protein 1, intermediate of fusion protein 2.1, intermediate of fusion protein 2.2, intermediate of fusion protein 2.3, intermediate of fusion protein 2.4, intermediate of fusion protein 3.1, intermediate of fusion protein 3.2, intermediate of fusion protein 3.3, intermediate of fusion protein 3.4, intermediate of fusion protein 3.5, fusion protein 3.6, protein 4.1, intermediate of protein 4.2, and intermediate of fusion protein 4.3) prepared in Example 1 were passed through a Ni-NTA column as follows: The conditioned medium was harvested by centrifugation (4°C, 4000 rpm and 40 min). Then the cell debris was removed by filtration, and the remaining solution was exchanged into PBS buffer supplemented with 0.8 mM EDTA. 300 ml of clarified culture medium was loaded into a 5 ml Ni-NTA column (purchased from GE), which was pre-equilibrated with buffer A (25 mM Tris, 150 mM NaCl, 10 mM imidazole, pH 8.0), at a flow rate of 3 ml/min. The column was washed with 20 column volumes (CVs) of equilibration buffer (25 mM Tris, 150 mM NaCl, 10 mM imidazole, pH 8.0), and then eluted with an eluent buffer (25 mM Tris, 150 mM NaCl, 500 mM imidazole, pH 8.0). The eluate was dialyzed to PBS pH 7.4, and finally filtered through MILLEX-MP 0.22 µm (purchased from MILLIPORE).

Then, the protein intermediate purified by Ni-NTA column were purified by Protein A column. Specifically, the conditioned medium was harvested by centrifugation (4°C, centrifuged at 4000 rpm and 40 min), and then filtered to remove cell debris. 50 ml of clarified culture medium was loaded into a 1 ml MabSelect SuRe Protein A column (purchased from GE), which was pre-equilibrated with an equilibration buffer (25 mM Tris, 150 mM NaCl, pH 8.0), at a flow rate of 1ml/min. The column was washed with 20 CVs of equilibration buffer (25 mM Tris, 150 mM NaCl, pH 8.0), and then the column loaded with protein was eluted with an eluent buffer (50 mM sodium citrate, 150 mM NaCl, pH 3.0). Afterwards, the eluate was neutralized with a neutralization buffer (1 M arginine, 400 mM succinic acid, pH 9.0) (1/10 volume of the eluate). The eluate was dialyzed directly to PBS pH 7.4, and finally filtered through MILLEX-MP 0.22 µm (purchased from MILLIPORE).

### 2.2. Cleavage by EK enzyme

The KLK1 fusion protein comprising an EK recognition sequence (VDDDDK) preliminarily purified in Example 2.1 was enzymatically cleaved with Histagged EK enzyme, by incubation overnight at KLK1 fusion protein: EK enzyme= 50: 1 (mg: mg) at 20-25°C. The amino acid sequences of human KLK1 protein or KLK1 fusion protein obtained by cleavage with the EK enzyme are respectively as shown in SEQ ID NOs: 56 (also referred to as "fusion protein 1"), SEQ ID NO: 57 (also referred to as "fusion protein 2.1"), SEQ ID NO: 58 (also referred to as "fusion protein 2.2" or "fusion protein 4.3"), SEQ ID NO: 59 (also referred to as "fusion protein 2.3"), SEQ ID NO: 60 (also referred to as "fusion protein 2.4"), SEQ ID NO: 61 (also referred to as "fusion protein 3.1"), SEQ ID NO: 62 (also referred to as "fusion protein 3.2"), SEQ ID NO: 63 (also referred to as "fusion protein 3.3"), SEQ ID NO: 64 (also referred to as "fusion protein 3.4"), SEQ ID NO: 65 (also referred to as "fusion protein 3.5"), SEQ ID NO: 2 (also referred to as "protein 4.2").

### 2.3. Further purification after enzymatic cleavage

The human KLK1 protein or KLK1 fusion protein obtained after the enzymatic cleavage in Example 2.2 was purified by a nickel column again, and the mixed product after the enzymatic cleavage was passed through a nickel column again. The final product was collected, the concentration was determined, filtered through a MILLEX-MP 0.22 µm filter membrane and stored at -80°C.

### Example 3: Characterization of KLK1 fusion protein

### 3.1. Methods of disulfide bond analysis

The protein prepared in Example 2.3 was used to prepare reduced and non-reduced samples respectively. For the reduced sample, a proper amount of the sample was added to a centrifuge tube, and a denaturing buffer was added and mixed well. Then a DTT solution was added, mixed well and incubated, to reduce the sample. Iodoacetamide was added to the reduced sample, mixed well and incubated, to alkylate the sample. For the non-reduced sample, a proper amount of the sample was added to a centrifuge tube, a buffer was added to dilute the sample to the required concentration, and the sample was marked.

The prepared reduced and non-reduced samples were transferred to an ultrafiltration tube respectively, and a buffer was added, followed by centrifugation and ultrafiltration. The buffer was added again, and the sample was centrifuged and ultrafiltered to the target concentration, and mixed well. PNGase F enzyme was added to the desalted sample, and incubated for deglycosylation by enzymolysis. A proper amount of trypsin/chymotrypsin/Glu-C was added to the deglycosylated sample, and incubated for enzymatic cleavage. After the reaction, the sample was transferred to an injection vial, and the enzymatically cleaved fragments were separated by UPLC. After that, the position and structure of cysteine-SH residue of the disulfide bond in the protein were confirmed by mass spectrometry.

### 3.2. Methods of molecule weight analysis

### 3.2.1. Analysis of molecular weight after deglycosylation and reduction

### (a) Preparation of deglycosylated and reduced protein sample

The name of the sample was marked on the centrifuge tube, and the sample was concentrated in an ultrafiltration tube. 50 µg of the protein sample (in solution) was added to the centrifuge tube, a proper amount of 5xQuick reaction buffer (reducing) was added, mixed uniformly, and incubated at 75°C. The reaction solution was cooled to room temperature. A proper amount of Quick PNGase F enzyme was added, and incubated at 75°C. The sample buffer was replaced by 100 mM ammonium bicarbonate solution, and centrifuged at 13000 rpm for 10 min. The supernatant was transferred to a sample vial, and 2 µL of sample was injected and analyzed.

After the reaction, the sample was transferred to an injection vial, and the prepared sample was separated by HPLC. Then the molecular weight of the deglycosylated sample was determined by mass spectrometry.

### (b) Instrument parameters

Parameters of HPLC (purchased from Agilent, Model 1290 Infinity II):
- Chromatographic column: Waters, BioResolve, RP mAb, Polyphenyl, 450A, 2.1 x 50 mm, 2.7 µm
- Column temperature: 75°C
- Automatic sampler temperature: 8°C
- Mobile phase: Mobile phase A (deionized water, with 0.1% formic acid); Mobile phase B (acetonitrile, with 0.1% formic acid).

Parameters of mass spectrometer (purchased from Agilent, Model 6545XT):
Scan range: 50-3200 m/z
Ion polarity: positive
ESI GAS Temp: 325°C
Fragmentor Voltage: 225V

### (c) Detection results

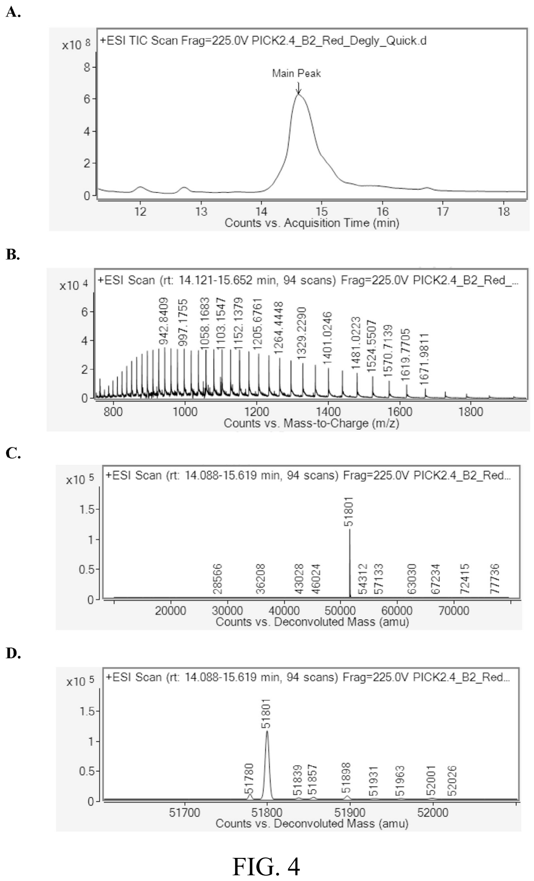

Taking fusion protein 2.4 as an example (the characterization results of other proteins prepared in Example 2.3 are not shown), the total ion current (TIC) spectrum, the mass spectrometry (MS) spectrum and the deconvoluted spectrum of fusion protein 2.4 are shown in FIG. 4. The detection results shows that the molecular weight of the deglycosylated and reduced fusion protein 2.4 is 51801 Da, which is consistent with the molecular weight of the target protein.

### 3.2.2. Analysis of intact molecular weight after deglycosylation

### (a) Preparation of deglycosylated complete protein sample

The name of the sample was marked on the centrifuge tube, and 50 µg of the protein sample solution was added to the centrifuge tube. A proper amount of 5xQuick reaction buffer (non-reducing) was added, mixed uniformly, and incubated at 75°C. The reaction solution was cooled to room temperature. A proper amount of Quick PNGase F enzyme was added, and incubated at 50°C. The sample buffer was replaced by 100 mM ammonium bicarbonate solution, and centrifuged at 13000 rpm for 10 min. The supernatant was transferred to a sample vial, and 2 µl of sample was injected and analyzed.

### (b) Instrument parameters

Parameters of HPLC (purchased from Agilent, Model 1290 Infinity II):
- Chromatographic column: Waters, BioResolve, RP mAb, Polyphenyl, 450A, 2.1 x 50 mm, 2.7 µm
- Column temperature: 75°C
- Automatic sampler temperature: 8°C
- Mobile phase: Mobile phase A (deionized water, with 0.1% formic acid); Mobile phase B (acetonitrile, with 0.1% formic acid).

Parameters of mass spectrometer (purchased from Agilent, Model 6545XT):
Scan range: 500-5000 m/z
Ion polarity: positive
ESI GAS Temp: 325°C
Fragmentor Voltage: 380V

### (c) Detection results

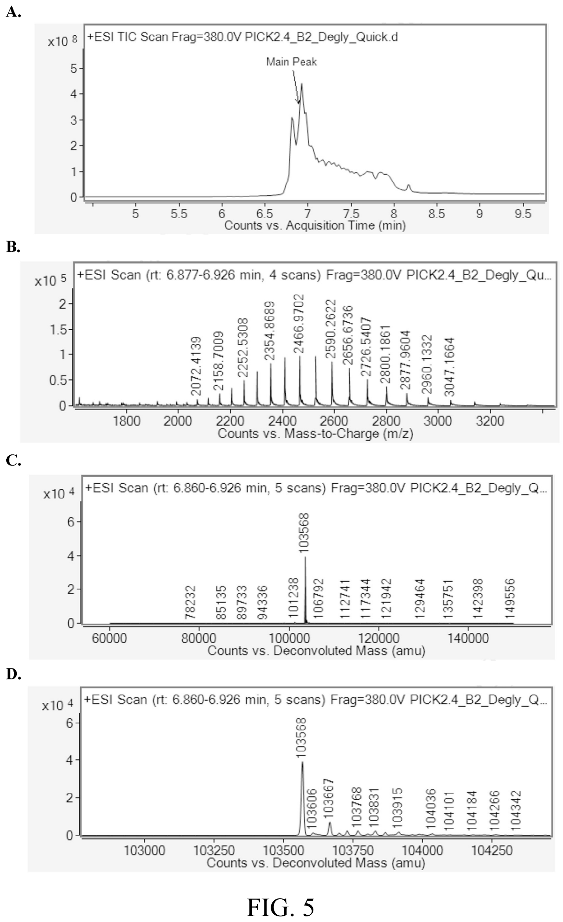

Taking fusion protein 2.4 as an example (the characterization results of other proteins prepared in Example 2.3 are not shown), the total ion current (TIC) spectrum, the mass spectrometry (MS) spectrum and the deconvoluted spectrum of fusion protein 2.4 are shown in FIG. 5. The detection results shows that the intact molecular weight of the deglycosylated fusion protein 2.4 is 103568 Da,

### 3.3. Methods of sequence coverage analysis

### 3.3.1. Sample denaturation, reduction, and alkylation

The required sample volume was calculated by the formula:

Required sample volume (µL) =300 (µg)/protein content in sample (µg/µL).

300 µg of the sample solution to be tested was transferred into an ultrafiltration tube, and centrifuged at 12000 rpm for 10 min. A denaturation solution was added to the 100 µL scale of the ultrafiltration tube, mixed, and transferred to a 1.5 mL centrifuge tube. 200 µL of the denaturation solution was added to give a final volume of 300 µL, and mixed well. 5 µL of 1 M DTT solution was added, and mixed well. The mixture was incubated at 37°C for 60 min. 10 µL of 1 M iodoacetamide solution was added, and mixed well. The mixture was incubated at 10-30°C for 15 min (3 samples were prepared in parallel: Group 1, Group 2, and Group 3).

### 3.3.2. Desalting of sample

Groups 1 and 2: The sample names were marked on the ultrafiltration tubes respectively, and 300 µL of 100 mM ammonium bicarbonate solution was added to each ultrafiltration tube, and centrifuged at 12000 rpm for 5 min. The samples were transferred to ultrafiltration tubes respectively, and centrifuged at 12000 rpm for 10 min. The samples were washed twice with 300 µL of 100 mM ammonium bicarbonate solution, and centrifuged at 12,000 rpm for 10 min each time. 300 µL of 100 mM ammonium bicarbonate solution was added to each ultrafiltration tube, and centrifuged at 12000 rpm for 15 min. 100 mM ammonium bicarbonate solution was added to the 100 µL scale line of the ultrafiltration tube, mixed uniformly, and then transferred to a centrifuge tube. 100 µL of 100 mM ammonium bicarbonate solution was added (the final concentration of the sample was about 1.5 mg/mL) and mixed well.

Group 3: The 100 mM ammonium bicarbonate solution in the operation steps of Groups 1 and 2 was replaced by 100 mM phosphate buffer, and the other experimental operations were the same.

### 3.3.3 Acidolysis/deglycosylation

Enzymolysis: 15 µL of a trypsin solution was added to the sample of Group 1, 15 µL of chymotrypsin was added to the sample of Group 2, and 15 µL of Glu-C protease was added to the sample of Group 3, followed by incubation at 37°C for 120 min respectively.

Deglycosylation and desialylation: 2 µL of Quick PNGase F solution and 2 µL of α 2-3,6,8,9 Neuraminidase A were added to the samples of Group 1, Group 2 and Group 3 respectively, and incubated at 37°C for 60 min. 5 µL of 10% formic acid solution was added to each centrifuge tube, and mixed uniformly. Then, the samples were prepared.

The prepared samples were transferred to the injection vials. The amino acid sequences of the peptide fragment of various enzymatically cleaved samples were analyzed by MS and MS/MS, and matched with the theoretical sequence of the sample to determine the sequence coverage of the test sample.

### Example 4: Determination of KLK enzyme activity

### 4.1. Methods

The protein content in the purified sample in Example 2.3 was determined using BCA protein assay kit (Bradford method) or Pierce^{™} 660 nm protein assay reagent (Biuret method).

Firstly, the following reagents were prepared:
- Incubation buffer: 200 mM Tris-HCl, pH 8.0
- Sample dilution buffer: 20 mM Tris-HCl, pH 8.0
- Reagents: 100 mm DMSO stock solutions of 4-nitroaniline and H-D-Val-Leu-Arg-pNA were prepared respectively.
- Samples: The human KLK1 protein or KLK1 fusion protein sample purified in Example 2.3 were stored at -80°C and thawed at room temperature. The undiluted stock solution was used to determine the protein concentration, and the 2-fold, 5-fold, and 10-fold dilutions were used to determine the enzymatic activity of KLK1.
- Positive control sample (Kailikang^{®}): The powder in the vial was dissolved in 100 µl of sample dilution buffer. 50 µl of the sample solution was used for protein concentration determination, and the remaining sample solution was 2-fold, 5-fold, and 10-fold diluted and used for enzymatic activity determination of KLK1.

Then, the enzymatic activity of each sample was determined as follows:
1) The appropriate range of a PNA standard curve was determined. 0 mM, 0.0625 mM, 0.125 mM, 0.25 mM, 0.5 mM and 1 mM PNA in the incubation buffer without the sample or substrate were measured at 405 nm using a spectrophotometer.
2) The diluted sample and incubation buffer were added to a detection plate, and incubated for 5 min. The background value of the plate was measured at a wavelength of 405 nm using a spectrophotometer.
3) 20 ml of H-D-Val-Leu-Arg-pNA was added to initiate the reaction, and the kinetic values of the plate were measured at 405 nm using a spectrophotometer at 37°C for 15 min.
4) A standard curve of PNA (p-nitroaniline) over concentrations of 0-100 nmol was plotted. The exact amount of PNA released from the sample was calculated according to the PNA standard curve, from which the enzymatic activity of KLK1 in the sample was calculated. The enzymatic activity of KLK1 in the test sample is described as the amount of PNA produced by cleaving the substrate by 1 mg of KLK1 fusion protein per minute, in µmole/min/mg (PNAU/mg). The higher value indicates the higher enzymatic activity.

### 4.2. Results

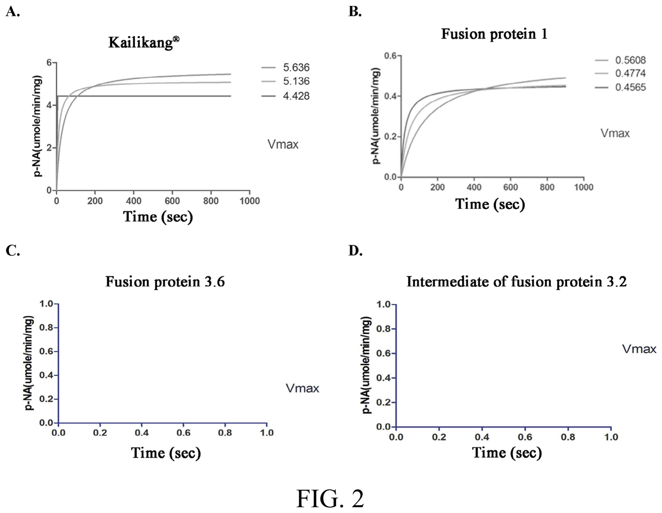

As shown in Table 2 and FIG. 2, KLK1 fusion proteins which have an EK recognition sequence in the preparation process and are obtained after cleavage by EK enzyme all have the enzymatic activity of KLK1, and KLK1 fusion protein without an EK recognition sequence in the preparation process or KLK1 fusion protein with EK recognition sequence and without cleavage by EK enzyme have no enzymatic activity of KLK1.

**Table 2. Comparison of enzymatic activities of various KLK1 fusion protein**

| **Sample name** | **Description** | **KLK1 enzyme activity (µmol/min/mg)** | **Conclusion** |
|---|---|---|---|
| Kailikang^{®} | Positive control | 4.428-5.636 | Enzymatically active |
| Fusion protein 1 | hKLK1 fusion protein formed after cleavage of intermediate of fusion protein 1 by EK enzyme (that is, SEQ ID NO: 56) | 0.4565-0.5608 | Enzymatically active |
| Fusion protein 3.6 | hKLK1 fusion protein without EK recognition sequence (that is, SEQ ID NO: 93) | NA | Nonenzymatically active |
| Intermediate of fusion protein 3.2 | KLK1 fusion protein with EK recognition sequence and without cleavage by EK enzyme (that, SEQ ID NO: 42) | NA | Nonenzymatically active |

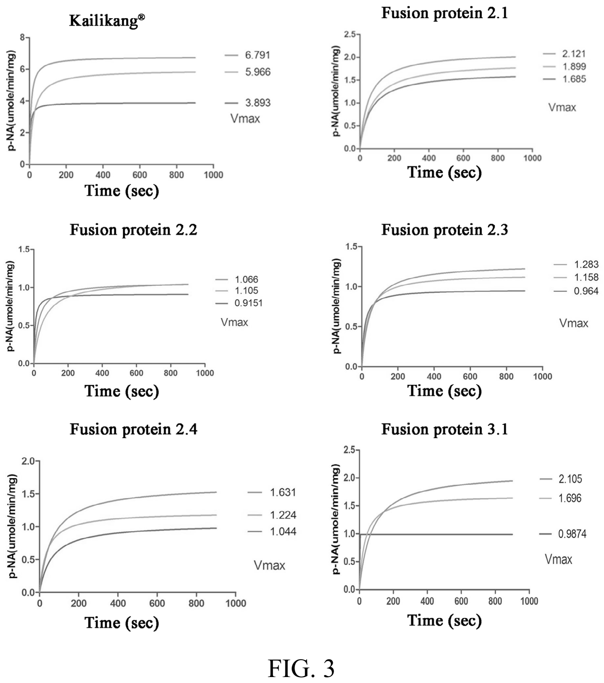

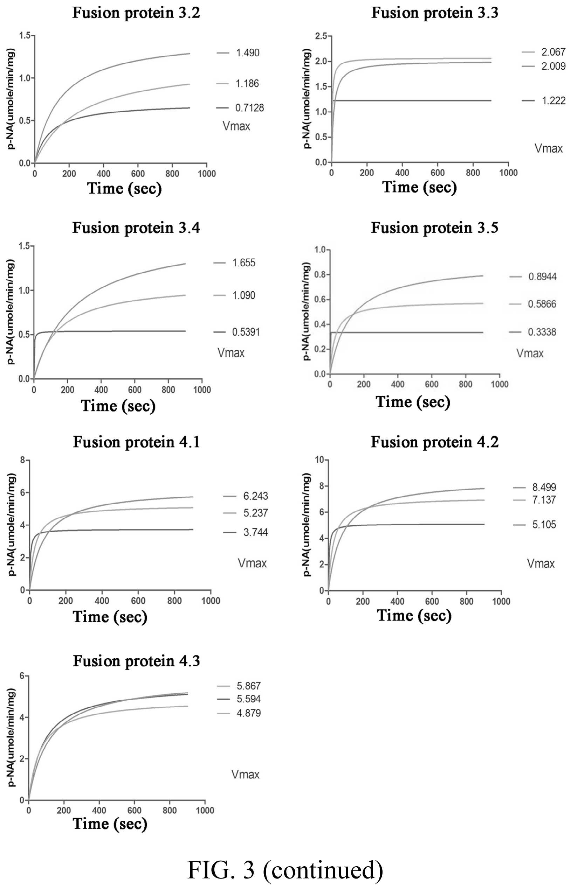

As shown in Table 3 and FIG. 3, Human KLK1 protein or KLK1 fusion proteins with different sequences were formed after cleavage by EK enzyme (which had an EK recognition sequence in the preparation process), and all the formed Human KLK1 protein or KLK1 fusion proteins have the enzymatic activity of KLK1.

**Table 3. Comparison of enzymatic activities of various KLK1 fusion protein**

| **Sample name** | **Description** | **KLK1 enzyme activity (µmol/min/mg)** | **Conclusion** |
|---|---|---|---|
| Kailikang^{®} | Positive control | 3.893-6.791 | Enzymatically active |
| Fusion protein 2.1 | hKLK1 fusion protein formed after cleavage of intermediate of fusion protein 2.1 by EK enzyme (that is, SEQ ID NO: 57) | 1.685-2.121 | Enzymatically active |
| Fusion protein 2.2 | hKLK1 fusion protein formed after cleavage of intermediate of fusion protein 2.2 by EK enzyme (that is, SEQ ID NO: 58) | 0.9151-1.105 | Enzymatically active |
| Fusion protein 2.3 | hKLK1 fusion protein formed after cleavage of intermediate of fusion protein 2.3 by EK enzyme (that is, SEQ ID NO: 59) | 0.964-1.283 | Enzymatically active |
| Fusion protein 2.4 | hKLK1 fusion protein formed after cleavage of intermediate of fusion protein 2.4 by EK enzyme (that is, SEQ ID NO: 60) | 1.044-1.631 | Enzymatically active |
| Fusion protein 3.1 | hKLK1 fusion protein formed after cleavage of intermediate of fusion protein 3.1 by EK enzyme (that is, SEQ ID NO: 61) | 0.9874-2.105 | Enzymatically active |
| Fusion protein 3.2 | hKLK1 fusion protein formed after cleavage of intermediate of fusion protein 3.2 by EK enzyme (that is, SEQ ID NO: 62) | 0.7128-1.490 | Enzymatically active |
| Fusion protein 3.3 | hKLK1 fusion protein formed after cleavage of intermediate of fusion protein 3.3 by EK enzyme (that is, SEQ ID NO: 63) | 1.222-2.067 | Enzymatically active |
| Fusion protein 3.4 | hKLK1 fusion protein formed after cleavage of intermediate of fusion protein 3.4 by EK enzyme (that is, SEQ ID NO: 64) | 0.5391-1.655 | Enzymatically active |
| Fusion protein 3.5 | hKLK1 fusion protein formed after cleavage of intermediate of fusion protein 3.5 by EK enzyme (that is, SEQ ID NO: 65) | 0.3338-0.8944 | Enzymatically active |
| Protein 4.1 | prepro-hKLK1 formed after removing signal peptide from human wild-type KLK1 protein (that is, SEQ ID NO: 94) | 3.744-6.243 | Enzymatically active |
| Protein 4.2 | hKLK1 formed after cleavage of intermediate of protein 4.2 by EK enzyme (that is, SEQ ID NO: 2) | 5.105-8.499 | Enzymatically active |
| Fusion protein 4.3 | hKLK1 fusion protein formed after cleavage of intermediate of fusion protein 4.3 by EK enzyme (that is, SEQ ID NO: 58) | 4.879-5.867 | Enzymatically active |

### Example 5: Pharmacokinetic study on protein concentrations of fusion protein 2.1 and Kailikang^{®} in serum of CD-1 mice after intravenous injection of fusion protein 2.1 and Kailikang^{®}

CD-1 mice were given fusion protein 2.1 and Kailikang^{®} by intravenous injection, and the serum was collected from the mice at various time points. The protein concentrations of fusion protein 2.1 and Kailikang ^{®} in serum of CD-1 mice were detected using a commercial kit, and pharmacokinetically analyzed. 24 female CD-1 mice with an average weight of 25-30 g were divided into 2 groups and dosed according to the dosing regimen in Table 4. Each mouse was labeled on the tail with a label and the experimental cage was labeled. Animals in each group were not fast, and free to drink water during the experiment.

**Table 4. Dosing regimen**

| **Group** | **Test agent** | **Route of administration** | **Animal No.** | **Dose (mg/kg)** | **Volume (mL/kg)** | **Concentration (mg/mL)** |
|---|---|---|---|---|---|---|
| 1 | Fusion protein 2.1 | I.V. | M01-M12 | 1 | 5 | 0.2 |
| 2 | Kailikang^{®} | I.V. | M13-M24 | 0.25 | 5 | 0.05 |

**Table 5. Serum sample collection scheme for PK measurement**

| **Group** | **Test agent** | **Animal No.** | **Blood cross-sampling point** |
|---|---|---|---|
| 1 | Fusion protein 2.1 | M01-M03 | Blood sampling before administration |
| | | M04-M06 | T0 (2.5 min), 1h, 24 h |
| | | M07-M09 | T0 (2.5 min), 2h, 48 h |
| | | M10-M12 | T0 (2.5 min), 6h, 120 h |
| 2 | Kailikang^{®} | M13-M15 | Blood sampling before administration |
| | | M16-M18 | T0 (2.5 min), 1 h |
| | | M19-M21 | T0 (2.5 min), 4 h |
| | | M22-M24 | T0 (2.5 min), 24 h |

The collection and pretreatment methods of serum samples for PK measurement were as follows. The whole blood was collected from the tip at various time points in Table 5. About 85 µL was collected from Group 1 at each time point, and about 130 µL was collected from Group 2 at each time point. Before administration, 260 µL of whole blood was collected at the blood collection point, added to a blood collection centrifuge tube without anticoagulant, allowed to stand on ice for 30 min, and then centrifuged (3,000 g, 2-8°C, 10 min). The supernatant was collected. The serum was stored in a centrifuge tube, quickly frozen in dry ice, and then stored in an ultra-low temperature freezer at -60°C or lower.

All animals were well tolerated after administration. There was no abnormality in clinical observation during and after administration in animals. After CD-1 mice were given 1 mg/kg of fusion protein 2.1 or 0.25 mg/kg of Kailikang^{®} intravenously, the protein concentrations of fusion protein 2.1 and Kailikang ^{®} in mouse serum were detected using a commercial kit, and the average PK parameters of fusion protein 2.1 and Kailikang^{®} were calculated.

The plasma concentration-time data was analyzed by a non-compartmental model using Phoenix^{®} WinNonlin^{®} (Version 8.2, Pharsight, Mountain View, NCA). The PK parameters include peak concentration (Cₘₐₓ), peak time (Tₘₐₓ), elimination half-life (T_{1/2}), area under serum concentration-time curve (AUC), clearance rate (CL), and mean residence time (MRT). The calculation formula and results of PK parameters are shown in Table 6.

**Table 6. Calculation formula and results of PK parameters**

| **Parameter** | **Calculation formula** | **Fusion protein 2.1** | **Kailikang^{®}** |
|---|---|---|---|
| Rsq | | 1.00 | 0.852 |
| λ_{z} (1/h) | obtained by semi-logarithmic linear regression of concentration points of elimination phase | 0.0172 | 0.941 |
| T_{1/2} (h) | t_{1/2}=Ln(2)/ λ_{z} | 40.2 | 0.737 |
| C₀ (ng/mL) | Determined value | 7060 | 2120 |
| Cₘₐₓ (ng/mL) | Determined value | 6760 | 1900 |
| Tₘₐₓ (h) | Determined value | 0.0417 | 0.0417 |
| AUC₀₋ₜ (h*ng/mL) | calculated by trapezoidal method: AUC (i,i+1)=(tᵢ+1-tᵢ) (Cᵢ+Cᵢ+1)/2, AUC is the sum of all AUC(i,i+1) | 63500 | 1350 |
| AUC_{0-∞} (h*ng/mL) | AUC_{0-∞}=AUC₀₋ₜ + Cₜ/λ_{z} (Ct is the last time point of measurable blood drug concentration) | 69700 | 1380 |
| %AUCex (%) | AUC_{extrap(%)}=((AUC_{0-∞}-AUC₀₋ₜ) /AUC_{0-∞})×100 | 8.98 | 2.22 |
| CL (mL/min/kg) | CL/F = Dose / AUC_{0-∞} | 0.239 | 3.01 |
| Vz (L/kg) | V_{z}/F=Dose/(AUC_{0-∞}×λ_{z}) | 0.832 | 0.192 |
| Vss (L/kg) | | 0.584 | 0.0893 |
| MRTₗₐₛₜ(h) | MRTₗₐₛₜ =AUMCₗₐₛₜ/ AUC₀₋ₜ | 27.2 | 0.391 |
| MRT_{INF} (h) | MRT_{inf} =AUMC_{inf}/ AUC_{0-∞} | 40.7 | 0.495 |

After a single intravenous injection of 1 mg/kg of fusion protein 2.1, the clearance rate (CL) of fusion protein 2.1 in CD-1 mice is 0.239 mL/min/kg, the apparent distribution volume (Vz) is 0.832 L/kg, the half-life (t_{1/2}) is 40.2 h, the mean residence time (MRT) is 27.2 h, and the area under the plasma concentration-time curve (AUC₀₋ₜ) from zero time point to the last blood sampling point with known concentration is 63500 h*ng/mL. The last blood sampling point of measurable concentration is 120 h, and the average concentration is 108 ng/mL.

After a single intravenous injection of 0.25 mg/kg Kailikang^{®}, the clearance rate (CL) of Kailikang^{®} in CD-1 mice is 3.01 mL/min/kg, the apparent distribution volume (Vz) is 0.192 L/kg, the half-life (t_{1/2}) is 0.737 h, the mean residence time (MRT) is 0.391 h, and the area under the plasma concentration-time curve (AUC₀₋ₜ) from zero time point to the last blood sampling point with known concentration is 1350 h*ng/mL. The last blood sampling point of measurable concentration is 4 h, and the average concentration is 28.9 ng/mL.

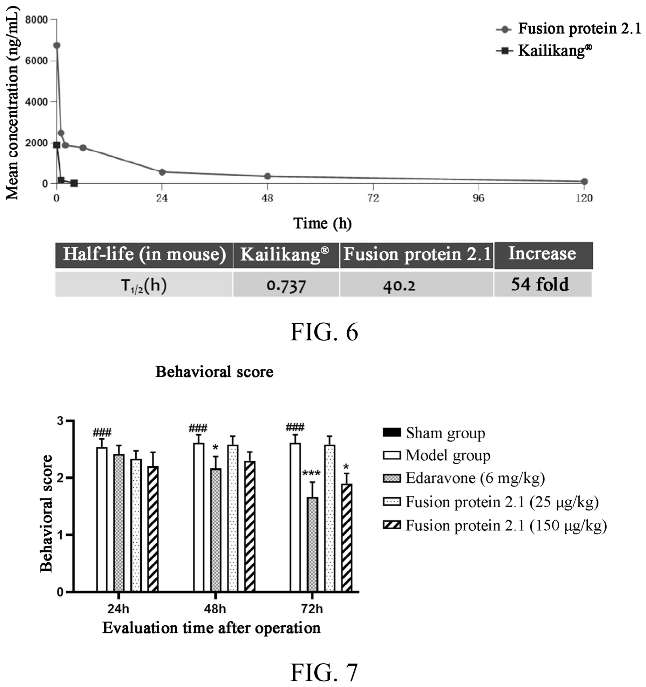

After single intravenous injections of 1 mg/kg of fusion protein 2.1 and 0.25 mg/kg Kailikang^{®} to CD-1 mice, the blood concentration-time curve in animals is as shown in FIG. 6. In this experiment, fusion protein 2.1 is administered to the mice only once, and the blood concentration of fusion protein 2.1 can still be detected on Day 3 after administration. Kailikang^{®} is also administered only once, but the blood concentration cannot be detected after 24 h. In addition, the half-life of fusion protein 2.1 is 54 times higher than that of Kailikang^{®}.

### Example 6: Effect of hKLK1 fusion protein in cerebral ischemia-reperfusion rats (i.e. tMCAO model)

### 6.1. Experimental Animals

The test drug information used in this example is shown below:
- Edaravone injection: positive control, purchased from Nanjing Xiansheng Dongyuan Pharmaceutical Co., Ltd. (batch number: 80-190516)
- Fusion protein 2.1 prepared in Example 2

The reagent information used in the experiment is shown in Table 7:

**Table 7. Reagent information**

| **Reagent** | **Supplier** | **Batch number** |
|---|---|---|
| 0.9% sodium chloride injection | Huayu (Wuxi) Pharmaceutical Co., Ltd. | 20011402 |
| Zoletil 50 | Virbac | 82T7A |
| Xylazine injection | Jinghua pasture animal health products Co., Ltd. | 20201118 |
| Gentamicin sulfate injection | Huazhong Pharmaceutical Co., Ltd. | 20180622 |
| Meloxicam injection | Qilu animal health products Co., Ltd. | 907101001 |
| TTC (2,3,5-triphenylchlorotetrazole) | Sigma Aldrich (Shanghai) Trading Co., Ltd. | BCBZ6388 |

### 6.2. Construction of animal model of tMCAO

50 SPF healthy 6-7-week-old male SD rats (200 g-250 g, purchased from Beijing Vital River Experimental Animal Technology Co., Ltd.) were included in the experiment for studying the effect of the test samples in cerebral ischemia-reperfusion rats (i.e. tMCAO model).

On the day of the test, the rats in each group were anesthetized with Zoletil 50 (50 mg/kg, *i.m*.)*+* Xylazine (8 mg/kg, *i.p*.) in combination. Then the rats were fixed in a supine position, the skin in the center of the neck was cut, then the layers of tissues were bluntly dissected to expose the left common carotid artery, and the external carotid artery and the internal carotid artery were separated. A slipknot was tied at a proximal part of the common carotid artery. The external carotid artery was looped with two sutures. The distal end was permanently ligated, and a loose ligature was made at the proximal end to secure the embolic filament. The internal carotid artery was clamped by an artery clamp. A small opening was made between the two sutures looped on the external carotid artery, and the embolic filament was inserted from the small opening toward the direction of the internal carotid artery. The embolic filament was gently tied with the fixing suture, and the artery clamp was loosened. The embolic filament was continuously gently pushed toward the direction of the internal carotid artery to reach the embolic filament mark (about 18mm±0.5mm). The fixing suture was tightly ligated to fix the embolic filament at the external carotid artery, and the necks of rats were sutured. The embolic filament was pulled to the external carotid artery after 1.5 h of ischemia to restore the blood supply from the common carotid artery to the internal carotid artery. If the cerebral blood flow in the ischemic area of rats decreased by 70% (rCBF≥70%), the model was judged to be successful. After operation, the body temperature of rats was kept at 37°C until the rats were awake. The model was verified 2 times by recording cerebral blood flow with laser Doppler instrument before operation and after ischemia. Meloxicam was given for analgesia during operation and gentamicin was given after operation to prevent infection.

The efficacy of the test drugs was evaluated by the following indicators detected at the end point:
- Detection of cerebral blood flow in ischemic area: the baseline value and the value after the MCAO model was established were detected, and measurements were taken twice.
- Behavioral test: After 24 h, 48 h, and 72 h of ischemia in rats, the behavioral test was carried out by Longa score method, and measurements were taken three times.
- Cerebral infarction area: 72 h after cerebral ischemia, the cerebral infarction area was determined by TTC staining.

### 6.3. Experimental methods

The experimental rats were grouped and dosed according to Table 8. Blood was collected at the end of the experiment, and serum was prepared for subsequent detection of serum markers. After the test, all groups of rats were euthanized by carbon dioxide inhalation.

**Table 8. Groups and dosing regimens of rats**

| **Group** | **Group** | **Number of animals** | **Dose** | **Volume of administration (ml/kg)** | **Route of administration** | **Administration frequency** |
|---|---|---|---|---|---|---|
| 1 | Sham group | 10 | - | 5 | I.V. | 1 administration after reperfusion |
| 2 | Model group | 10 | - | 5 | I.V. | |
| 3 | Positive control group (Edaravone) | 10 | 6 mg/kg | 5 | I.V. | |
| 4 | Low-dose fusion protein 2.1 | 10 | 25 µg/kg | 5 | I.V. | |
| 5 | High-dose fusion protein 2.1 | 10 | 150 µg/kg | 5 | I.V. | |

The behavioral scores of animals in each group are shown in FIG. 7. As shown in FIG. 7, low-dose (25 µg/kg) and high-dose (150 µg/kg) fusion protein 2.1 both improve the behavior of rats 72 h after ischemia-reperfusion, where the high-dose group (150 µg/kg) can achieve significant improvement.

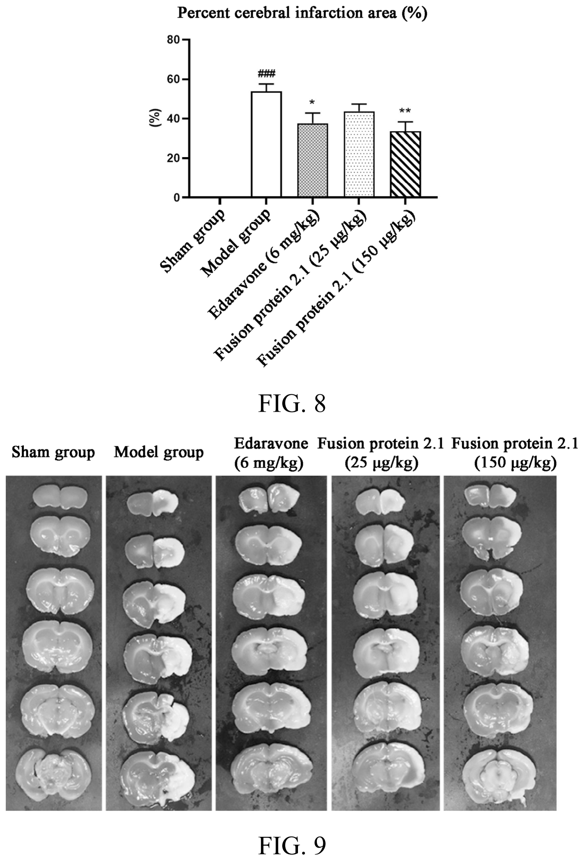

The TTC staining results of cerebral infarction area of animals in each group are shown in FIG. 8, and the images of brain tissues after ischemia-reperfusion of animals in each group are shown in FIG. 9. As shown in FIGs. 8 and 9, compared with the model group, the low-dose (25 µg/kg) and high-dose (150 µg/kg) fusion protein 2.1 show a dose-dependent protective trend in the brain tissue of rats after ischemia-reperfusion.

Although the present application has been illustrated and described with reference to specific embodiments, those skilled in the art should understand that various changes can be made to the above disclosure with respect to the forms and details without departing from the spirit and scope of the present application.

## Claims

1. A method for preparing a fusion protein comprising tissue kallikrein 1 (KLK1), comprising Step (1): introducing a recombinant vector comprising a nucleic acid molecule encoding a fusion protein into a suitable protein expression system, wherein the fusion protein comprises a masking peptide, a first polypeptide and a second polypeptide, the masking peptide, the first polypeptide and the second polypeptide are linked directly or covalently via a linker, and
a) the masking peptide has an amino acid sequence that can be specifically recognized and cleaved by a protease;
b) the first polypeptide comprises KLK1 protein or a variant thereof; and
c) the second polypeptide is used to extend the half-life of the first polypeptide.

2. The method according to claim 1, further comprising Step (2): mixing the fusion protein obtained in Step (1) with a protease to perform a specific enzymatic cleavage reaction, thereby removing the masking peptide.

3. The method according to claim 2, wherein the fusion protein obtained in Step (1) is purified before performing the specific enzymatic cleavage reaction in Step (2).

4. The method according to any one of the preceding claims, wherein the masking peptide is selected from the group consisting of: an enterokinase (EK) recognition sequence, a thrombin recognition sequence, a blood coagulation factor Xa recognition sequence, a tobacco etch virus (TEV) protease recognition sequence, an SUMO protease recognition sequence, and a sortase recognition sequence.

5. The method according to claim 4, wherein the masking peptide is an EK recognition sequence.

6. The method according to claim 5, wherein the EK recognition sequence consists of an amino acid sequence as shown in SEQ ID NO: 4 (DDDDK) or SEQ ID NO: 5 (VDDDDK).

7. The method according to any one of the preceding claims, wherein the first polypeptide is human KLK1 protein or a variant thereof.

8. The method according to claim 7, wherein the first polypeptide comprises an amino acid sequence having at least 90%, at least 95% or at least 98% sequence identity to the amino acid sequence as shown in SEQ ID NO: 2, and yet retains the function or activity of KLK1.

9. The method according to claim 8, wherein an amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121, or SEQ ID NO: 122.

10. The method according to any one of the preceding claims, wherein the second polypeptide is an Fc domain or albumin.

11. The method according to claim 10, wherein the Fc domain is derived from human IgG Fc domain.

12. The method according to claim 11, wherein the Fc domain is derived from human IgG1 Fc domain.

13. The method according to claim 11, wherein the Fc domain is derived from human IgG4 Fc domain.

14. The method according to claim 12, wherein the Fc domain comprises an amino acid sequence having at least 70%, at least 75%, at least 80%, at least 85%, at least 90%, or at least 95% sequence identity to the amino acid sequence as shown in SEQ ID NO: 11.

15. The method according to claim 14, wherein the Fc domain comprises the amino acid sequence as shown in SEQ ID NO: 11; or the Fc domain consists of the amino acid sequence as shown in SEQ ID NO: 11.

16. The method according to claim 13, wherein the Fc domain comprises an amino acid sequence having at least 70%, at least 75%, at least 80%, at least 85%, at least 90%, or at least 95% sequence identity to the amino acid sequence as shown in SEQ ID NO: 12.

17. The method according to claim 16, wherein the Fc domain comprises the amino acid sequence as shown in SEQ ID NO: 12; or the Fc domain consists of the amino acid sequence as shown in SEQ ID NO: 12.

18. The method according to any one of claims 10 to 17, wherein the Fc domain comprises mutation(s) at one, two, three, four, five or more amino acid positions.

19. The method according to claim 18, wherein the mutations are M252Y, S254T, and T256E.

20. The method according to claim 19, wherein the Fc domain comprises an amino acid sequence as shown in SEQ ID NO: 13, or the Fc domain consists of the amino acid sequence as shown in SEQ ID NO: 13.

21. The method according to claim 19, wherein the Fc domain comprises an amino acid sequence as shown in SEQ ID NO: 14, or the Fc domain consists of the amino acid sequence as shown in SEQ ID NO: 14.

22. The method according to claim 10, wherein the albumin comprises one or more domains of human serum albumin.

23. The method according to claim 22, wherein the albumin comprises D3 domain of human serum albumin.

24. The method according to any one of the preceding claims, wherein the second polypeptide is located at N-terminus or C-terminus of the first polypeptide.

25. The method according to any one of the preceding claims, wherein the fusion protein comprises, in order from the N-terminus to the C-terminus, the masking peptide, the first polypeptide, and the second polypeptide.

26. The method according to any one of the preceding claims, wherein the amino acid sequence of the masking peptide is as shown in SEQ ID NO: 5, the amino acid sequence of the first polypeptide is as shown in SEQ ID NO: 2, SEQ ID NO: 115, SEQ ID NO: 117, SEQ ID NO: 118, SEQ ID NO: 119, SEQ ID NO: 120, SEQ ID NO: 121 or SEQ ID NO: 122, and the amino acid sequence of the second polypeptide is as shown in SEQ ID NO: 11, SEQ ID NO: 12, SEQ ID NO: 13, SEQ ID NO: 14 or SEQ ID NO: 15.

27. The method according to any one of claims 2 to 26, wherein the amino acid sequence of the fusion protein obtained after removing the masking peptide in Step (2) is as shown in SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 114 or SEQ ID NO: 128.

28. The method according to any one of the preceding claims, wherein the fusion protein further comprises a tag.

29. The method according to claim 28, wherein the tag is selected from the group consisting of: a fluorescent tag, a luminescent tag, a purification tag, and a chromogenic tag.

30. The method according to claim 29, wherein the tag is a purification tag.

31. The method according to claim 29, wherein the tag is selected from the group consisting of: HIS tag, GST tag, MBP tag, Myc tag, NusA tag, SUMO tag, FLAG tag, Avi tag, Halo tag, SNAP tag, Strep-II tag, and TrX tag.

32. The method according to claim 31, wherein the tag is a HIS tag.

33. The method according to claim 32, wherein the tag is a HIS tag comprising 5, 6, 7, 8, 9 or 10 histidine.

34. The method according to any one of the preceding claims, wherein the fusion protein comprises, from the N-terminus to the C-terminus, a structure of:
LB-MP-L1-P1-L2-P2,
wherein LB is absent or represents a tag, MP represents the masking peptide, L1 represents a bond or a first linker, P1 represents the first polypeptide, L2 represents a bond or a second linker, and P2 represents the second polypeptide.

35. The method according to claim 34, wherein the first linker and the second linker are each independently selected from the group consisting of: a cleavable linker, a non-cleavable linker, a flexible linker, a rigid linker, a helical linker, and a non-helical linker.

36. The method according to claim 35, wherein the rigid linker comprises one, two, three, four or more repeats of the sequence as shown in SEQ ID NO: 23 (EAAAK).

37. The method according to claim 35, the flexible linker comprises a linker comprising glycine and serine.

38. The method according to claim 37, wherein the linker comprising glycine and serine comprises one, two, three, four or more repeats of the sequence as shown in SEQ ID NO: 17, SEQ ID NO: 18, SEQ ID NO: 19, SEQ ID NO: 20, SEQ ID NO: 21 or SEQ ID NO: 22.

39. The method according to any one of claims 34 to 38, wherein the first linker or the second linker comprises or consists of an amino acid sequence as shown in SEQ ID NO: 20, SEQ ID NO: 21, SEQ ID NO: 22 or SEQ ID NO: 23.

40. The method according to any one of the preceding claims, wherein the amino acid sequence of the fusion protein is as shown in any one of SEQ ID NOs: 36-55, SEQ ID NOs: 100-109, SEQ ID NOs: 111-113, and SEQ ID NOs: 125-127.

41. The method according to any one of the preceding claims, wherein the fusion protein further comprises a signal peptide.

42. The method according to claim 41, wherein the amino acid sequence of the signal peptide is as shown in SEQ ID NO: 24 or SEQ ID NO: 25 or has at least 70%, at least 75%, at least 80%, at least 85%, at least 90% or at least 95% sequence identity to the amino acid sequence as shown in SEQ ID NO: 24 or SEQ ID NO: 25.

43. The method according to any one of the preceding claims, wherein the half-life of the fusion protein obtained after removing the masking peptide by specific enzymatic cleavage is at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, or at least 14 days in the blood circulation of a subject.

44. The method according to any one of the preceding claims, wherein the nucleic acid molecule encoding the fusion protein comprises:
(a) a nucleotide sequence as shown in SEQ ID NO: 76, a nucleotide sequence as shown in SEQ ID NO: 77, and a nucleotide sequence as shown in SEQ ID NO: 78;
(b) a nucleotide sequence as shown in SEQ ID NO: 76, a nucleotide sequence as shown in SEQ ID NO: 77, and a nucleotide sequence as shown in SEQ ID NO: 79;
(c) a nucleotide sequence as shown in SEQ ID NO: 76, a nucleotide sequence as shown in SEQ ID NO: 77, and a nucleotide sequence as shown in SEQ ID NO: 80;
(d) a nucleotide sequence as shown in SEQ ID NO: 76, a nucleotide sequence as shown in SEQ ID NO: 77, and a nucleotide sequence as shown in SEQ ID NO: 81;
(e) a nucleotide sequence as shown in SEQ ID NO: 76, a nucleotide sequence as shown in SEQ ID NO: 123, and a nucleotide sequence as shown in SEQ ID NO: 78;
(f) a nucleotide sequence as shown in SEQ ID NO: 76, a nucleotide sequence as shown in SEQ ID NO: 123, and a nucleotide sequence as shown in SEQ ID NO: 79;
(g) a nucleotide sequence as shown in SEQ ID NO: 76, a nucleotide sequence as shown in SEQ ID NO: 123, and a nucleotide sequence as shown in SEQ ID NO: 80;
(h) a nucleotide sequence as shown in SEQ ID NO: 76, a nucleotide sequence as shown in SEQ ID NO: 123, and a nucleotide sequence as shown in SEQ ID NO: 81;
(i) a nucleotide sequence as shown in SEQ ID NO: 76, a nucleotide sequence as shown in SEQ ID NO: 130, and a nucleotide sequence as shown in SEQ ID NO: 78;
(j) a nucleotide sequence as shown in SEQ ID NO: 76, a nucleotide sequence as shown in SEQ ID NO: 130, and a nucleotide sequence as shown in SEQ ID NO: 79;
(k) a nucleotide sequence as shown in SEQ ID NO: 76, a nucleotide sequence as shown in SEQ ID NO: 130, and a nucleotide sequence as shown in SEQ ID NO: 80; or
(l) a nucleotide sequence as shown in SEQ ID NO: 76, a nucleotide sequence as shown in SEQ ID NO: 130, and a nucleotide sequence as shown in SEQ ID NO: 81.

45. The method according to any one of the preceding claims, wherein the nucleic acid molecule encoding the fusion protein comprises a nucleotide sequence having at least 70%, at least 75%, at least 80%, at least 85%, at least 90%, or at least 95% sequence identity to the nucleotide sequence as shown in SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 116 or SEQ ID NO: 129.

46. The method according to claim 44 or 45, wherein the nucleic acid molecule encoding the fusion protein comprises or consists of the nucleotide sequence as shown in SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 116 or SEQ ID NO: 129.

47. The method according to any one of the preceding claims, wherein the recombinant vector is selected from the group consisting of: a plasmid, an artificial chromosome, and a viral vector.

48. The method according to claim 47, wherein the recombinant vector is pcDNA plasmid (for example, pcDNA3.4 plasmid).

49. The method according to any one of the preceding claims, wherein the protein expression system is a cell expression system, and the cell expression system is selected from the group consisting of: a bacterium, a fungus, a plant or animal cell.

50. The method according to claim 49, wherein the bacterium is selected from the group consisting of: *Bacilli, Clostridia, Corynebacterium, Cupriavidus, Delftia, Escherichia, Enterobacter, Erwinia, Klebsiella, Lactobacillus, Lactococcus, Proteus, Pseudomonas, Rhodococcus, Salmonella, Serratia, Shigella,* and *Streptomyces.*

51. The method according to claim 49, wherein the bacterium is selected from the group consisting of: *Escherichia coli, Salmonella typhimurium, Serratia marcescens, Bacillus subtilis, B. licheniformis,* and *P. aeruginosa.*

52. The method according to claim 49, wherein the fungus is selected from the group consisting of: *Candida, Saccharomyces cerevisiae, Kluyveromyces, Pichia pastoris,* and *Schizosaccharomyces pombe.*

53. The method according to claim 49, wherein the animal cell is a mammalian cell.

54. The method according to claim 53, wherein the mammalian cell is a human cell or Chinese hamster ovary (CHO) cell.

55. The method according to claim 53, wherein the mammalian cell is a human embryonic kidney 293 cell (HEK293 cell).

56. The method according to claim 53, wherein the mammalian cell is a CHO cell (for example, CHO-K1 cell, and CHO-S cell).

57. A fusion protein prepared by the method according to any one of the preceding claims, having a cleavage activity of 0.3-3 PNAU/mg for kininogen to release kinin.

58. A fusion protein comprising a masking peptide, a first polypeptide and a second polypeptide, wherein the masking peptide, the first polypeptide and the second polypeptide are linked directly or covalently via a linker, and
a) the masking peptide has an amino acid sequence that can be specifically recognized and cleaved by a protease;
b) the first polypeptide comprises KLK1 or a variant thereof; and
c) the second polypeptide is used to extend the half-life of the first polypeptide.

59. The fusion protein according to claim 58, wherein the masking peptide is selected from the group consisting of: an enterokinase (EK) recognition sequence, a thrombin recognition sequence, a blood coagulation factor Xa recognition sequence, a tobacco etch virus (TEV) protease recognition sequence, an SUMO protease recognition sequence, and a sortase recognition sequence.

60. The fusion protein according to claim 59, wherein the EK recognition sequence is as shown in SEQ ID NO: 5 (VDDDDK).

61. The fusion protein according to any one of claims 57 to 60, wherein the first polypeptide is human KLK1 protein or a variant thereof.

62. The fusion protein according to claim 61, wherein the first polypeptide comprises an amino acid sequence having at least 90%, at least 95% or at least 98% sequence identity to the amino acid sequence as shown in SEQ ID NO: 2, and yet retains the function or activity of KLK1.

63. The fusion protein according to any one of claims 57 to 62, wherein the second polypeptide is an Fc domain or albumin.

64. The fusion protein according to any one of claims 57 to 63, further comprising a tag.

65. The fusion protein according to claim 64, wherein the tag is a HIS tag.

66. The fusion protein according to any one of claims 57 to 65, wherein the fusion protein comprises, from the N-terminus to the C-terminus, a structure of:
LB-MP-L1-P1-L2-P2,
wherein LB is absent or represents a tag, MP represents the masking peptide, L1 represents a bond or a first linker, P1 represents the first polypeptide, L2 represents a bond or a second linker, and P2 represents the second polypeptide.

67. The fusion protein according to claim 66, wherein the amino acid sequence of the fusion protein is as shown in any one of SEQ ID NOs: 36-55, SEQ ID NOs: 100-109, SEQ ID NOs: 111-113, and SEQ ID NOs: 125-127.

68. A nucleic acid molecule comprising a nucleotide sequence encoding the fusion protein according to any one of claims 57 to 67.

69. A vector comprising the nucleic acid molecule according to claim 68.

70. A protein expression system comprising the nucleic acid molecule according to claim 68 or the vector according to claim 69.

71. A pharmaceutical composition comprising the fusion protein according to any one of claims 57 to 67, the nucleic acid molecule according to claim 68, the vector according to claim 69, or the protein expression system according to claim 70, and a pharmaceutically acceptable carrier.

72. A method for constructing a protein expression system, comprising: introducing a nucleic acid molecule encoding the fusion protein according to any one of claims 57 to 67 into a vector to construct an expression vector; and introducing the expression vector into a protein expression system.

73. A method for treating or preventing a disease, comprising administering an isolated fusion protein prepared by the method according to any one of claims 1 to 56 to a subject in need thereof, wherein the disease is selected from the group consisting of an ischemic disease, a hemorrhagic disease, a kidney disease, a metabolic disease, and a neurodegenerative disease.

74. The method according to claim 73, wherein the ischemic disease is selected from the group consisting of cerebral ischemia (ischemic stroke), myocardial ischemia, ischemic colitis, critical limb ischemia, skin ischemia, ischemic stroke (for example, acute ischemic stroke), transient ischemic attack (TIA), and traumatic brain injury (TBI).

75. The method according to claim 73, wherein the ischemic disease is acute ischemic stroke.

76. The method according to claim 73, wherein the kidney disease is chronic nephropathy or acute renal injury.
